# FASCICULE DE BREVET EUROPEEN

(11) **EP 1 525 177 B1**
(45) Date de publication et mention de la délivrance du brevet: **27.06.2007**
(21) Numéro de dépôt: 03762749.4
(22) Date de dépôt: 08.07.2003
(51) Int. Cl.: C07C 51/00, C07C 67/00, C07C 59/90, C07C 59/88, C07C 59/84, C07C 69/712, C07C 69/736, C07C 69/738, C07C 69/67, C07C 251/48, C07C 323/63, C07C 323/61, C07C 323/64, C07C 281/00, C07C 323/09

(54) **DERIVES DE 1,3-DIPHENYLPROP-2-EN-1-ONE SUBSTITUES, PREPARATIONS ET UTILISATIONS**
SUBSTITUIERTE 1,3-DIPHENYLPROP-2-EN-1-ON DERIVATE, DEREN HERSTELLUNG UND ANWENDUNGEN
SUBSTITUTED 1,3-DIPHENYLPROP-2-EN-1-ONE DERIVATIVES, PREPARATION AND USES THEREOF

(30) Priorité: 08.07.2002 FR 0208571
(43) Date de publication de la demande: 27.04.2005
(73) Titulaire: Genfit, 59120 Loos (FR)
(72) Inventeur: NAJIB, Jamila, 40000 MARRAKECH (MA); CAUMONT-BERTRAND, Karine, F-59236 Frelinghien (FR)
(74) Mandataire: Tezier Herman, Béatrice
(86) Numéro de dépôt international: PCT/FR2003/002127
(87) Numéro de publication internationale: WO 2004/005233

(56) Documents cités:
- EP-A- 1 254 658
- WO-A-01/98291
- WO-A-98/27970
- DE-A- 4 327 365
- FR-A- 2 248 829
- FR-A- 2 383 157
- US-A- 3 994 955
- US-A- 4 656 305
- US-A- 5 276 058
- SHIBATA S: "ANTI-TUMORIGENIC CHALCONES" STEM CELLS, ALPHAMED PRESS, DAYTON, OH, US, vol. 12, 1994, pages 44-52, XP002949260 ISSN: 1066-5099
- DIMMOCK J R ET AL: "BIOACTIVITIES OF CHALCONES" CURRENT MEDICINAL CHEMISTRY, BENTHAM SCIENCE PUBLISHERS BV, BE, vol. 6, no. 12, 1999, pages 1125-1149, XP000917367 ISSN: 0929-8673
- LEBEAU J ET AL: "ANTIOXIDANT PROPERTIES OF DI-TERT-BUTYLHYDROXYLATED FLAVONOIDS" FREE RADICAL BIOLOGY AND MEDICINE, ELSEVIER SCIENCE, XX, vol. 29, no. 9, 1 novembre 2000 (2000-11-01), pages 900-912, XP001149406 ISSN: 0891-5849
- MUKHERJEE S ET AL: "SYNTHETIC AND BIOLOGICAL ACTIVITY EVALUATION STUDIES ON NOVEL 1,3-DIARYLPROPENONES" BIOORGANIC & MEDICINAL CHEMISTRY, ELSEVIER SCIENCE LTD, GB, vol. 9, no. 2, 2001, pages 337-345, XP001149402 ISSN: 0968-0896
- CHENG Z-J ET AL: "BROUSSOCHALCONE A, A POTENT ANTIOXIDANT AND EFFECTIVE SUPPRESSOR OF INDUCIBLE NITRIC OXIDE SYNTHASE IN LIPOPOLYSACCHARIDE-ACTIVATED MACROPHAGES" BIOCHEMICAL PHARMACOLOGY, PERGAMON, OXFORD, GB, vol. 61, no. 8, 15 avril 2001 (2001-04-15), pages 939-946, XP001149401 ISSN: 0006-2952
- CHENG Z-J ET AL: "ANTIOXIDANT PROPERTIES OF BUTEIN ISOLATED FROM DALBERGIA ODORIFERA" BBA - LIPIDS AND LIPID METABOLISM, ELSEVIER SCIENCE BV. AMSTERDAM, NL, vol. 1392, no. 2/3, 15 juin 1998 (1998-06-15), pages 291-299, XP001149409 ISSN: 0005-2760
- RAJAKUMAR D V ET AL: "ANTIOXIDANT PROPERTIES OF PHENYL STYRYL KETONES" FREE RADICAL RESEARCH,, YVERDON, CH, vol. 22, no. 4, 1995, pages 309-317, XP008014946 ISSN: 1071-5762
- ARTY I S ET AL: "Synthesis of benzylideneacetophenones and their inhibition of lipid peroxidation" EUROPEAN JOURNAL OF MEDICINAL CHEMISTRY 2000 FRANCE, vol. 35, no. 4, 2000, pages 449-457, XP004330428 ISSN: 0223-5234
- DATABASE CAPLUS [en ligne] CHEMICAL ABSTRACTS SERVICE, COLUMBUS, OHIO, US; retrieved from STN, accession no. 1998:679920 Database accession no. 130:105262 XP002236011 & ZHANG ET AL.: "Antioxidation of Puerperia lobata isoflavones" TONGJI YIKE DAXUE XUEBAO, vol. 26, no. 5, 1997, pages 340-342, china
- STOLL R ET AL: "Chalcone derivatives antagonize interactions between the human oncoprotein MDM2 and p53." BIOCHEMISTRY. UNITED STATES 16 JAN 2001, vol. 40, no. 2, 16 janvier 2001 (2001-01-16), pages 336-344, XP002262903 ISSN: 0006-2960
- DATABASE CA [en ligne] CHEMICAL ABSTRACTS SERVICE, COLUMBUS, OHIO, US; retrieved from STN, accession no. 86:55125 XP002262904 & SHI ET AL.: "Synthesis of ethyl flavone(or chalcone)oxyisobutyrate and its derivatives as potential antilipidemic agents" TAIWAN YAOXUE ZAZHI, vol. 27, no. 1-2, 1975, pages 12-16,
- LEBEAU J ET AL: "Beneficial effects of different flavonoids, on functional recovery after ischemia and reperfusion in isolated rat heart" BIOORGANIC & MEDICINAL CHEMISTRY LETTERS, OXFORD, GB, vol. 11, no. 1, 8 janvier 2001 (2001-01-08), pages 23-27, XP004225314 ISSN: 0960-894X
- PATENT ABSTRACTS OF JAPAN vol. 014, no. 126 (C-0699), 9 mars 1990 (1990-03-09) & JP 02 003670 A (NIPPON OIL & FATS CO LTD), 9 janvier 1990 (1990-01-09)

## Description

La présente invention concerne de nouveaux dérivés de 1,3-diphénylprop-2-èn-1-one substitués, des compositions pharmaceutiques les comprenant, leurs applications en thérapeutique, notamment pour le traitement de l'ischémie cérébrale. Elle a également trait à un procédé de préparation de ces dérivés.

En France, la pathologie vasculaire cérébrale (150000 nouveaux cas par an) représente la troisième cause de mortalité et la première cause de handicap chez l'adulte. Les accidents ischémiques et hémorragiques concernent respectivement 80% et 20% de cette pathologie. Les accidents ischémiques cérébraux constituent un enjeu thérapeutique important pour diminuer la morbidité et la mortalité de cette affection. Des avancées ont été faites non seulement dans le traitement de la phase aiguë de l'ischémie mais également dans sa prévention. Il est aussi important de noter que l'identification et la prise en charge des facteurs de risque sont essentielles au traitement de cette pathologie.

Les traitements médicamenteux des accidents ischémiques cérébraux sont fondés sur différentes stratégies. Une première stratégie consiste à prévenir la survenue des accidents ischémiques cérébraux par la prévention des facteurs de risque (hypertension artérielle, hypercholestérolémie, diabète, fibrillation auriculaire, etc.) ou par la prévention de la thrombose, en particulier à l'aide d'anti-aggrégants plaquettaires ou d'anticoagulants (Gorelick 2002) (Adams 2002).

Une deuxième stratégie consiste à traiter la phase aiguë de l'ischémie, afin d'en diminuer les conséquences à long terme (Lutsep and Clark 2001).

La physiopathologie de l'ischémie cérébrale peut être décrite de la façon suivante : la zone de pénombre, zone intermédiaire entre le coeur de l'ischémie où les neurones sont nécrosés et le tissu nerveux intact, est le siège d'une cascade physiopathologique qui aboutit en quelques jours à la mort neuronale, si la reperfusion n'est pas assurée ou si la neuroprotection n'est pas assez efficace. Le premier événement, qui survient dans les premières heures, est une libération massive de glutamate qui aboutit à une dépolarisation neuronale ainsi qu'à un oedème cellulaire. L'entrée de calcium dans la cellule induit des dégâts mitochondriaux favorisant la libération de radicaux libres ainsi que l'induction d'enzymes qui provoquent la dégradation membranaire des neurones. L'entrée de calcium et la production de radicaux libres activent à leur tour certains facteurs de transcription, comme NF-κB. Cette activation induit des processus inflammatoires comme l'induction de protéines d'adhésion au niveau endothélial, l'infiltration du foyer ischémique par les polynucléaires neutrophiles, l'activation microgliale, l'induction d'enzymes comme l'oxyde nitrique (NO) synthase de type II ou la cyclooxygenase de type II. Ces processus inflammatoires conduisent à la libération de NO ou de prostanoïdes qui sont toxiques pour la cellule. L'ensemble de ces processus aboutit à un phénomène d'apoptose provoquant des lésions irréversibles (Dirnagl, ladecola et al. 1999).

Le concept de neuroprotection prophylactique s'appuie sur des bases expérimentales mettant en évidence une résistance vis-à-vis de l'ischémie dans des modèles animaux. En effet, différentes procédures appliquées préalablement à la réalisation d'une ischémie cérébrale expérimentale permettent de rendre celle-ci moins sévère. Différents stimuli permettent d'induire une résistance à l'ischémie cérébrale : le préconditionnement (ischémie brève précédant une ischémie prolongée) ; un stress thermique ; l'administration d'une faible dose de lipopolysaccharide bactérien (Bordet, Deplanque et al. 2000).

Ces stimuli induisent des mécanismes de résistance qui activent des signaux déclenchant les mécanismes de protection. Différents mécanismes de déclenchement ont été mis en évidence : cytokines, voies de l'inflammation, radicaux libres, NO, canaux potassique ATP dépendant, adénosine. Le délai observé entre le déclenchement des évènements précoces et la résistance à l'ischémie provient de la nécessité d'une synthèse protéique. Différents types de protéines ont été décrits comme induisant la résistance à l'ischémie : les protéines du choc thermique, les enzymes anti-oxydantes et les protéines anti-apoptotiques (Nandagopal, Dawson et al. 2001).

Il existe donc un réel besoin de composés capables de prévenir l'apparition des facteurs de risque de l'accident vasculaire cérébral tels que l'athérosclérose, le diabète, l'obésité, etc., capables d'exercer une activité prophylactique en terme de neuroprotection mais également d'assurer une neuroprotection active dans la phase aiguë des accidents ischémiques cérébraux.

Les fibrates sont largement utilisés dans le cadre du traitement des hypertriglycéridémies. Ils ont également des effets favorables au niveau de l'hypercholestérolémie. Les fibrates ont un spectre d'action pléiotropique. Ils activent une classe de récepteurs nucléaires (PPARs) impliqués dans la coordination de l'expression de protéines responsables du transport ou du métabolisme des lipides. Le caractère pléiotropique du spectre d'action des fibrates réside dans la diversité des gènes cibles des PPARs. En effet, les fibrates sont capables de normaliser la lipémie et donc de réduire le développement de l'athérosclérose, mais ils ont également des propriétés anti-inflammatoires sur la paroi vasculaire et sur la thrombose (Fruchart, Staels et al. 2001).
Les PPARs (α,β,γ) appartiennent à la famille des récepteurs nucléaires activés par les hormones. Lorsqu'ils sont activés par une association avec leur ligand, ils s'hétérodimérisent avec le Retinoïd-X-Receptor (RXR) et se fixent alors sur des « Peroxisome Proliferator Response Elements » (PPREs) qui sont localisés dans la séquence des promoteurs des gènes cibles. La fixation de PPAR sur le PPRE induit ainsi l'expression du gène cible (Fruchart, Staels et al. 2001).
Les PPARs sont distribués dans une grande variété d'organes, mais avec une certaine tissu-spécificité pour chacun d'entre eux à l'exception de PPARβ dont l'expression semble ubiquitaire. L'expression de PPARα est particulièrement importante au niveau du foie et le long de la paroi intestinale alors que PPARγ s'exprime principalement dans le tissu adipeux et la rate. Au niveau du système nerveux central les trois sous types (α, β, γ) sont exprimés. Les cellules telles que les oligodendrocytes ainsi que les astrocytes expriment plus particulièrement le sous-type PPARα (Kainu, Wikstrom et al. 1994).

Les gène cibles des PPARs contrôlent le métabolisme des lipides et des glucides. Cependant, des découvertes récentes suggèrent que les PPARs participent à d'autres processus biologiques. L'activation des PPARs par leurs ligands induit le changement de l'activité transcriptionnelle de gènes qui modulent le processus inflammatoire, les enzymes antioxydantes, l'angiogénèse, la prolifération et la différenciation cellulaire, l'apoptose, les activités des iNOS, MMPases et TIMPs (Smith, Dipreta et al. 2001; Clark 2002). L'activation de PPAR α et γ est par exemple responsable de l'arrêt de la prolifération des kératinocytes épidermiques et favorise leur différenciation (Ellis, Varani et al. 2000; Komuves, Hanley et al. 2000).

Les radicaux libres interviennent dans un spectre très large de pathologies comme les allergies, l'initiation et la promotion cancéreuse, les pathologies cardiovasculaires (athérosclérose, ischémie), les désordres génétiques et métaboliques (diabètes), les maladies infectieuses et dégénératives (Alzheimer, Parkinson, Prion, etc.) ainsi que les problèmes ophtalmiques (Mates, Perez-Gomez et al. 1999).

Les espèces réactives oxygénées (ROS) sont produites pendant le fonctionnement normal de la cellule. Les ROS sont constituées de radicaux hydroxyle (OH), de l'anion superoxyde (O₂⁻), du peroxyde d'hydrogène (H₂O₂) et de l'oxyde nitrique (NO). Ces espèces sont très labiles et, du fait de leur grande réactivité chimique, elles constituent un danger pour les fonctions biologiques des cellules. Elles provoquent des réactions de peroxydation lipidique, l'oxydation de certains enzymes et des oxydations très importantes des protéines qui mènent à leur dégradation. La protection vis-à-vis de la peroxydation lipidique est un processus essentiel chez les organismes aérobies, car les produits de peroxydation peuvent causer des dommages à l'ADN. Ainsi un dérèglement ou une modification de l'équilibre entre la production, la prise en charge et l'élimination des espèces radicalaires par les défenses antioxydantes naturelles conduisent à la mise en place de processus délétères pour la cellule ou l'organisme.
La prise en charge des ROS se fait via un système antioxydant qui comprend une composante enzymatique et non enzymatique. Le système enzymatique se compose de plusieurs enzymes dont les caractéristiques sont les suivantes :
- La superoxyde dismutase (SOD) détruit le radical superoxyde en le convertissant en peroxyde. Ce dernier est lui même pris en charge par un autre système enzymatique. Un faible niveau de SOD est constamment généré par la respiration aérobie. Trois classes de SOD ont été identifiées chez l'homme, elles contiennent chacune du Cu, Zn, Fe, Mn, ou Ni comme cofacteur. Les trois formes de SOD humaines sont reparties de la manière suivante Cu-Zn SOD qui sont cytosoliques, une Mn-SOD mitochondriale et une SOD extracellulaire.
- La catalase est très efficace pour convertir le peroxyde d'hydrogène (H₂O₂) en eau et en O₂. Le peroxyde d'hydrogène est catabolisé de manière enzymatique dans les organismes aérobies. La catalase catalyse également la réduction d'une variété d'hydroperoxydes (ROOH).
- La glutathion peroxydase contient du sélénium comme cofacteur et catalyse la réduction d'hydroperoxydes (ROOH et H₂O₂) en utilisant du glutathion, et protège ainsi les cellules contre les dommages oxydatifs.

Les défenses cellulaires antioxydantes non enzymatiques sont constituées par des molécules qui sont synthétisées ou apportées par l'alimentation.

II existe des molécules antioxydantes présentes dans différents compartiments cellulaires. Les enzymes détoxifiantes sont par exemple chargées d'éliminer les radicaux libres et sont indispensables à la vie de la cellule. Les trois types de composés antioxydants les plus importants sont les caroténoïdes, la vitamine C et la vitamine E (Gilgun-Sherki, Melamed et al. 2001).

Le brevet US4656305, la demande de brevet FR2383157, et les articles de Dimmock et al., « Bioactivities of chalones », Curr. med. chem. vol. 6, no. 12, 1999, p. 1125-1149, de Stoll et al., 2001, « Chalcone derivatives antagonize interactions between the human oncoprotein MDM2 and p53 », Biochemistry vol. 40, no. 2, 16 janvier 2001, p. 336-344 et de Shi et al., « Synthesis of ethyl flavone (or chalcone) oxyisobutyrate and its derivatives as potential antilipidemic agents », Taiwan Yaoxue Zazhi. vol. 27, no. 1-2, 1975, p. 12-16 décrivent des dérivés de chalcone.

La demande de brevet WO0198291 décrit des composés 1,3-bis-phenyl-2-propèn-1-one substitués par un groupement aryl, un hétéroaromatique ou un hétérocycle.

Le brevet US3994955 décrit des acides phénoxydialkylacétiques substitués et les esters correspondants.

La demande de brevet DE4327365 décrit des composés dérivés du phénol.

L'article de Malik et al., « Synthesis and bioefficacy evaluation of 2-[4-(3-arylprop-2-enoyl)phenoxy]-N-substituted acetamides and 2-[4-(5-aryl-4,5-dihydro-1H-pyrazol-3-yl)-phenoxy]acetic acid hydrazides as potential pesticides », Indian Journal of Chemistry, vol. 40B, août 2001, pages 682-687 décrit la synthèse de composés acétamides 2-[4-(3-arylprop-2-énoyl)phénoxy]-N-substituées et de composés hydrazides d'acide 2-[4-(5-aryl-4,5-dihydro-1H-pyrazol-3-yl)-phenoxy]acétique.

Pour éviter le phénomène d'apoptose induit par l'ischémie cérébrale et ses conséquences secondaires, les inventeurs ont mis au point de nouveaux composés capables de prévenir l'apparition des facteurs de risque décrits ci-dessus et capables d'exercer une activité prophylactique en terme de neuroprotection, mais également d'assurer une neuroprotection active dans la phase aiguë des accidents ischémiques cérébraux.

Les inventeurs ont également mis en évidence que les composés selon l'invention ont à la fois des propriétés d'activateurs PPAR, d'antioxydants et d'antiinflammatoires et, à ces titres, les composés présentent un haut potentiel thérapeutique ou prophylactique des accidents ischémiques cérébraux.

La présente invention concerne ainsi de nouveaux dérivés de 1,3-diphénylprop-2-èn-1-one substitués, des compositions pharmaceutiques les comprenant, leurs applications en thérapeutique, notamment pour le traitement de l'ischémie cérébrale.

La présente invention a donc pour but de proposer de nouveaux dérivés de 1,3-diphénylprop-2-èn-1-one substitués présentant une formule améliorée et une efficacité thérapeutique satisfaisante.

Ces buts et d'autres sont atteints par la présente invention qui a notamment pour objet, selon la revendication 1, des dérivés de 1,3-diphénylprop-2-èn-1-one substitués de formule (I) X suivante : 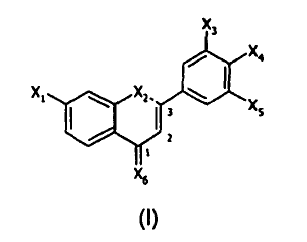 dans laquelle :
X1 représente un halogène ou un groupement -R1 ou un groupement répondant à la formule suivante : -G1-R1,
X2 représente un atome d'hydrogène ou un groupement thionitroso ou un groupement hydroxy ou un groupement alkyloxy non substitué ou un groupement alkylcarbonyloxy ou un groupement thiol ou un groupement alkylthio ou un groupement alkylcarbonylthio, X2 peut également représenter un atome d'oxygène ou de soufre lié au carbone 3 de la chaîne propène, pour former un dérivé de type 2-phényl-4H-1-benzopyran-4-one ou de type 2-phenyl-4H-1-benzothiopyran-4-one (cette possibilité est représentée dans la formule (I) par les pointillés),
X3 représente un groupement -R3 ou un groupement répondant à la formule suivante : -G3-R3.
X4 représente un halogène ou un groupement thionitroso ou un groupement -R4 ou un groupement répondant à la formule suivante : -G4-R4,
X5 représente un groupement -R5 ou un groupement répondant à la formule suivante : -G5-R5,
X6 est un atome d'oxygène,
R1, R3, R4, R5, identiques ou différents, représentent un atome d'hydrogène ou un groupement alkyle substitué ou non par au moins un groupement faisant partie du groupe 1 ou du groupe 2 définis ci-dessous,
G1, G3, G4, G5, identiques ou différents, représentent un atome d'oxygène ou de soufre,
avec au moins un des groupements X1, X3, X4 ou X5 répondant à la formule -G-R dans laquelle G est un atome de soufre, et
avec au moins un des groupements R1, R3, R4 ou R5 présent sous la forme d'un radical alkyle portant au moins un substituant du groupe 1 ou 2, ledit radical alkyle étant lié directement au cycle ou étant associé à un groupement G selon la formule -GR,
les substituants du groupe 1 sont choisis parmi les groupements carboxy de formule : -COOR₆ et les groupements carbamoyle de formule : -CONR₆R₇,
les substituants du groupe 2 sont choisis parmi l'acide sulfonique (-SO₃H) et les groupements sulfonamide de formule : -SO₂NR₆R₇,
avec R₆ et R₇, identiques ou différents, représentant un atome d'hydrogène ou un radical alkyle éventuellement substitué par au moins un groupe de type 1 ou 2,
leurs isomères optiques et géométriques, leurs racémates, leurs tautomères, leurs sels, leurs hydrates et leurs mélanges,
à l'exclusion des composés de formule (I) dans laquelle :
   - X₂ représente un atome d'hydrogène et X₁ représente -G1R1 où G1 représente un atome d'oxygène et R1 représente CH2COOH.

la présente invention a également pour objet des dérivés de 1,3-diphénylprop-2-èn-one substitués de formule (I) telle que définie dans la revendication 2.

La présente invention a également pour objet une composition pharmaceutique comprenant, dans un support acceptable sur le plan pharmaceutique, au moins un composé de formule (I) tel que décrit ci-dessus, éventuellement en association avec un autre actif thérapeutique.

Elle concerne aussi l'utilisation d'au moins un composé de formule (I) pour la préparation d'une composition pharmaceutique destinée à traiter une pathologie vasculaire cérébrale, tel que l'ischémie cérébrale ou un accident hémorragique cérébral.

La présente invention a enfin pour objet un procédé de préparation des composés de formule (I).

Dans le cadre de la présente invention, les dérivés de formule (I) tels que décrits ci-dessus peuvent adopter la conformation cis ou trans.

De manière avantageuse, aucun des groupements X3, X4 et X5 ne représente un atome d'hydrogène. Les composés de la formule (I) répondant à la précédente définition constituent les composés de la famille générale (II).

De manière avantageuse, un ou deux des groupements X3, X4 et X5 représente un atome d'hydrogène et X1 est un groupement alkyle non substitué. Les composés de la formule (I) répondant à la précédente définition constituent les composés de la famille générale (III).

De manière avantageuse, un ou deux des groupements X3, X4 et X5 représente un atome d'hydrogène et X2 est groupement thionitroso ou un groupement alkylcarbonyloxy ou un groupement thiol ou un groupement alkylthio ou un groupement alkylcarbonylthio, X2 peut également représenter un atome d'oxygène ou de soufre lié au carbone 3 de la chaîne propène, pour former un dérivé de type 2-phényl-4H-1-benzopyran-4-one (cette possibilité est représentée dans la formule (I) par les pointillés). Les composés de la formule (I) répondant à la précédente définition constituent les composés de la famille générale (IV).

De manière avantageuse, un ou deux des groupements X3, X4 et X5 représente un atome d'hydrogène et au moins un des groupements X1, X3, X4 ou X5 est de la forme GR dans laquelle G est un atome de soufre. Les composés de la formule (I) répondant à la précédente définition constituent les composés de la famille générale (V).

De manière avantageuse, un ou deux des groupements X3, X4 et X5 représente un atome d'hydrogène et au moins un des groupements X1, X3, X4 ou X5 est de la forme -G-R dans laquelle G est un atome d'oxygène et R est un groupement alkyle substitué par un substituant du groupe 1 dans lequel R6 n'est pas un atome d'hydrogène. Les composés de la formule (I) répondant à la précédente définition constituent les composés de la famille générale (VI).

De manière avantageuse, un ou deux des groupements X3, X4 et X5 représente un atome d'hydrogène et au moins un des groupements X1, X3, X4 ou X5 est de la forme GR dans laquelle G est un atome d'oxygène et R est un groupement alkyle substitué par une sulfonamide telle que définie ci-dessus. Les composés de la formule (I) répondant à la précédente définition constituent les composés de la famille générale (VII).

De manière avantageuse, X4 est un groupement thionitroso ou un groupement -R4 ou un groupement répondant à la formule -G4-R4. Les dérivés de la formule (I) dans lesquels X4 répond à la précédente définition représentent les dérivés de formule générale (VIII) dans laquelle G4 et R4 sont tels que définis précédemment.

De manière avantageuse, X2 est un groupement thionitroso ou un groupement hydroxy ou un groupement alkyloxy ou un groupement thiol ou un groupement alkylthio. Les dérivés de la formule (I) dans lesquels X2 répond à la précédente définition représentent les dérivés de formule générale (IX).

D'autres dérivés avantageux de la formule (I) de l'invention ont une formule générale (X) telle que X4 est un groupement thionitroso ou un groupement -R4 ou un groupement répondant à la formule -G4-R4 et X2 est un groupement thionitroso ou un groupement hydroxy ou un groupement alkyloxy ou un groupement thiol ou un groupement alkylthio, G4 et R4 étant tels que définis précédemment.

D'autres dérivés avantageux de la formule (I) de l'invention ont une formule générale (XI) telle que X1 représente un groupement -R1 ou un groupement répondant à la formule -G1-R1, avec R1 étant un groupement alkyle substitué par un groupement faisant partie du groupe 1 et G1 et le substituant du groupe 1 étant tels que définis précédemment.

Plus préférentiellement, un autre objet de l'invention concerne les dérivés de la formule (XI) tels que décrits précédemment, caractérisés en ce que X1 est un groupement -G1-R1.

Encore plus préférentiellement, un autre objet de l'invention concerne les dérivés de la formule (XI) tels que décrits précédemment, caractérisés en ce que X1 est un groupement -G1-R1 dans lequel G1 est un atome d'oxygène.

D'autres dérivés avantageux de la formule (I) de l'invention ont une formule générale (XII) telle que X1 représente un groupement -R1 ou un groupement répondant à la formule -G1-R1, avec R1 étant un groupement alkyle substitué par un groupement faisant partie du groupe 2 et G1 et le substituant du groupe 2 étant tels que définis précédemment.

D'autres dérivés avantageux de la formule (I) de l'invention ont une formule générale (XIII) telle que X3 représente un groupement -R3 ou un groupement répondant à la formule -G3-R3, avec R3 étant un groupement alkyle substitué par un groupement faisant partie du groupe 1 et G3 et le substituant du groupe 1 étant tels que définis précédemment.

D'autres dérivés avantageux de la formule (I) de l'invention ont une formule générale (XIV) telle que X3 représente un groupement -R3 ou un groupement répondant à la formule -G3-R3, avec R3 étant un groupement alkyle substitué par un groupement faisant partie du groupe 2 et G3 et le substituant du groupe 2 étant tels que définis précédemment.

D'autres dérivés avantageux de la formule (I) de l'invention ont une formule générale (XV) telle que X4 représente un groupement -R4 ou un groupement répondant à la formule -G4-R4 avec R4 étant un groupement alkyle substitué par un groupement faisant partie du groupe 1 et G4 et le substituant du groupe 1 étant tels que définis précédemment.

Plus préférentiellement, un autre objet de l'invention concerne des dérivés de la formule (XV) tels que décrits précédemment, caractérisés en ce que X4 est un groupement -G4-R4.

Encore plus préférentiellement, un autre objet de l'invention concerne des dérivés de la formule (XV) tels que décrits précédemment, caractérisés en ce que X4 est un groupement -G4-R4 dans lequel G4 est un atome d'oxygène.

Encore plus préférentiellement, un autre objet de l'invention concerne des dérivés de la formule (XV) tels que décrits précédemment, caractérisés en ce que X4 est un groupement -G4-R4 dans lequel G4 est un atome d'oxygène, et X3 ou X5 représente respectivement R3 ou G3R3, d'une part, et R5 ou G5R5, d'autre part, avec R3 ou R5 étant des groupements alkyles portant un substituant du groupe 1, ledit substituant du groupe 1 étant tel que défini précédemment.

D'autres dérivés avantageux de la formule (I) de l'invention ont une formule générale (XVI) telle que X4 représente un groupement -R4 ou un groupement répondant à la formule -G4-R4 avec R4 étant un groupement alkyle substitué par un groupement faisant partie du groupe 2 et G4 le substituant du groupe 2 étant tels que définis précédemment.

D'autres dérivés avantageux de la formule (I) de l'invention ont une formule générale (XVII) telle que X1 représente un halogène.

D'autres dérivés avantageux de la formule (I) de l'invention ont une formule générale (XVIII) telle que X1 représente un groupement -R1 avec R1 étant un groupement alkyle de C1 à C4 substitué ou non par au moins un groupement faisant partie du groupe 1 ou du groupe 2 définis ci-dessus.

D'autres dérivés avantageux de la formule (I) de l'invention ont une formule générale (XIX) telle que X1 représente un groupement -G1 R1 avec R1 étant un groupement alkyle de C1 à C3 substitué ou non par au moins un groupement faisant partie du groupe 1 ou du groupe 2 définis ci-dessus.

D'autres dérivés avantageux de la formule (I) de l'invention ont une formule générale (XX) telle que X1 représente un groupement -R1 avec R1 étant un groupement alkyle de C5 à C24 substitué ou non par au moins un groupement faisant partie du groupe 1 ou du groupe 2 définis ci-dessus.

D'autres dérivés avantageux de la formule (I) de l'invention ont une formule générale (XXI) telle que X1 représente un groupement -G1R1 avec R1 étant un groupement alkyle de C4 à C24 substitué ou non par au moins un groupement faisant partie du groupe 1 ou du groupe 2 définis ci-dessus.

D'autres dérivés avantageux de la formule (I) de l'invention ont une formule générale (XXII) telle que X6 représente un atome d'oxygène.

Un autre objet de l'invention concerne des dérivés de la formule (I) tels que décrits précédemment, caractérisés en ce que X4 est un groupement -G4-R4, R4 est tel que défini précédemment et X3 ou X5 représentent respectivement R3 ou G3R3, d'une part, et R5 ou G5R5, d'autre part, avec R3 ou R5 qui représente un groupement alkyle portant un substituant du groupe 1, ledit substituant du groupe 1 étant tel que défini précédemment.

Un autre objet de l'invention concerne les dérivés de formule (I) dans laquelle X1, X3, X4 ou X5 représente OC(CH3)2COOR6 avec R6 étant tel que défini précédemment.

Un autre objet de l'invention concerne les dérivés de formule (I) dans laquelle X1, X3, X4 ou X5 représente SC(CH3)2COOR6 avec R6 étant tel que défini précédemment.

Selon la présente invention, le terme "alkyle" désigne un radical hydrocarboné saturé, linéaire, ramifié ou cyclique, ayant plus particulièrement de 1 à 24, de préférence 1 à 10, atomes de carbone tels que méthyle, éthyle, propyle, isopropyle, butyle, isobutyle, *tert*-butyle, pentyle, néopentyle, n-hexyle. Les groupes comportant un ou deux atomes de carbone ou comportant de deux à sept atomes de carbone sont particulièrement préférés. Les groupes méthyle et éthyle sont tout particulièrement préférés.

Le terme thionitroso fait référence à un groupement nitroso lié au cycle aromatique par l'intermédiaire d'un atome de soufre.

Le terme halogène représente un atome de chlore ou un atome de brome ou un atome d'iode ou un atome de fluor.

Le terme alkyloxy fait référence à une chaîne alkyle liée au cycle par l'intermédiaire d'un atome d'oxygène. La chaîne alkyle répond à la définition précédemment énoncée.

Le terme alkylthio fait référence à une chaîne alkyle liée au cycle aromatique par l'intermédiaire d'un atome de soufre (liaison thioéther). La chaîne alkyle répond à la définition précédemment énoncée.

Selon un mode particulier de l'invention, les composés préférés sont indiqués ci-dessous avec les formules qui leur sont associées :
le 1-[2-hydroxy-4-carboxydiméthylméthyloxyphényl]-3-[3,5-di*tert*butyl-4-hydroxyphényl]prop-2-èn-1-one, le 1-[2-hydroxy-4-isopropyloxycarbonyl diméthylméthyloxyphényl]-3-[3,5-di*tert*butyl-4-hydroxyphényl]prop-2-èn-1-one et le 1-[2-hydroxy-4-éthoxycarbonyldiméthylméthyloxyphényl]-3-[3,5-ditertiobutyl-4-hydroxyphényl]prop-2-èn-1-one : 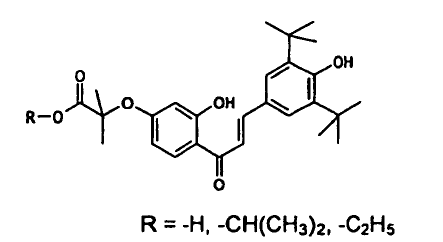
le 1-[2-hydroxyphényl]-3-[3-carboxydiméthylméthyloxy-4-hydroxy-5-*tert*butylphényl]prop-2-èn-1-one et le 1-[2-hydroxyphényl]-3-[3-*iso*propyloxycarbonyldiméthylméthyloxy-4-hydroxy-5-*tert*butylphényl]prop-2-èn-1-one: 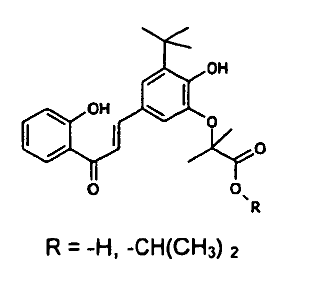
le 1-[2-hydroxy-4-chlorophényl]-3-[3-carboxydiméthylméthyloxy-4-hydroxy-5-*tert*butylphényl]prop-2-èn-1-one et le 1-[2-hydroxy-4-chlorophényl]-3-[3-*iso*propyloxycarbonyldiméthylméthyloxy-4-hydroxy-5-*tert*butylphényl]prop-2-èn-1-one: 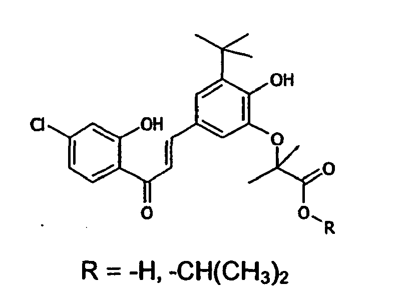
le 1-[2-hydroxyphényl]-3-[3-carboxydiméthylméthyl-4-hydroxy-5-*tert*butylphényl]prop-2-èn-1-one et le 1-[2-hydroxyphényl]-3-[3-isopropyloxycarbonyldiméthylméthyl-4-hydroxy-5-tertbutylphényl]prop-2-èn-1-one: 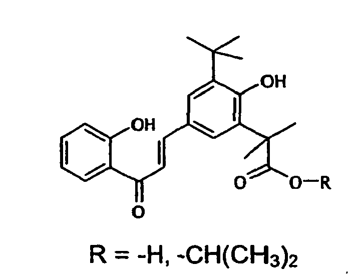
le 1-[2-hydroxy-4-chlorophényl]-3-[3-carboxyd iméthylméthyl-4-hydroxy-5-tertbutylphényl]prop-2-èn-1-one et le 1-[2-hydroxy-4-chlorophényl]-3-[3-*is*opropyloxycarbonyldiméthylméthyl-4-hydroxy-5-tertbutylphényl]prop-2-èn-1-one: 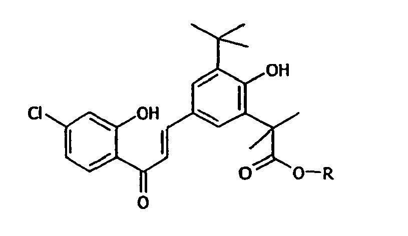 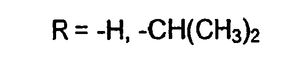
le 1-[2-hydroxy-4-chlorophényl]-3-[3,5-diméthoxy-4-carboxydiméthylméthyl oxyphényl]prop-2-èn-1-one et le 1-[2-hydroxy-4-chlorophényl]-3-[3,5-diméthoxy-4-*iso*propyloxycarbonyldiméthylméthyloxy phényl]prop-2-èn-1-one: 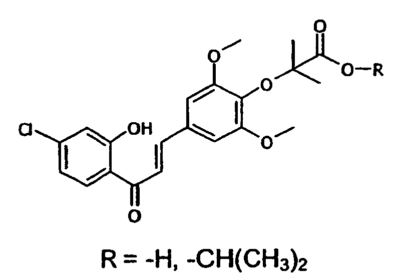
le 1-[2-hydroxyphényl]-3-[3,5-diméthoxy-4-carboxydiméthylméthyloxyphényl]prop-2-èn-1-one et le 1-[2-hydroxyphényl]-3-[3,5-diméthoxy-4-*iso*propyloxycarbonyldiméthylméthyloxyphényl]prop-2-èn-1-one: 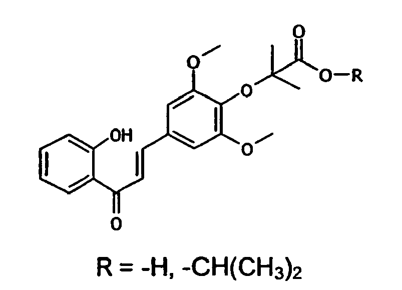
le 1-[2-hydroxy-4-carboxydiméthylméthyloxyphényl]-3-[3,5-diméthoxy-4-hydroxyphényl]prop-2-èn-1-one et le 1-[2-hydroxy-4-*iso*propyloxycarbonyldiméthylméthyloxyphényl]-3-[3,5-diméthoxy-4-hydroxyphényl]prop-2-èn-1-one: 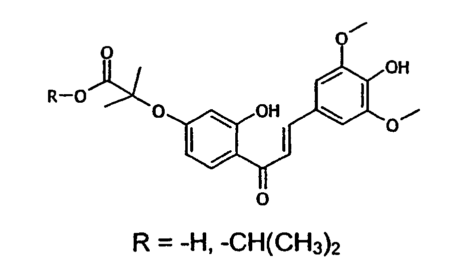
le 1-[2-hydroxy-4-chlorophényl]- 3-[3,4-dihydroxy-5-carboxydiméthylméthyloxyphényl]prop-2-èn-1-one et le 1-[2-hydroxy-4-chlorophényl]- 3-[3,4-dihydroxy-5-*iso*propyloxycarbonyldiméthylméthyloxyphényl]-2-propen-1-one: 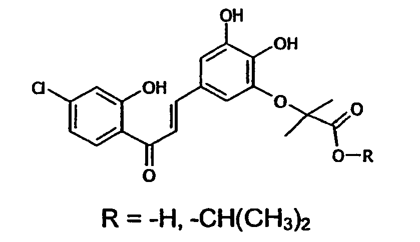
le 1-[2-hydroxy-4-carboxydiméthylméthyloxyphényl]- 3-[3,5-diméthyl-4-hydroxyphényl]prop-2-èn-1-one et le 1-[2-hydroxy-4*-iso*propyloxycarbonyl diméthylméthyloxyphényl]- 3-[3,5-diméthyl-4-hydroxyphényl]prop-2-èn-1-one: 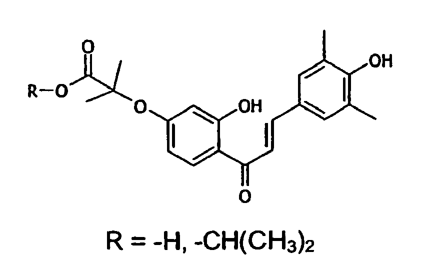
le 1-[2-hydroxy-4-chlorophényl]-3-[3,5-diméthyl-4-carboxydiméthylméthyl oxyphényl]prop-2-èn-1-one et le 1-[2-hydroxy-4-chlorophényl]-3-[3,5-diméthyl-4-*iso*propyloxycarbonyldiméthylméthyloxy phényl]prop-2-èn-1-one (composé-15) : 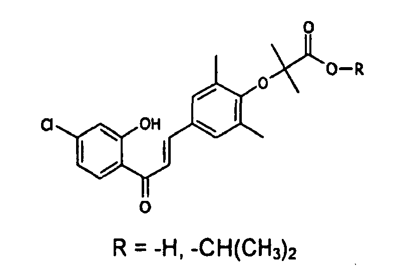
et le 1-[2-hydroxyphényl]-3-[3,5-diméthyl-4-carboxydiméthylméthyloxyphényl]prop-2-èn-1-one et le 1-[2-hydroxyphényl]-3-[3,5-diméthyl-4-*iso*propyloxycarbonyl diméthylméthyloxyphényl]prop-2-èn-1-one: 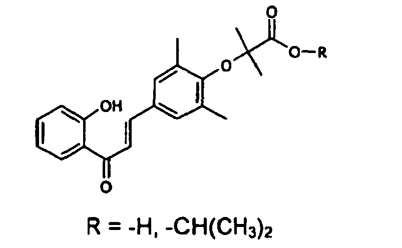
le 1-[2-hydroxyphényl]-3-[4-carboxydiméthylméthylthiophényl]prop-2-èn-1-one et le 1-[2-hydroxyphényl]-3-[4-*iso*propyloxycarbonyldiméthylméthylthiophényl]prop-2-èn-1-one (composé-18) : 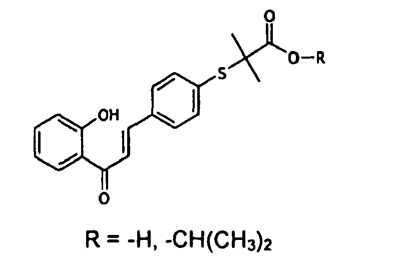
le 1-[2-hydroxy-4-carboxydiméthylméthyloxyphényl]-3-[4-méthylthiophényl]prop-2-èn-1-one,
le 1-[4-chlorophényl]-3-[3,5-diméthyl-4-tertiobutyloxycarbonyldiméthylméthyloxyphényl]prop-2-èn-1-one
le 1-[4-chlorophényl]-3-[3,5-diméthyl-4-isopropyloxycarbonyldiméthylméthyloxyphényl]prop-2-èn-1-one
le 1-[4-chlorophényl]-3-[3,5-diméthyl-4-carboxydiméthylméthyloxyphényl]prop-2-èn-1-one
le 1-[2-hydroxyphényl]-3-[4-carboxydiméthylméthylthiophényl]prop-2-èn-1-one
le 1-[4-chloro-2-hydroxyphényl]-3-[4-carboxydiméthylméthylthiophényl]prop-2-èn-1-one
le 1-[4-carboxydiméthylméthyloxyphényl]-3-[3,5-diméthyl4-hydroxyphényl]prop-2-èn-1-one
le 1-[4-méthylthiophényl]-3-[4-carboxydiméthylméthyloxyphényl]prop-2-èn-1-one
le 1-[4-carboxydiméthylméthylthiophényl]-3-[4-méthylthiophényl]prop-2-èn-1-one
le 1-[2-hydroxy-4-bromophényl]-3-[3,5-diméthyl-4-carboxydiméthylméthyloxyphényl]prop-2-èn-1-one
le 1-[4-carboxydiméthylméthyloxyphényi]-3-[4-méthytthiophényl]prop-2-èn-1-one
le 1-[4-méthylthiophényl]-3-[3,5-diméthyl-4-tertiobutyloxycarbonyldiméthylméthyloxyphényl]prop-2-èn-1-one
le 1-[4-méthylthiophényl]-3-[3,5-diméthyl-4-isopropyloxycarbonyldiméthylméthyloxyphényl]prop-2-èn-1-one
le 1-[4-méthylthiophényl]-3-[3,5-diméthyl-4-carboxydiméthylméthyloxyphényl]prop-2-èn-1-one
le 1-[2-méthoxyphényl]-3-[3,5-diméthyl-4-tertiobutyloxycarbonyldiméthylméthyloxyphényl]prop-2-èn-1-one
le 1-[2-méthoxyphényl]-3-[3,5-diméthyl-4-carboxydiméthylméthyloxyphényl]prop-2-èn-1-one
le 1-[4-hexyloxyphényl]-3-[3,5-diméthyl-4-tertiobutyloxycarbonyld iméthylméthyloxyphényl]prop-2-èn-1-one
le 1-[4-hexyloxyphényl]-3-[3,5-diméthyl-4-carboxydiméthylméthyloxyphényl]prop-2-èn-1-one
le 1-[2-méthyloxy-4-chlorophényl]-3-[3,5-diméthyl-4-tertiobutyloxycarbonyldiméthylméthyloxyphényl]prop-2-èn-1-one
le 1-[2-méthyloxy-4-chlorophényl]-3-[3,5-diméthyl-4-carboxydiméthylméthyloxyphényl]prop-2-èn-1-one
le 1-[4-heptylphényl]-3-[3,5-diméthyl-4-tertiobutyloxycarbonyldiméthylméthyloxyphényl]prop-2-èn-1-one
le 1-[4-heptylphényl]-3-[3,5-diméthyl-4-carboxydiméthylméthyloxyphényl]prop-2-èn-1-one
le 1-[4-bromophényl]-3-[3,5-diméthyl-4-tertiobutyloxycarbonyldiméthylméthyloxyphényl]prop-2-èn-1-one
le 1-[4-bromophényl]-3-[3,5-diméthyl-4-carboxy diméthylméthyloxyphényl]prop-2-èn-1-one.

Le procédé de la présente invention comprend une mise en contact en milieu basique ou en milieu acide d'au moins un composé de formule (A) avec au moins un composé de formule (B), les formules (A) et (B) étant : 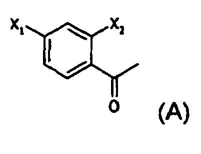 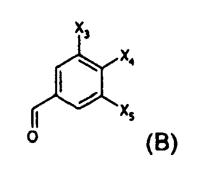 formules dans lesquelles X1, X2, X3, X4 et X5 ont les définitions données précédemment.

Les conditions de mise en oeuvre de cette réaction en milieu acide ou basique sont à la portée de l'homme du métier et peuvent varier dans une large mesure.

La mise en contact de ces deux composés est avantageusement réalisée de manière stoechiométrique. Elle est réalisée de préférence à une température ambiante (entre environ 18°C et 25°C) et à pression atmosphérique.

En milieu basique, la réaction est de préférence réalisée en présence d'une base forte, tel qu'un hydroxyde de métal alcalin, comme l'hydroxyde de sodium ou un alcoolate de métal alcalin comme l'éthylate de sodium.

En milieu acide, la réaction est de préférence réalisée en présence d'un acide fort, tel que l'acide chlorhydrique.

Le schéma réactionnel peut être représenté comme suit :

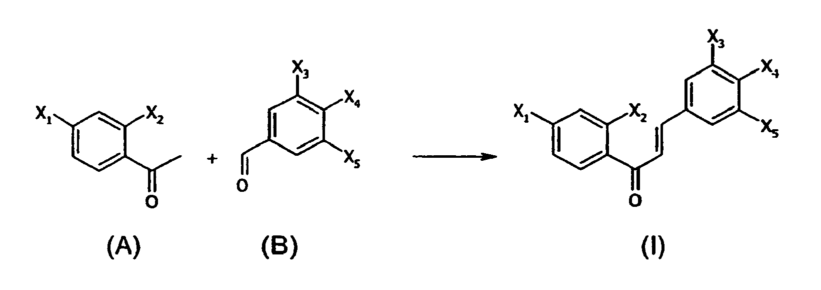

La synthèse en milieu basique peut être réalisée de la façon suivante :

La cétone (composé (A)) à 1 équivalent-molaire et l'aldéhyde (composé (B)) à 1 équivalent-molaire sont solubilisés dans une solution hydroalcoolique d'hydroxyde de sodium à 20 équivalents-molaire. L'ensemble est agité pendant environ 18 heures à température ambiante (entre 18 et 25°C). Le milieu est ensuite acidifié (pour atteindre en particulier un pH d'environ 2) notamment avec de l'acide chlorhydrique.
La 1,3-diphénylprop-2-èn-1-one substituée attendue peut être obtenue par précipitation ou extraction solide/liquide après évaporation du milieu réactionnel. Elle peut être ensuite purifiée par chromatographie sur gel de silice ou par recristallisation.

La synthèse en milieu acide peut être réalisée de la façon suivante :

La cétone (composé (A)) à 1 équivalent-molaire et l'aldéhyde (composé (B)) à 1 équivalent-molaire sont solubilisés dans une solution d'éthanol saturée d'acide chlorhydrique gazeux. L'ensemble est agité à température ambiante pendant environ 6 heures, le solvant est éliminé, notamment par évaporation sous pression réduite. La 1,3-diphénylprop-2-èn-1-one substituée est purifiée, notamment par chromatographie sur gel de silice.

Le procédé de préparation des composés de formule (I) permet de préparer des composés appelés ci-dessous matières premières et composés intermédiaires. La présente invention a également pour objet certaines matières premières et composés intermédiaires obtenus dans le cadre de la présente invention.

Ces composés (matières premières et intermédiaires) sont plus particulièrement choisis parmi :
le 1-[4-chlorophényl]-3-[3,5-diméthyl-4-hydroxyphényl]prop-2-èn-1-one (composé intermédiaire 1),
le 4-Ethyloxycarbonyl diméthylméthylthiocétophénone (matière première12)
le 1-[4-méthylthiophényl]-3-[3,5-diméthyl-4-hydroxyphényl]prop-2-èn-1-one (composé intermédiaire 2)
le 1-[2-méthoxyphényl]-3-[3,5-diméthyl-4-hydroxyphényl]prop-2-èn-1-one (composé intermédiaire 3)
le 1-[4-hexyloxyphényl]-3-[3,5-diméthyl-4-hydroxyphényl]prop-2-èn-1-one (composé intermédiaire 4)
le 1-[2-hydroxy-4-chlorophényl]-3-[3,5-diméthyl-4-hydroxyphényl]prop-2-èn-1-one (composé intermédiaire 5)
le 2-(3,5-diméthyl-4-hydroxyphényl)-7-chloro-4H-1-benzopyran-4-one (compose intermédiaire 6)
le 1-[2-méthyloxy-4-chlorophényl]-3-[3,5-diméthyl-4-hydroxydiméthylméthyloxyphényl]prop-2-èn-1-one (composé intermédiaire 7)
le 1-[4-heptylphényl]-3-[3,5-diméthyl-4-hydroxyphényl]prop-2-èn-1-one (composé intermédiaire)
le 1-[4-bromophényl]-3-[3,5-diméthyt-4-hydroxyphényl]prop-2-èn-1-one (composé intermédiaire 8)

Un autre objet de la présente invention concerne toute composition pharmaceutique comprenant dans un support acceptable sur le plan pharmaceutique au moins un composé de formule (I) tel que décrit ci-dessus.

Il s'agit avantageusement d'une composition pharmaceutique pour le traitement ou la prophylaxie des pathologies vasculaires cérébrales et plus particulièrement de l'ischémie cérébrale ou des accidents vasculaires cérébraux. Il a en effet été trouvé de manière surprenante que les composés de formule (I) possédaient à la fois des propriétés d'activateurs PPAR, d'antioxydants et d'anti-inflammatoires et possédaient une activité prophylactique et neuroprotective aiguë de l'ischémie cérébrale.

L'invention concerne également l'utilisation d'un composé tel que défini ci-avant pour la préparation d'une composition pharmaceutique destinée à la mise en oeuvre d'une méthode de traitement ou de prophylaxie du corps humain ou animal.

L'invention concerne également l'utilisation pour la préparation d'une composition pharmaceutique destinée à traiter de manière curative ou préventive des pathologies vasculaires cérébrales et plus particulièrement l'ischémie cérébrale, d'un composé de formule (I), y compris les composés de formule générale (I) dans laquelle :
- X₁, X₂, X₃ et X₅ représentent simultanément un atome d'hydrogène, X₆ représente un atome d'oxygène et X₄ représente un groupement de formule -O-CR₈R₉-COOR₁₀, avec R₈ et R₉, identiques ou différents, représentent un radical alkyle de C1 à C2, et R₆ représente un atome d'hydrogène ou un radical alkyle de C1 à C7,
- X₂, X₃ et X₅ représentent simultanément un atome d'hydrogène, X₁ représente un atome d'halogène ou un radical R1 ou -G1R1, où R1 représente un radical alkyle non substitué de C1 à C2 et G1 représente un atome d'oxygène, X₆ représente un atome d'oxygène et X₄ représente un groupement de formule -O-CR₁₁R₁₂-COOR₁₀, avec R₁₁ et R₁₂, identiques ou différents, représentent un atome d'hydrogène ou un radical alkyle de C1 à C2, et R₁₀ représente un atome d'hydrogène ou un radical alkyle de C1 à C7 et
- X₂ représente un atome d'hydrogène et X₁ représente -G1R1 où G1 représente un atome d'oxygène et R1 représente CH2COOH.

L'invention concerne également une méthode de traitement des pathologies vasculaires cérébrales et plus particulièrement de l'ischémie cérébrale, comprenant l'administration à un sujet, notamment humain, d'une dose efficace d'un composé ou d'une composition pharmaceutique tels que définis ci-avant, y compris les composés de formule générale (I) dans laquelle :
- X₁, X₂, X₃ et X₅ représentent simultanément un atome d'hydrogène, X₆ représente un atome d'oxygène et X₄ représente un groupement de formule -O-CR₈R₉-COOR₁₀, avec R₈ et R₉, identiques ou différents, représentent un radical alkyle de C1 à C2, et R₆ représente un atome d'hydrogène ou un radical alkyle de C1 à C7,
- X₂, X₃ et X₅ représentent simultanément un atome d'hydrogène, X₁ représente un atome d'halogène ou un radical R1 ou -G1R1, où R1 représente un radical alkyle non substitué de C1 à C2 et G1 représente un atome d'oxygène, X₆ représente un atome d'oxygène et X₄ représente un groupement de formule -O-CR₁₁R₁₂-COOR₁₀, avec R₁₁, et R₁₂, identiques ou différents, représentent un atome d'hydrogène ou un radical alkyle de C1 à C2, et R₁₀ représente un atome d'hydrogène ou un radical alkyle de C1 à C7, et
- X₂ représente un atome d'hydrogène et X₁ représente -G1R1 où G1 représente un atome d'oxygène et R1 représente CH2COOH.

De préférence, la méthode de traitement des pathologies vasculaires cérébrales et plus particulièrement de l'ischémie cérébrale, comprend l'administration à un sujet, notamment humain, d'une dose efficace d'un composé de formule (I) ou d'une composition pharmaceutique tels que définis ci-avant, à l'exclusion des composés de formule générale (I) dans laquelle :
- X₁, X₂, X₃ et X₅ représentent simultanément un atome d'hydrogène, X₆ représente un atome d'oxygène et X₄ représente un groupement de formule -O-CR₈R₉-COOR₁₀, avec R₈ et R₉, identiques ou différents, représentent un radical alkyle de C1 à C2, et R₆ représente un atome d'hydrogène ou un radical alkyle de C1 à C7,
- X₂, X₃ et X₅ représentent simultanément un atome d'hydrogène, X₁ représente un atome d'halogène ou un radical R1 ou -G1R1, où R1 représente un radical alkyle non substitué de C1 à C2 et G1 représente un atome d'oxygène, X₆ représente un atome d'oxygène et X₄ représente un groupement de formule -O-CR₁₁R₁₂-COOR₁₀, avec R₁₁ et R₁₂, identiques ou différents, représentent un atome d'hydrogène ou un radical alkyle de C1 à C2, et R₁₀ représente un atome d'hydrogène ou un radical alkyle de C1 à C7, et
- X₂ représente un atome d'hydrogène et X₁ représente -G1R1 où G1 représente un atome d'oxygène et R1 représente CH2COOH.
   Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc.. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.
   Les composés ou compositions selon l'invention peuvent être administrés de différentes manières et sous différentes formes. Ainsi, ils peuvent être injectés par voie orale ou systémique , comme par exemple par voie intraveineuse, intramusculaire, sous-cutanée, trans-dermique, intra-artérielle, etc.. Pour les injections, les composés sont généralement conditionnés sous forme de suspensions liquides, qui peuvent être injectées au moyen de seringues ou de perfusions, par exemple. II est entendu que le débit et/ou la dose injectée peuvent être adaptés par l'homme du métier en fonction du patient, de la pathologie, du mode d'administration, etc.. Typiquement, les composés sont administrés à des doses pouvant varier entre 1 µg et 2g /administration, préférentiellement de 0,1 mg à 1 g /administration. Les administrations peuvent être quotidiennes ou répétées plusieurs fois par jour, le cas échéant. D'autre part, les compositions selon l'invention peuvent comprendre, en outre, d'autres agents ou principes actifs.

### LEGENDES DES FIGURES

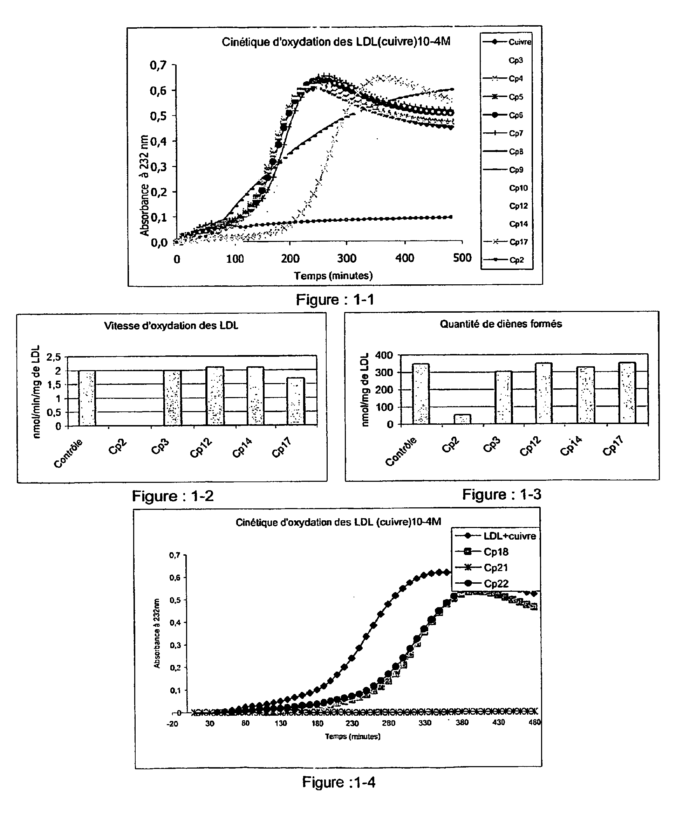

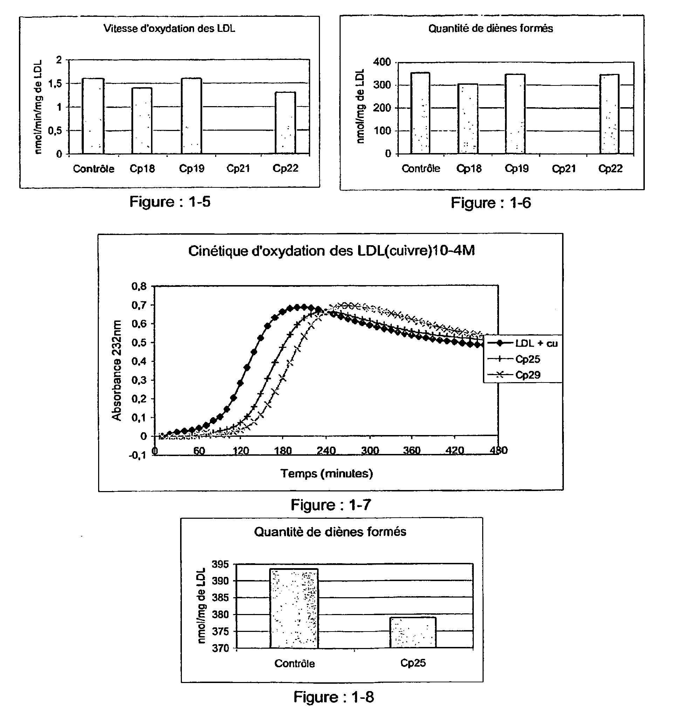

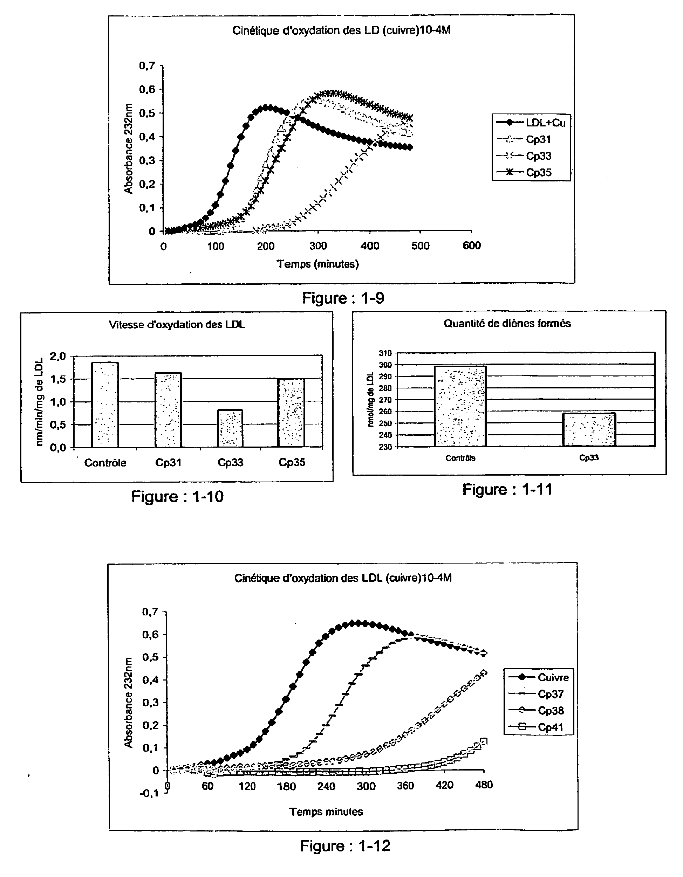

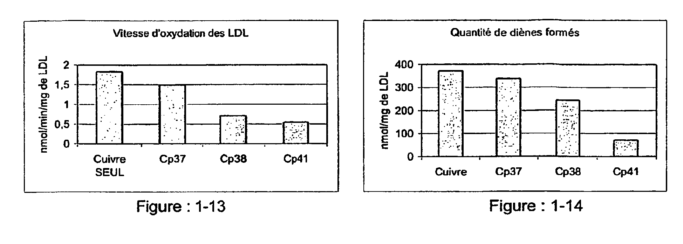

Figure 1-1, 1-2, 1-3: Evaluation des propriétés antioxydantes des composé 2, composé 12 et composé 17 sur l'oxydation des LDL par le cuivre (Cu)

Sur la figure 1-1, sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 111 minutes pour le cuivre seul alors que ce délai d'apparition des diènes conjugués est respectivement de 132, 145, 134 et 203 minutes lorsque les LDL sont incubées avec les composé, 12 composé 17. La Lag-Phase est supérieure à 480 minutes dans le cas où les LDL sont incubées avec le composé 2. Ce retard de formation de diènes conjugués est caractéristique de produits antioxydants.

La figure 1-2 représente la vitesse de formation des diènes en fonction des différents traitements. L'incubation des composés avec les LDL en présence de cuivre ralentit la vitesse de formation des diènes conjugués. La vitesse de formation des diènes conjugués est de 2 nmol/min/mg de LDL avec le cuivre seul, elle est de 1, 7 nmol/min/mg de LDL lorsque les LDL sont incubées en présence du composé 17 à 10⁻⁴M, cette vitesse est non déterminée avec le composé 2 à 10⁻⁴M (non mesurable car trop faible),

La figure 1-3 représente la quantité maximum de diènes conjugués formés au cours du temps. L'incubation des LDL avec le cuivre entraîne la formation 348 nmol/mg de LDL de diènes conjugués, l'incubation avec le composé 2 à 10⁻⁴M diminue de 84% la formation de diènes conjugués (54.4 nmol/mg de LDL). Cette concentration est respectivement de 303 nmol/mg de LDL et 327 nmol/mg de LDL en présence de composé 17.

Figure 1-4, 1-5, 1-6 : Evaluation des propriétés antioxydantes des composé 18, composé 19, composé 21 et composé 22 sur l'oxydation des LDL par le cuivre (Cu)

Sur la figure 1-4, on peut observer que l'incubation des LDL avec les composés testés à la concentration de 10⁻⁴M retarde la formation de diènes conjugués. La Lag-Phase est de 178 minutes pour le cuivre seul alors que ce délai d'apparition des diènes conjugués est respectivement de 241, 182 et 241 minutes (de la mesure expérimentale) lorsque les LDL sont incubées avec les composé 18, composé 19, ou le composé 22. Elle est supérieure à 480 minutes dans le cas où les LDL sont incubées avec le composé 21. Ce retard de formation de diènes conjugués est caractéristique de produits antioxydants.

La figure 1-5 représente la vitesse de formation des diènes en fonction des différents traitements. La vitesse de formation des diènes conjugués est de 1,6 nmol/min/mg de LDL avec le cuivre seul, elle est de 1,4 nmol/min/mg de LDL lorsque les LDL sont incubées en présence des composés 18 à 10⁻⁴M et 1,3 nmol/min/mg de LDL lorsque les LDL sont incubées en présence des composés 22, cette vitesse est non déterminée avec le composé 21 à 10⁻⁴M (non mesurable car trop faible).

La Figure 1-6 représente la quantité maximum de diènes conjugués formée au cours du temps. L'incubation des LDL avec le cuivre entraîne la formation de 353 nmol/mg de LDL de diènes conjugués. L'incubation avec le composé 21 à 10⁻⁴M inhibe la formation de diènes conjugués. En présence des composés 18, 19 et 22, elle est respectivement de 305 nmol/mg de LDL, 345 nmol/mg de LDL et 345 nmol/mg de LDL.

Figures 1-7, 1-8: Evaluation des propriétés antioxydantes des composé 25 et composé 29 sur l'oxydation des LDL par le cuivre (Cu)

Sur la figure 1-7 sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 82 minutes pour le cuivre seul alors que ce délai d'apparition des diènes conjugués est respectivement de 120 et 135 minutes (de la mesure expérimentale) lorsque les LDL sont incubées avec les composé 25 et avec le composé 29.

La figure 1-8 représente la quantité maximum de diènes conjugués formée au cours du temps. L'incubation des LDL avec le cuivre entraîne la formation de 393 nmol/mg de LDL de diènes conjugués. En présence du composé 25 elle est de 378 nmol/mg de LDL.

Figures 1-9, 1-10, 1-11 : Evaluation des propriétés antioxydantes des composé 31, composé 33 et composé 35 sur l'oxydation des LDL par le cuivre (Cu)

Sur la figure 1-9 sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 80 minutes pour le cuivre seul alors que ce délai d'apparition des diènes conjugués est respectivement de 139, 247 et 149 minutes (de la mesure expérimentale) lorsque les LDL sont incubées avec les composé 31, composé 33 et le composé 35. Ce retard de formation de diènes conjugués est caractéristique de produits antioxydants.

La figure 1-10 représente la vitesse de formation des diènes en fonction des différents traitements. L'incubation des composés avec les LDL en présence de cuivre ralentit la vitesse de formation des diènes conjugués. La vitesse de formation des diènes conjugués est de 1,9 nmol/min/mg de LDL avec le cuivre seul, elle est de 1,6 nmol/min/mg de LDL lorsque les LDL sont incubées en présence des composés 31 à 10⁻⁴M et 0,8 nmol/min/mg de LDL lorsque les LDL sont incubées en présence du composé 33 et 1,5 nmol/min/mg de LDL lorsque les LDL sont incubées en présence du composé 35.

La Figure 1-11 représente la quantité maximum de diènes conjugués formée au cours du temps. L'incubation des LDL avec le cuivre entraîne la formation de 298 nmol/mg de LDL de diènes conjugués, en présence du composé 33 elle est de 257 nmol/mg de LDL.

Figures 1-12, 1-13, 1-14 : Evaluation des propriétés antioxydantes des composé 37, composé 38 et composé 41 sur l'oxydation des LDL par le cuivre (Cu)

Sur la figure 1-12 sont représentés les résultats de l'expérience mesurant la formation de diènes conjugués en fonction du temps. On peut observer que l'incubation des LDL avec les composés testés à la concentration de 10⁻⁴M retarde la formation de diènes conjugués. La Lag-Phase est de 120 minutes pour le cuivre seul alors que ce délai d'apparition des diènes conjuguées est respectivement de 196, 284 et 411 minutes (de la mesure expérimentale) lorsque les LDL sont incubées avec les composé 37, composé 38 et le composé 41.

La figure 1-13 représente la vitesse de formation des diènes en fonction des différents traitements. L'incubation des composés avec les LDL en présence de cuivre ralentit la vitesse de formation des diènes conjugués. La vitesse de formation des diènes conjugués est de 1,8 nmol/min/mg de LDL avec le cuivre seul, elle est de 1,49 nmol/min/mg de LDL lorsque les LDL sont incubées en présence des composés 37 à 10⁻⁴M et 0,71 de LDL nmol/min/mg lorsque les LDL sont incubées en présence des composés 38 et 0,54 nmol/min/mg de LDL lorsque les LDL sont incubées en présence des composés 41.

La Figure 1-14 représente la quantité maximum de diènes conjugués formée au cours du temps. L'incubation des LDL avec le cuivre entraîne la formation de 372 nmol/mg de LDL de diènes conjugués. En présence des composés 37, 38 et 41, elle est respectivement de 338 nmol/mg de LDL, 244 nmol/mg de LDL et 71 nmol/mg de LDL.

Le retard de formation de diènes conjugués, la diminution de la vitesse de formation des diènes et la diminution de la quantité totale de diènes formés sont caractéristiques de produits antioxydants.

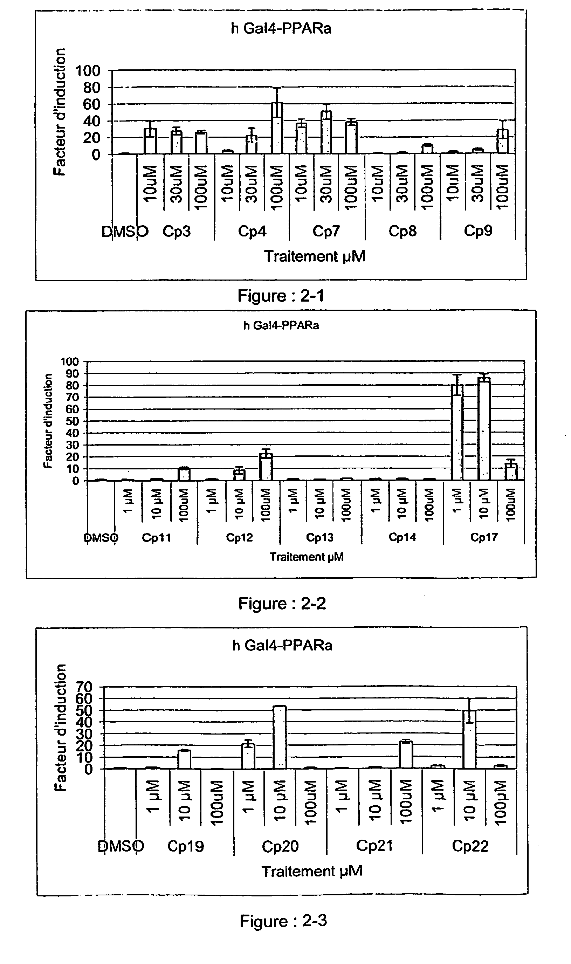

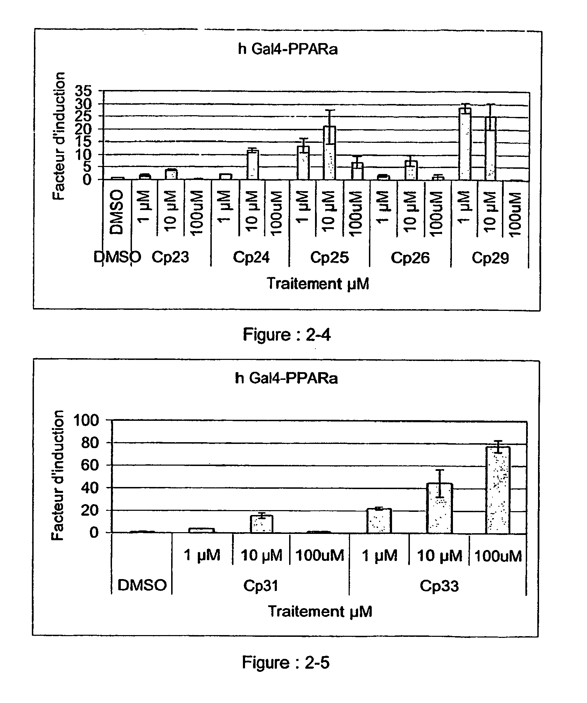

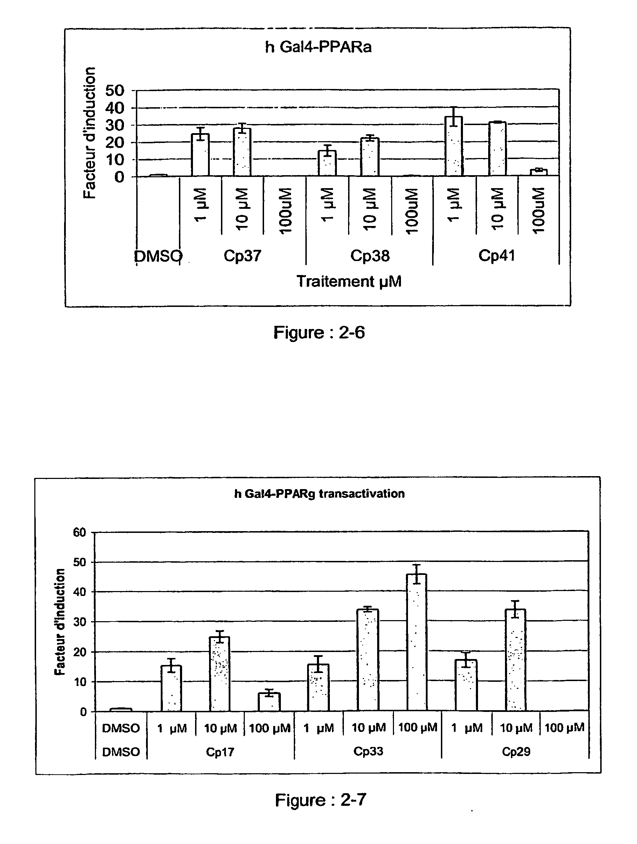

Figures 2-1, 2-2, 2-3, 2-4, 2-5, 2-6 : Evaluation des propriétés d'agoniste PPARα des composés selon l'invention avec le système de transactivation PPARα/Gal4.

Les cellules RK13 sont incubées avec différents composés aux concentrations suivantes 10, 30 et 100 µM ou 1, 10 et 100 µM pendant 24h. Les résultats sont représentés par le facteur d'induction (signal luminescent par rapport aux cellules non traitées) en fonction des différents traitements. Plus le facteur d'induction est élevé meilleure est la propriété d'agoniste pour PPARα.

### Figure 2-1 :

Les résultats montrent les facteurs d'induction des composé 7 et composé 8. Les valeurs de ces facteurs d'induction sont notées dans le tableau 2-1.

**Tableau 2-1**

| Composé | Concentration | Facteur d'induction |
|---|---|---|
| Cp7 | 10µM | 36,48 |
| | 30µM | 50,37 |
| | 100µM | 37,84 |
| Cp8 | 10µM | 0,62 |
| | 30µM | 1,27 |
| | 100µM | 9,98 |

Les résultats montrent que le composé 7 a un facteur d'induction maximal de 50 à 100µM. Le composé 8 active le système avec un facteur d'induction maximal de 10 pour la concentration de100 µM.

### Figure 2-2 :

Les résultats montrent les facteurs d'induction des composés composé 11, composé 12 et composé 17. Les valeurs de ces facteurs d'induction sont notées dans le tableau 2-2.

**Tableau: 2-2**

| Composé | Concentration | Facteur d'induction |
|---|---|---|
| Cp11 | 1 µM | 1,20 |
| | 10 µM | 1,39 |
| | 100µM | 10,19 |
| Cp12 | 1 µM | 1,12 |
| | 10 µM | 8,45 |
| | 100µM | 22,54 |
| Cp17 | 1 µM | 79,76 |
| | 10 µM | 85,69 |
| | 100µM | 13,80 |

Les résultats montrent que le composé 11 permet l'induction d'un facteur maximal de 10 à 100 µM, le composé 12 a un facteur d'induction maximal de 22 à 100 µM, de 8 à 30 µM et de 1 à 10 µM. Le composé 17 active le système avec un facteur d'induction maximal de 85 pour la concentration de 10 µM et un facteur d'induction minimal de 13,8 pour la concentration de 100µM.

### Figure : 2-3

Les résultats montrent les facteurs d'induction des composé 19, composé 20, composé 21, composé 22 . Les valeurs de ces facteurs d'induction sont notées dans le tableau 2-3.

**Tableau : 2-3**

| Composé | Concentration | Facteur d'induction |
|---|---|---|
| Cp19 | 1 µM | 1,20 |
| | 10 µM | 15,62 |
| | 100µM | 0,07 |
| Cp20 | 1 µM | 21,50 |
| | 10 µM | 53,45 |
| | 100µM | 1,22 |
| Cp21 | 1 µM | 0,78 |
| | 10 µM | 1,10 |
| | 100µM | 22,80 |
| Cp22 | 1 µM | 2,40 |
| | 10 µM | 49,49 |
| | 100µM | 2,73 |

Les résultats montrent que le composé 19 permet l'induction d'un facteur maximal de 15,6 à 10 µM, le composé 20 a un facteur d'induction maximal de 53 à 10 µM. Le composés 21 a des facteurs d'induction compris entre 0,8 et 22 pour les différentes concentrations testées. Le composé 22 active le système avec un facteur d'induction maximal de 50 pour la concentrations de 10 µM.

### Figure : 2-4

Les résultats montrent les facteurs d'induction des composé 24, composé 25, composé 26, composé 29. Les valeurs de ces facteurs d'induction sont notées dans le tableau 2-4.

**Tableau 2-4**

| Composé | Concentration | Facteur d'induction |
|---|---|---|
| Cp24 | 1 µM | 2,06 |
| | 10 µM | 11,62 |
| | 100µM | 0,00 |
| Cp25 | 1 µM | 13,48 |
| | 10 µM | 21,03 |
| | 100µM | 7,01 |
| Cp26 | 1 µM | 1,75 |
| | 10 µM | 7,85 |
| | 100µM | 1,08 |
| Cp29 | 1 µM | 28,36 |
| | 10 µM | 25,26 |
| | 100µM | 0,27 |

Le composé 24 a un facteur d'induction maximal de 11 à 10 µM. Le composé 25 active le système avec des facteurs compris entre 7 et 21 selon les concentrations testées. Le composé 26 a un facteur d'induction maximal de 7,8 pour la concentrations de10 µM. le composé 29 a des facteurs d'induction de 28 et 25 pour les concentrations de 1 et 10µM respectivement.

### Figure : 2-5

Les résultats montrent les facteurs d'induction des composé 31 et composé 33. Les valeurs de ces facteurs d'induction sont notées dans le tableau 2-5.

**Tableau : 2-5**

| Composé | Concentration | Facteur d'induction |
|---|---|---|
| Cp31 | 1 µM | 3,77 |
| | 10 µM | 15,52 |
| | 100µM | 1,21 |
| Cp33 | 1 µM | 22,05 |
| | 10 µM | 44, 52 |
| | 100µM | 77,62 |

Le composé 31 active le système avec un facteur d'induction de 15,5 à la concentration de 10µM. Les facteurs d'induction observés pour le composé 33 sont de 22, 44 et 77 pour les concentration de 1, 10 et 100µM respectivement.

### Figure : 2-6

Les résultats montrent les facteurs d'induction des composé 37, composé 38, composé 41. Les valeurs de ces facteurs d'induction sont notées dans le tableau 2-6.

**Tableau : 2-6**

| Composé | Concentration | Facteur d'induction |
|---|---|---|
| Cp37 | 1 µM | 24,55 |
| | 10 µM | 27,83 |
| | 100µM | 0,02 |
| Cp38 | 1 µM | 14,70 |
| | 10 µM | 22,22 |
| | 100µM | 0,311 |
| Cp41 | 1 µM | 34,61 |
| | 10 µM | 31,18 |
| | 100µM | 3,39 |

Les facteurs d'induction maximum pour les composés 37, 38 et 41 sont respectivement de 27, 22 et 31, ils sont observés pour la concentration de 10µM.

Ces résultats montrent que les composés selon l'invention testés possèdent la propriété de ligand vis-à-vis de PPARα et permettent aussi son activation au niveau transcriptionnel.

### Figure 2-7 : Evaluation des propriétés d'agoniste PPARγ des composés selon l'invention avec le système de transactivation PPARγ/Gal4.

Les cellules RK13 sont incubées avec différents composés aux concentrations suivantes 1, 10 et 100 µM pendant 24h. Les résultats sont représentés par le facteur d'induction (signal luminescent par rapport aux cellules non traitées) en fonction des différents traitements. Plus le facteur d'induction est élevé meilleure est la propriété d'agoniste pour PPARγ.

Les résultats de la figure montrent les facteurs d'induction des composé 17, composé 33, et composé 29. Les valeurs de ces facteurs d'induction sont notées dans le tableau 2-7.

**Tableau 2-7 :**

| | Composé | Facteur d'induction |
|---|---|---|
| Cp17 | 1 µM | 15,37 |
| | 10 µM | 24,92 |
| | 100 µM | 6,13 |
| Cp33 | 1 µM | 15,65 |
| | 10 µM | 33,90 |
| | 100 µM | 45,58 |
| Cp29 | 1 µM | 17,05 |
| | 10 µM | 33,89 |
| | 100 µM | 0,01 |

Les résultats montrent que le composé 17 permet l'induction d'un facteur maximal de 25 à 10 µM. Le composé 33 a un facteur d'induction maximal de 45,6 à 100 µM et le composé 29 de 33,9 à la concentration de 10µM.

Ces résultats montrent que les composés selon l'invention testés possèdent la propriété de ligand vis-à-vis de PPARγ et permettent aussi son activation au niveau transcriptionnel.

### Figures 3-1 et figure 3-2 :

Evaluation des propriétés neuroprotectices prophylactiques et en phase aiguë des composés selon l'invention.

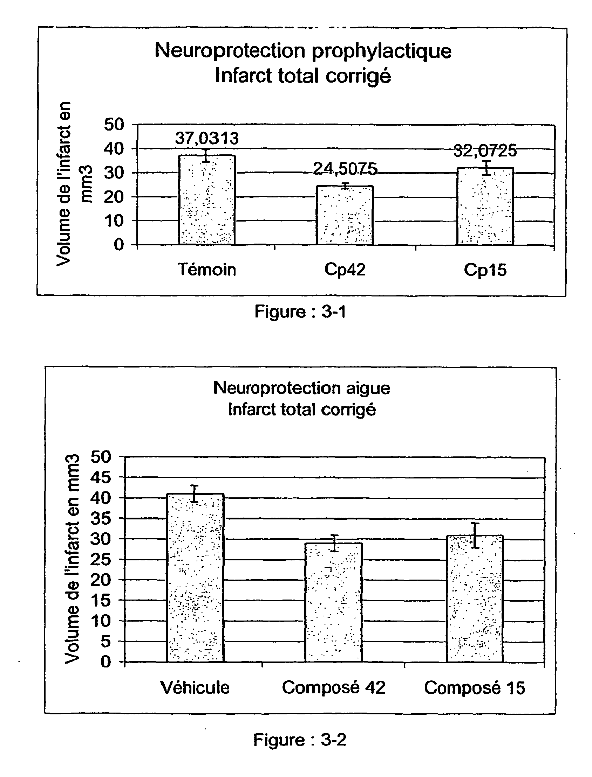

### Figure 3-1 : neuroprotection prophylactique.

Cette figure montre la taille de l'infarct en mm3 mesuré après l'occlusion intraluminale de l'artère moyenne cérébral pendant 60 minutes, reperfusion pendant 24 heures avant sacrifice. La figure 3-1 représente la taille de l'infarct obtenu avec trois groupes de souris C57 black/6. Deux de ces groupes d'animaux sont traités par gavage avec 200 mg par kg par jour de composé 15 ou 200 mg par kg par jour de composé 42 pendant 14 jours avant l'occlusion.

Cette figure permet de constater que la taille de l'infarct chez les animaux non traités est de 37 mm3 alors qu'elle est de 24 mm3 pour les animaux traités avec le composé 42 et de 32 mm3 avec le composé 15.

### Figure 3-2 : neuroprotection aiguë.

La figure 3-2 représente la taille de l'infarct obtenu avec trois groupes de souris C57 black/6. Les animaux sont traités avec 200 mg par kg par jour de composé 15 ou 200 mg par kg par jour de composé 42 pendant 72 heures après l'occlusion. Cette figure permet de constater que la taille de l'infarct total corrigé chez les animaux non traités est de 50 mm3 alors qu'elle est de 39 mm 3 pour les animaux traités avec le composé 42 et de 43 mm3 avec le composé 15.

Les résultats présentés par les figures 3-1 et 3-2 montrent l'efficacité des composés en tant que composés neuroprotecteurs. Ils sont actifs en traitement prophylactique et en traitement de la phase aiguë.

D'autres aspects et avantages de la présente invention apparaîtront à la lecture des exemples qui suivent, qui doivent être considérés comme illustratifs et non limitatifs.

### EXEMPLES

Les composés de l'invention sont préparés selon les méthodes générales décrites ci-dessous.

### Description des méthodes générales de synthèse selon l'invention :

### Synthèse de 1,3-diphénylprop-2-en-1-ones :

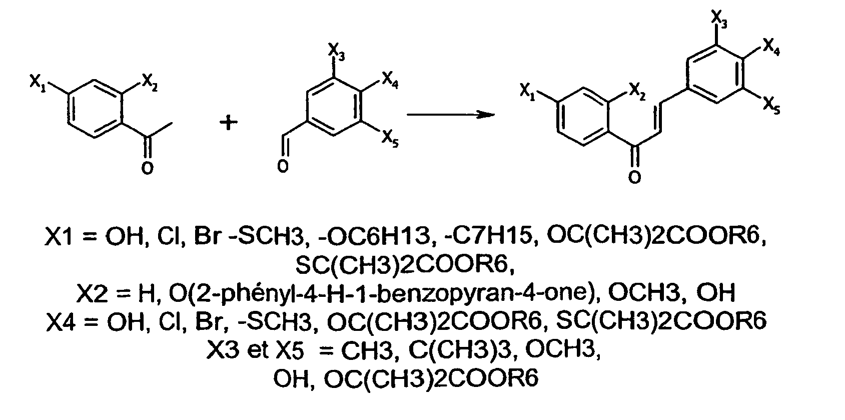

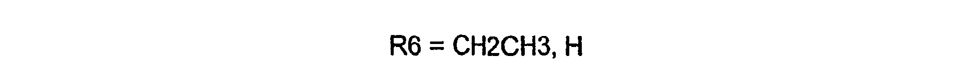

### Méthode générale 1 :

### Synthèse de 1,3-diphénylprop-2-en-1-ones en milieu acide :

La cétone (1 éq) et l'aldéhyde (1 éq) sont solubilisés dans une solution d'éthanol saturée d'acide chlorhydrique gazeux. L'ensemble est agité à température ambiante pendant 6h puis le solvant est éliminé par évaporation sous pression réduite. La 1,3-diphénylprop-2-èn-1-one est purifiée par chromatographie sur gel de silice.

### Méthode générale 2 :

### Synthèse de 1,3-diphénylprop-2-en-1-ones en milieu basique :

La cétone (1 éq) et l'aldéhyde (1 éq) sont solubilisés dans une solution hydroalcoolique d'hydroxyde de sodium (20 éq). L'ensemble est agité 18 heures à température ambiante. Le milieu est acidifié (pH = 2) avec de l'acide chlorhydrique.
La 1,3-diphénylprop-2-èn-1-one est obtenue par précipitation ou extraction solide liquide après évaporation du milieu réactionnel. Elle est purifiée par chromatographie sur gel de silice ou par recristallisation.

### Méthode générale 3 :

### Synthèse de 1,3-diphénylprop-2-en-1-ones substituées en présence d'éthylate de sodium :

Le sodium (1 éq) est dissout dans l'éthanol absolu. La cétone (1 éq) et l'aldéhyde (1 éq) sont ajoutés. L'ensemble est maintenu sous agitation à température ambiante pendant 12 heures puis une solution de soude 2N (5 éq) est ajoutée. L'ensemble est maintenu à 100°C pendant 12 heures. Le milieu réactionnel est acidifié par addition d'un solution aqueuse d'acide chlorhydrique 6N. Le solvant est éliminé par évaporation sous pression réduite. Le résidu est purifié par chromatographie sur gel de silice ou par recristallisation.

### O-Alkylation de phénols et S-alkylation de thiophénols

### Méthode générale 4 :

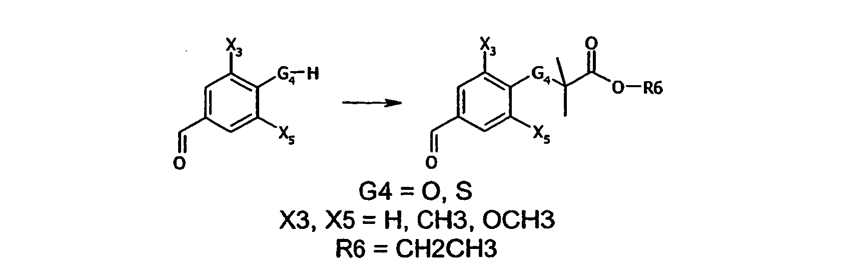

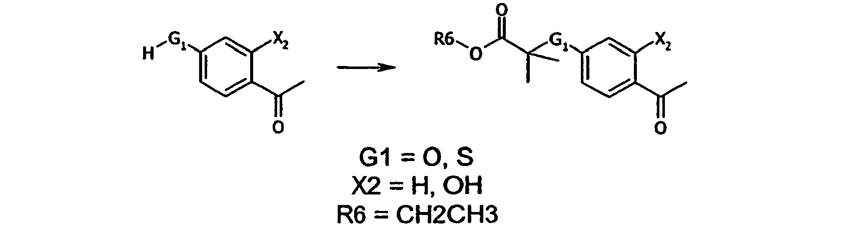

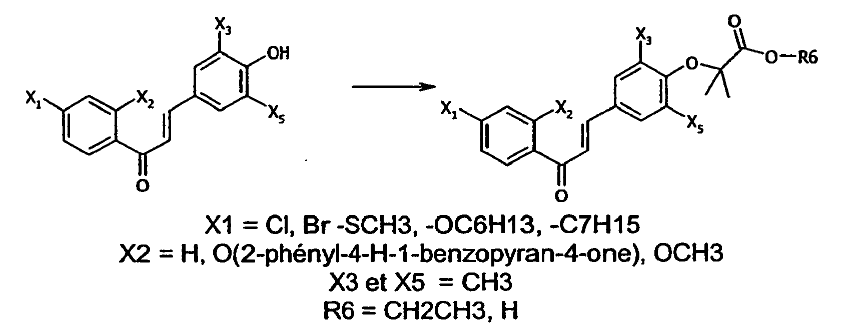

Le phénol (1 éq) est solubilisé dans l'acétonitrile, le dérivé halogéné (1 à 10 éq), puis le carbonate de potassium (5 éq) sont ajoutés. Le milieu réactionnel est maintenu environ 10 heures sous vive agitation à reflux. Les sels sont éliminés par filtration, le solvant et l'excès de réactif sont éliminés par évaporation sous pression réduite, le produit attendu est purifié par chromatographie sur gel de silice.

### Acidolyse d'esters tertiobutyliques :

### Méthode générale 5 :

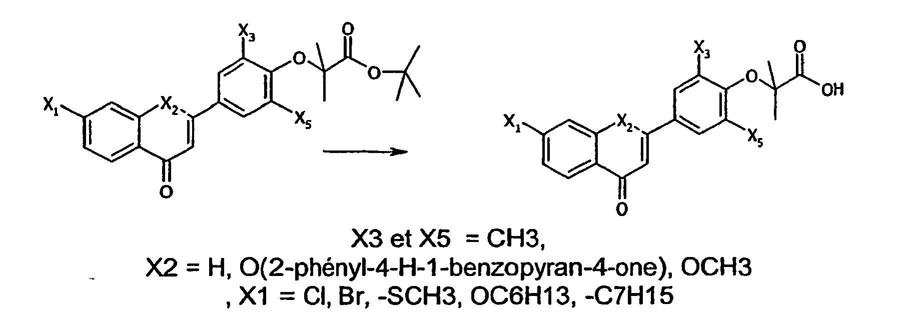

L'ester tertiobutylique (1 éq) est solubilisé dans du dichlorométhane, l'acide trifluoroacétique (10 éq) est additionné, l'ensemble est maintenu 12 heures sous agitation à température ambiante. Le produit formé est purifié par chromatographie sur gel de silice ou par recristallisation.

### Synthèse des matières premières servant à la synthèse des composés selon l'invention :

### Matière première 1 : 2'-Hydroxy-4'-(éthoxycarbonyldiméthylméthoxy)acétophénone :

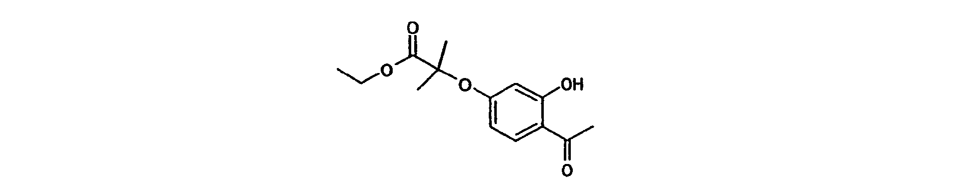

Ce composé est synthétisé à partir de 2',4'-dihydroxyacétophénone et de bromoisobutyrate d'éthyle (1 éq) selon la méthode générale 4 précédemment décrite.
Purification par chromatographie sur gel de silice (élution : cyclohexane-acétate d'éthyle : 9-1).
RMN 1H CDCl₃ δppm : 1.25 (t, J = 7.17 Hz, 3H), 1.67 (s, 6H), 2.56 (s, 3H), 4.24 (q, J = 7.17, 2H), 6.27 (d, J = 2.55 Hz, 1 H), 6.37 (dd, J = 2.55Hz, J = 8.72 Hz, 1 H), 7.62 (d, J = 8.72, 1 H), 12,6 (signal, 1 H).
Bibliographie : Brevet US n° 3,629,290 (1970), Fisons Pharmaceutical

### Matière première 2 : Acétate de 3-chlorophényle

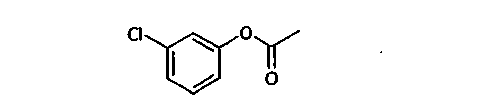

Le 3-chlorophénol est solubilisé dans le dichlorométhane. La triéthylamine (1éq) et l'anhydride acétique (2 éq) sont ajoutés. L'ensemble est agité 5 heures à température ambiante. Le solvant est éliminé par évaporation sous pression réduite. Le résidu d'évaporation est repris par du dichlorométhane, séché sur sulfate de magnésium puis le solvant est éliminé par évaporation sous pression réduite. Purification par chromatographie sur gel de silice (élution : cyclohexane-acétate d'éthyle : 95-5).
RMN 1 H CDCl₃ δppm: 2.29 (s, 3 H), 6.99-7.33 (m, 4 H)

### Matière première 3: 4'-Chloro-2'-hvdroxyacétophénone

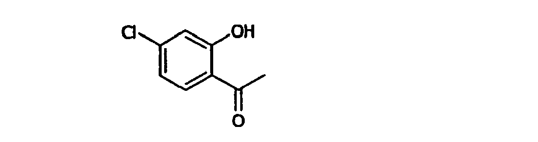

L'acétate de 3-chlorophényle (matière première 2) est mélangé au chlorure d'aluminium (3 éq). Le mélange est chauffé 1 heure à 200°C. Le milieu réactionnel est ramené à température ambiante puis versé dans la glace. La phase aqueuse est extraite par du chlorure de méthylène qui est séché sur sulfate de magnésium puis évaporé sous pression réduite.
Purification par chromatographie sur gel de silice (élution : cyclohexane-acétate d'éthyle : 95-5).
RMN 1 H CDCl₃ δppm: 3.41 (s, 3 H), 6.81 (dd, J = 8.82Hz, J = 1.47Hz, 1 H), 6.91 (d, J = 1.47Hz, 1 H), 7.60 (d, 8.82Hz, 1 H), 12.33 (s, 1 H)
Bibliographie : Chen *et al,* J Chem Soc, 1958, 146-148.

### Matière première 4 : 4-Ethvioxycarbon IlLdiméthylméthyloxybenzaldéhLrde

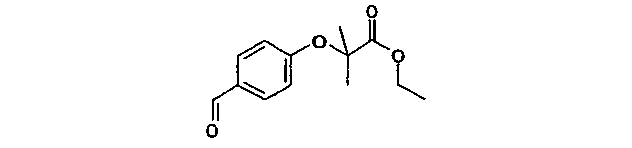

Ce composé est synthétisé à partir de 4-hydroxyabenzaldéhyde et de bromoisobutyrate d'éthyle selon la méthode générale 4 précédemment décrite.
Purification par chromatographie sur gel de silice (élution : cyclohexane-acétate d'éthyle : 9-1).
RMN 1H CDCl₃ δ ppm : 1.20(t, = 6.96Hz, 3H), 1.67(s, 6H), 4.21(q, J = 6.96Hz, 2H), 6.89(d, J = 8.91Hz, 2H),7.79 (d, J = 8.94Hz, 2H), 9.87(S, 1 H).

### Matière première 5 : 3,5-diméthyloxy-4-éthyloxycarbonyldiméthylméthyloxybenzaldéhyde

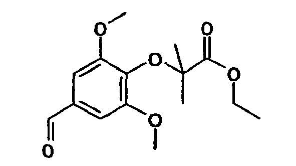

Ce composé est synthétisé à partir de 3,5-diméthyloxy-4-hydroxyabenzaldéhyde et de bromoisobutyrate d'éthyle selon la méthode générale 4 précédemment décrite.
Purification par chromatographie sur gel de silice (élution : cyclohexane-acétate d'éthyle : 8-2).
RMN 1 H CDCl₃ δ ppm : 1.33(t, J = 7.29Hz, 3H), 1.50(s, 6H), 3.84(s, 6H), 4.27(q, J = 7.29Hz, 2H), 7.08(s, 2H), 9.86(s, 1 H)

### Matière première 6: 3,5-diméthyl-4-éthyloxycarbonyldiméthylméthyrloxybenzaldéhyde

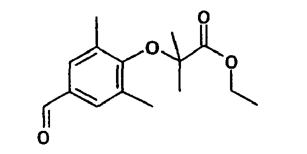

Ce composé est synthétisé à partir de 3,5-diméthyl-4-hydroxyabenzaldéhyde et de bromoisobutyrate d'éthyle selon la méthode générale 4 précédemment décrite.
Purification par chromatographie sur gel de silice (élution : cyclohexane-acétate d'éthyle : 95-5).
RMN 1H CDCl₃ δ ppm :1.37(t, J = 7.14Hz, 3H), 1.50(s, 6H), 2.29(s, 6H), 4.30(q, J = 7.14Hz, 2H), 7.54(s, 2H), 9.88 (s, 1 H)

### Matière première 7 : 3-Ethyloxycarbonyldiméthylméthyloxybenzaldéhyde :

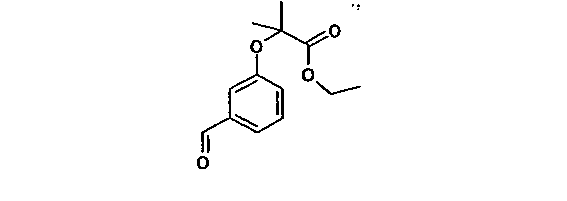

Ce composé est synthétisé à partir de 3-hydroxybenzaldéhyde et de bromoisobutyrate d'éthyle selon la méthode générale 4 précédemment décrite.
Purification par chromatographie sur gel de silice (élution : cyclohexane-acétate d'éthyle : 9-1).
RM N 1 H CDCl₃ δ ppm : 1.24(t, J = 7.27Hz, 3H), 1.62(s, 6H), 4.25(q, J = 7.27Hz, 2H), 7.11(m, 1H), 7.31(m, 1H), 7.40(t, J = 8.19Hz, 1H), 7.49(m, 1H), 9.93(s, 1 H).

### Matière première 8 : 4-Ethyloxycarbonyldiméthylméthyl thiobenzaldéhyde

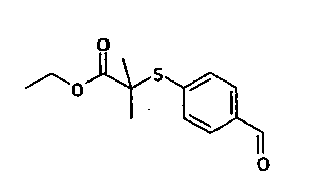

Le 4-méthylthiobenzaldéhyde (1 éq) est solubilisé dans du chlorure de méthylène, la solution est refroidie à 0°C . L'acide métachloroperbenzoique (1.5 éq) est ajouté par petites fractions. L'avancement de la réaction est suivi par chromatographie sur couche mince. Un éventuel complément d'acide métachloroperbenzoique est ajouté de façon à obtenir la disparition totale du produit de départ. Le précipité formé est éliminé par filtration. De l'hydroxyde de calcium (1,5 éq) est ajouté. L'agitation est prolongée pendant 15 min. Le solide est éliminé par filtration, le filtrat est séché sur sulfate de magnésium puis le chlorure de méthylène est éliminé par évaporation sous pression réduite.
Le résidu d'évaporation est repris par de l'anhydride acétique, l'ensemble est chauffé 30 min à reflux puis évaporé à sec. Le résidu est repris par la solution méthanol/triéthylamine, agité 15 minutes à température ambiante puis les solvants sont éliminés par évaporation sous pression réduite. Le résidu huileux est repris par une solution aqueuse saturée de chlorure d'ammonium puis extrait par du chlorure de méthylène. La phase organique est séchée sur sulfate de magnésium puis évaporée sous pression réduite.

Le 4-mercaptobenzaldéhyde intermédiaire ainsi obtenu est utilisé sans autre purification. Il est alkylé selon la méthode générale 4 pour donner le 4-éthyloxycarbonyldiméthylméthylthiobenzaldéhyde.
Purification par chromatographie sur gel de silice (élution : cyclohexane-acétate d'éthyle : 9-1).
RMN 1H CDCl₃ δ ppm : 1.22(t, J = 7.46Hz, 3H), 2.60(s, 6H), 4.15(q, J = 7.46Hz, 2H), 7.53(d, J = 8.38Hz, 2H), 7.88(d, J = 8.39, 2H), 9.99(s, 1 H)
Bibliographie : Young NR, Gauthier J Y., Coombs w (1984). Tetrahedron Letters 25(17): 1753-1756.

### Matière première 9 : 4'-Ethyloxycarbonyldiméthylméthyloxyacétophénone :

Ce composé est synthétisé à partir de 4'-hydroxyacétophénone et de bromoisobutyrate d'éthyle selon la méthode générale 4 précédemment décrite. 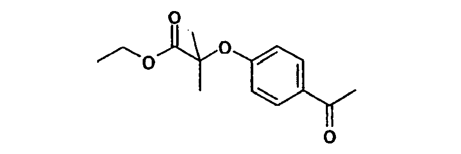 Purification par chromatographie sur gel de silice (élution : cyclohexane-acétate d'éthyle : 9-1).
RMN 1 H CDCl₃ δ ppm : 1.17(t, J = 5.64Hz, 3H), 1.61(s, 6H), 2.50(s, 3H), 4.18(q, J = 5.64Hz, 2H), 6.78(d, J = 8.82Hz, 2H), 7.83(d, J = 8.81Hz, 2H).

### Matière première 10 : Acétate de 3-bromophényle

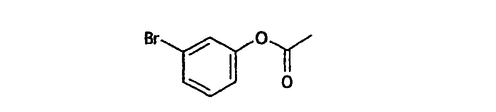

Le 3-bromophénol est solubilisé dans le dichlorométhane. La triéthylamine (1éq) et l'anhydride acétique (2 éq) sont ajoutés. L'ensemble est agité 5 heures à température ambiante. Le solvant est éliminé par évaporation sous pression réduite. Le résidu d'évaporation est repris par du dichlorométhane puis séché sur sulfate de magnésium. Le solvant est éliminé par évaporation sous pression réduite.
Purification par chromatographie sur gel de silice (élution : cyclohexane-acétate d'éthyle : 95-5).
RMN 1 H CDCl₃ δ ppm : 2.30(s, 3H), 7.0-7.4(m, 4H)

### Matière première 11 : 2'-hydroxy-4'-bromoacétophénone

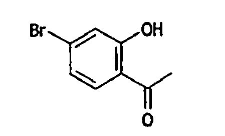

L'acétate de 3-bromophényle (matière première 10) est mélangé au chlorure d'aluminium (3 éq), le mélange est chauffé 1 heure à 200°C. Le milieu réactionnel est ramené à température ambiante puis versé dans la glace. La phase aqueuse est extraite par du chlorure de méthylène qui est séché sur sulfate de magnésium.
Purification par chromatographie sur gel de silice (élution : cyclohexane-acétate d'éthyle : 95-5).
RMN 1H CDCl₃ δ ppm : 2.59(s, 3H), 7.01(d, J = 8.5Hz, 1H), 7.13(s, 1H), 7.55(d, J = 8.5Hz, 1 H), 12.33(s, 1 H)

### Matière première 12 : 4'-Ethyloxycarbonyl diméthylméthylthiocétophénone

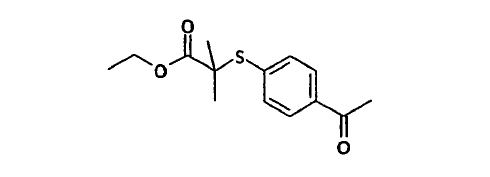

La 4'-méthylthioacétophénone est solubilisée dans du chlorure de méthylène, la solution est refroidie à 0°C. L'acide métachloroperbenzoique (1.5 éq) est ajouté par petites fractions. L'avancement de la réaction est suivi par chromatographie sur couche mince. Un éventuel complément d'acide métachloroperbenzoique est ajouté de façon à obtenir la disparition totale du produit de départ. Le précipité formé est éliminé par filtration. De l'hydroxyde de calcium (1,5 éq) est ajouté. L'agitation est prolongée pendant 15 min. Le solide est éliminé par filtration, le filtrat est séché sur sulfate de magnésium puis le chlorure de méthylène est éliminé par évaporation sous pression réduite.
Le résidu d'évaporation est repris par de l'anhydride acétique, l'ensemble est chauffé 30 min à reflux puis évaporé à sec. Le résidu est repris par la solution Méthanol/Triéthylamine, agité 15 mn à température ambiante puis les solvants sont éliminés par évaporation sous pression réduite. Le résidu huileux est repris par une solution aqueuse saturée de chlorure d'ammonium puis extrait par du chlorure de méthylène. La phase organique est séchée sur sulfate de magnésium puis évaporée sous pression réduite.

La 4-mercaptoacétophénone intermédiaire ainsi obtenue est utilisée sans autre purification. Elle est alkylée selon la méthode générale 4 pour donner la 4-Ethyloxycarbonyldiméthylméthylthioacétophénone.
Purification par chromatographie sur gel de silice (élution : cyclohexane-acétate d'éthyle : 9-1).
Bibliographie : Young NR, Gauthier J Y., Coombs w (1984). Tetrahedron Letters 25(17): 1753-1756.
RMN 1 H CDCl₃ δ ppm : 1.21(t, J = 7.32Hz, 3H), 1.51 (s, 6H), 2.59(s, 3H), 4.12(q, J = 7.32Hz, 2H), 7.51 (d, J = 8.40Hz, 2H), 7.79(d, J = 8.40Hz, 2H)

### Synthèse de composés intermédiaires servant à la synthèse des composés selon l'invention :

### Composé intermédiaire 1 : 1-[4-chlorophényl] -3-[3,5-diméthyl-4-hydroxyphényl]prop-2-èn-1-one

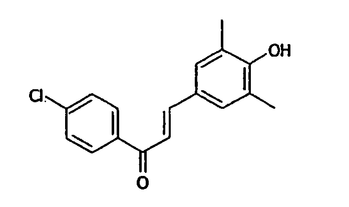

Ce composé est synthétisé à partir de 4-chloroacétophénone et de 3,5-diméthyl-4-hydroxybenzaldéhyde selon la méthode générale 1 précédemment décrite.
Purification par chromatographie sur gel de silice (élution : cyclohexane-acétate d'éthyle : 95-5).
RM N 1 H CDCl₃ δ ppm : 2.30(s, 6H), 7.32(s, 2H), 7.34(d, J = 15.25Hz, 1H), 7.47(d, J = 8.86Hz, 2H), 7.75(d, J = 15.26, 1H), 7.97(d, J = 8.86Hz, 2H).

### Composé intermédiaire 2 : 1-[4-méthylthiophényl] -3-[3,5-diméthyl-4-hydroxyphényl]prop-2-èn-1-one :

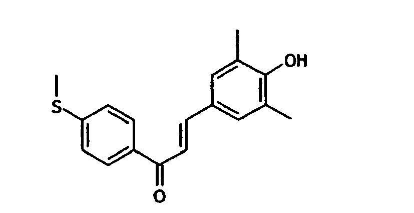

Ce composé est synthétisé à partir de 4'-méthylthioacétophénone et de 3,5-diméthyl-4-hydroxybenzaldéhyde selon la méthode générale 1 précédemment décrite.
Purification par chromatographie sur gel de silice (élution : cyclohexane-acétate d'éthyle : 8-2).
RMN 1 H DMSO δ ppm : 2.22(s, 6H), 2.54(s, 3H), 7.36(d, J = 8.20Hz, 2H), 7.48(s, 2H), 7.62(d, J = 15.7Hz, 1 H), 7.74(d, J = 15.7Hz, 1 H), 8.10(d, J = 8.20Hz, 2H), 8.92(s, 1 H)

### Composé intermédiaire 3 : 1-[2-méthoxyphényl] -3-[3,5-dirnéthyl-4-hydroxyphényl]prop-2-èn-1-one

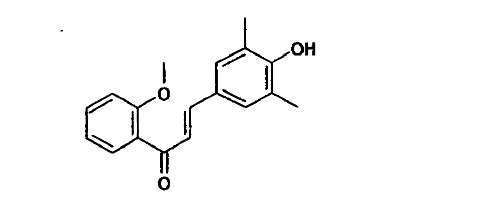

Ce composé est synthétisé à partir de 2'-méthoxyacétophénone et de 3,5-diméthyl-4 hydroxybenzaldéhyde selon la méthode générale 1 précédemment décrite.
Purification par chromatographie sur gel de silice (cyclohexane-acétate d'éthyle : 8-2).
RMN 1 H DMSO δ ppm : 2.39(s, 6H), 2.22(s, 6H), 7.58(s, 2H), 7.67-7.62(m, 3H), 7.82(d, J = 15,5 Hz, 1 H), 8.17(d, 1H), 12.96(s, 1H)

### Composé intermédiaire 4 : 1-[4-hexyloxyphényl] -3-[3,5-diméthyl-4-hydroxyphényl]prop-2-èn-1-one:

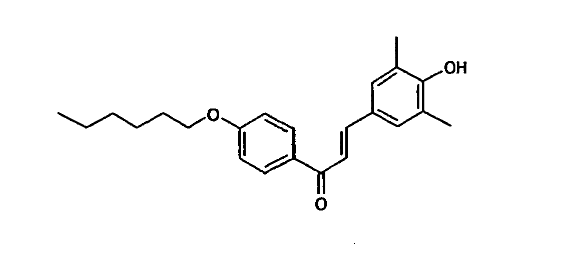

Ce composé est synthétisé à partir de 4-hexyloxyacétophénone et de 3,5-diméthyl-4-hydroxybenzaldéhyde selon la méthode générale 1 précédemment décrite.

Le composé attendu précipite dans le milieu réactionnel, il est essoré puis est utilisé sans autre purification pour la réaction suivante.
RM N 1 H DMSO δ ppm : 0.88(m, 3H), 1.28-1.43(m, 6H), 1.72(m, 2H), 2.21(s, 6H), 4.05(t, J = 6.42Hz, 2H), 7.40(d, J = 8.43Hz, 2H), 7.48(s, 2H), 7.57(d, J = 15.24Hz, 1 H), 7.72(d, J = 15.24Hz, 1H), 8.12(d, J = 8.43Hz, 2H), 8.89(s, 1H)

### Composé intermédiaire 5 : 1-[2-hydroxy-4-chlorophényl] -3-[3,5-diméthyl-4-hydroxyphényl]prop-2-èn-1-one

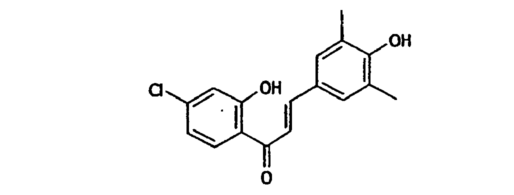

Ce composé est synthétisé à partir de 4'-chloro-2'-hydroxyacétophénone (matière première 3) et de 3,5-diméthyl-4-hydroxybenzaldéhyde selon la méthode générale 1 précédemment décrite.
Purification par chromatographie sur gel de silice (toluène : 10).
RMN 1 H DMSO δppm : 2.21(s, 6H), 7.1(m, 2H), 7.55(s, 2H), 7.72(d, J = 15.4Hz, 1H), 7.80(d, J = 15.4Hz, 1H), 8.25(d, J=9.0Hz, 1H), 9.09(s, 1H), 13.04(s, 1 H)

### Composé intermédiaire 6 : 2-(3,5-diméthyl-4-hydroxyahényl)-7-chloro-4H-1-benzopyran-4-one

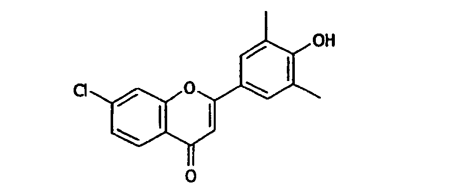

Ce composé est synthétisé à partir de 1-[2-hydroxy-4-chlorophényl]-3-[3,5-diméthyl-4-hydroxyphényl]prop-2-èn-1-one (composé intermédiaire 5) selon la méthode suivante :

La 1-[2-hydroxy-4-chlorophényl]-3-[3,5-diméthyl-4-hydroxyphényl]prop-2-èn-1-one est solubilisée dans du diméthylsulfoxyde, un cristal d'iode est ajouté, l'ensemble est maintenu 10 minutes à reflux.

Le milieu réactionnel est ramené à température ambiante, hydrolysé. Le précipité est essoré, rincé avec une solution de thiosulfate de sodium puis avec de l'eau.
Purification par dissolution dans le chlorure de méthylène et précipitation par addition d'heptane.
RMN 1 H DMSO δppm : 2.25(s, 6H), 6.87(s, 1H), 7.51 (d, J = 8.55Hz, 1H), 7.73(s, 2H), 7.98(m, 2H)
Bibliographie : Doshi AG, S. P., Ghiya BJ (1986). Indian J Chem Sect B **25**: 759.

### Composé intermédiaire 7 : 1-[2-méthyloxy-4-chlorophényl] -3-[3,5-diméthyl-4-hydroxydiméthylméthloxyphényl]prop-2-èn-1-one :

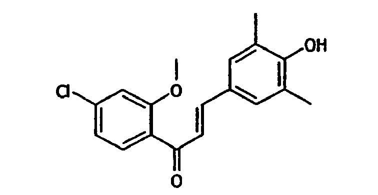

Ce composé est synthétisé à partir de 4'-chloro-2'-méthoxyacétophénone et de 3,5-diméthyl-4-hydroxybenzaldéhyde selon la méthode générale 1 précédemment décrite.
Purification par chromatographie sur gel de silice (cyclohexane-acétate d'éthyle : 85-15).
RMN 1 H DMSO δ ppm : 2.21(s, 6H), 3.90(s, 3H), 7.12(m, 1H), 7.23(d, J = 15.5Hz, 1H), 7.29(s, J = 1.80Hz, 1H), 7.38(d, J = 15.5 Hz, 1H), 7.41 (s, 2H), 7.48(d, J = 7.98Hz, 1 H)

### Composé intermédiaire 8: 1-[4-bromophényl]-3-[3,5-diméthyl-4-hydroxyphényl]prop-2-èn-1-one :

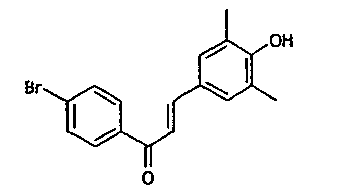

Ce composé est synthétisé à partir de 4'-bromoacétophénone et de 3,5-diméthyl-4-hydroxybenzaldéhyde selon la méthode générale 1 précédemment décrite.
Purification par chromatographie sur gel de silice (cyclohexane-acétate d'éthyle : 85-15).
RMN 1H DMSO δ ppm : 2,30(s, 6H), 7.32 (s, 2H), 7.56-7.66(m, 3H), 7.75(d, J = 15.27Hz, 1H), 7.90(d, J = 8.70Hz, 2H), 9.82(s, 1 H)

### Composé intermédiaire 9 : 1-[4-heptylphényl] -3-[3,5-diméthyl-4-hydroxyphényl]prop-2-èn-1-one :

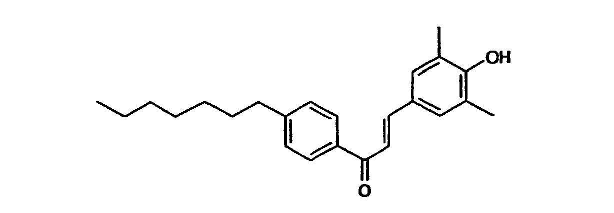

Ce composé est synthétisé à partir de 4'-heptylacétophénone et de 3,5-diméthyl-4-hydroxybenzaldéhyde selon la méthode générale 1 précédemment décrite.
Purification par chromatographie sur gel de silice (cyclohexane-acétate d'éthyle : 85-15).
RMN 1H DMSO δ ppm : 0.84 (m, 3H), 1.25(m, 8H), 1.60(m, 2H), 2.21(s, 6H), 2.65(t, J = 7.50Hz, 2H), 7.35(d, J = 8.02Hz, 2H), 7.48(s, 2H), 7.60(d, J = 15.48Hz, 1 H), 7.71 (d, J = 15.48Hz, 1 H), 8.05(d, J = 8.02Hz, 2H), 8.92(s, 1 H)

### Synthèse des composés selon l'invention :

### Composé 1: 1-[2-hydroxy-4-éthoxycarbonyldiméthylméthyloxyphényl] -3-[3,5-ditertiobutyl-4-hydroxyphényl]prop-2-èn-1-one

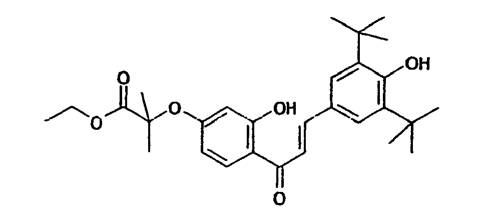

Ce composé est synthétisé à partir de 2'-Hydroxy-4'-(éthoxycarbonyldiméthylméthoxy)acétophénone (matière première 1) et de 3,5-ditertiobutyl-4-hydroxybenzaldéhyde selon la méthode générale 1 précédemment décrite.
Purification par chromatographie sur gel de silice (cyclohexane-acétate d'éthyle : 9-1).
RMN 1 H CDCl₃ δ ppm : 1.25(t, J = 7.11Hz, 3H), 1.45(s, 18H), 1.70(s, 6H), 4.26(q, J = 7.11 Hz, 2H), 5.63(s, 1 H), 6.33(d, J = 2.37Hz, 1 H), 6.42(dd, J = 8.8Hz, J = 2.37Hz, 1H), 7.41 (d, J = 15.39Hz, 1 H), 7.5(s, 2H), 7.83(d, J = 8.8Hz, 1H), 7.88(J = 15.39Hz, 1H), 13.5(s, 1H)

### Composé 2 : 1-[2-hydroxy-4-carboxydiméthylméthyloxyphényl] -3-[3,5-ditertiobutyl-4-hydroxyphényl]prop-2-èn-1-one:

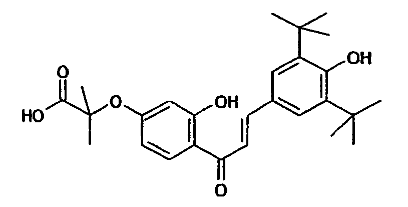

Ce composé est synthétisé à partir de 1-[2-hydroxy-4-éthoxycarbonyldiméthylméthyloxyphényl]-3-[3,5-ditertiobutyl-4-hydroxyphényl] prop-2-èn-1-one (composé 1) selon la méthode suivante :
L'ester est solubilisé dans l'éthanol, une solution aqueuse de soude 1N (5 éq) est ajoutée, l'ensemble est maintenu 10 heures à reflux. Le milieu est acidifié par addition d"acide chlorhydrique (12N) puis extrait par de l'acétate d'éthyle. La phase organique est séchée sur du sulfate de magnésium puis évaporée sous pression réduite.
Purification par HPLC préparative (phase inverse RP18, licrospher 12µm, élution : eau - méthanol - acide trifluoroacétique : 22-78-0.1).
RMN 1H CDCl₃ δ ppm : 1.49(s, 18H), 1.73(s, 6H), 5.62(s, 1H), 6.44(d, J = 15.5 Hz, 1H), 7.01 (m, 2H), 7.57(t, 1H), 7.81 (d, J= 15.5Hz, 1 H), 7.87(d, 2H), 7.93(d, 1 H), 8.26(d, 1 H)
SM(ES-MS) : 453.2 (M-1)

### Composé 5: 1-[2-hydroxyphényl] -3-[3,5-diméthoxy-4-carboxydiméthylméthyloxyphényl]prop-2-èn-1-one :

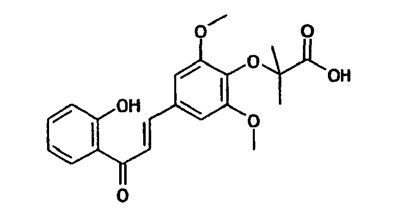

Ce composé est synthétisé à partir de 2'-hydroxyacétophénone et de 3,5-diméthyloxy-4-éthyloxycarbonyldiméthylméthyloxybenzaldéhyde (matière première 5) selon la méthode générale 2 précédemment décrite.
Purification par chromatographie sur gel de silice (élution : cyclohexane-acétate d'éthyle : 9-1).
RMN 1H DMSO δ ppm : 1.35(s, 6H), 3.80(s, 6H), 7.00-7.03(m, 2H), 7.25(s, 2H), 7.59(t, 1 H,J = 8,07 Hz, 1H), 7.81 (d, J = 15,5 Hz, 1H), 8.00(d, J = 15,5 Hz, 1H), 8.31 (d, J = 8,07 Hz, 1H), 12.36(s, 1 H), 12.69(s, 1H)
SM(ES-MS) : 385.3 (M-1)

### Composé 6 : 1-[2-hydroxy-4-chlorophényl] -3-[3,5-diméthoxy-4-carboxydiméthylméthyloxyphényl]prop-2-èn-1-one :

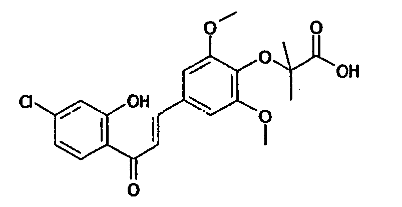

Ce composé est synthétisé à partir de 2'-hydroxy-4'-chloroacétophénone (matière première 3) et de 3,5-diméthyloxy-4-éthyloxycarbonyldirnéthylméthyloxybenzaldéhyde (matière première 5) selon la méthode générale 2 précédemment décrite.
Purification par chromatographie sur gel de silice (élution : cyclohexane-acétate d'éthyle : 9-1).
RMN 1 H DMSO δ ppm : 1.34(s, 6H), 3.80(s, 6H), 7.08(dd, J = 1.77Hz, 1H), 7.12(d, J = 1.77Hz,1H), 7.24(s, 2H), 7.79(d, J = 15,4 Hz, 1H), 7.93(d, J = 15,4 Hz, 1H), 8.27(d, J = 8,3 Hz, 1H), 12.36(s, 1 H), 12.69(s, 1H)
SM(ES-MS) : 419.0 (M-1)

### Composé 7 : 1-[2-hydroxy-4-chlorophényl]-3-[3,5-diméthyl-4-carboxydiméthyl méthyloxyphényl]prop-2-èn-1-one :

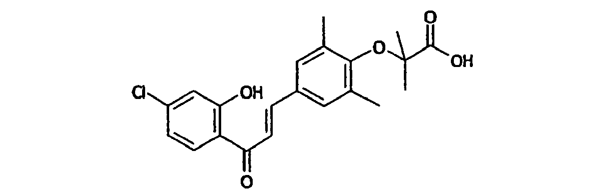

Ce composé est synthétisé à partir de 2'-hydroxy-4'-chloroacétophénone (matière première 3) et de 3,5-diméthyl-4-éthyloxycarbonyldiméthylméthyloxybenzaldéhyde selon (matière première 6) la méthode générale 2 précédemment décrite.
Purification par chromatographie sur gel de silice (élution : cyclohexane-acétate d'éthyle : 9-1).
RMN 1H DMSO δ ppm : 1.39(s, 6H), 2.22(s, 6H), 7.07(m, 1H), 7.12(d, J= 2,07 Hz, 1H), 7.61(s, 2H), 7.74(d, J= 15,5 Hz, 1 H), 7.87(d, J = 15,5 Hz, 1H), 8.26(d, 1 H), 12.76(s, 1H)
SM(ES-MS) : 387.1 (M-1)

### Composé 8 : 1-[2-hydroxy-4-carboxydiméthylméthyloxyphényl]-3-[3,5-dibromo-4-hydroxyphényl]prop-2-èn-1-one :

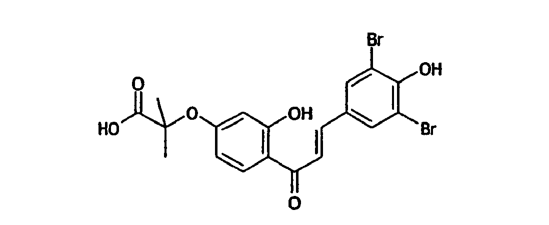

Ce composé est synthétisé à partir de 2'-hydroxy-4'-éthyl oxycarbonyldiméthylméthyloxyacétophénone (matière première 1) et de 3,5-dibromo-4-hydroxybenzaldéhyde selon la méthode générale 2 précédemment décrite.
Purification par chromatographie sur gel de silice (élution : cyclohexane-acétate d'éthyle : 9-1).
RMN 1 H CDCl₃ δ ppm : 1,60(s, 6H), 6.24(d, J = 2.47 Hz, 1H), 6.43(dd, J = 2.47, J = 8.52Hz, 1H), 7.70 (d, J = 15.5 Hz, 1 H), 7.96 (d, J = 15.5 Hz, 1 H), 8.22(s, 2H), 8.34(d, J = 9.16 Hz, 1 H), 13.34(s, 1 H)
SM(ES-MS) : 498.6 (M-1)

### Composé 11 : 1-[2-hydroxyphényl] -3-[3,5-diméthyl-4-carboxydiméthylméthyloxyphényl]prop-2-èn-1-one :

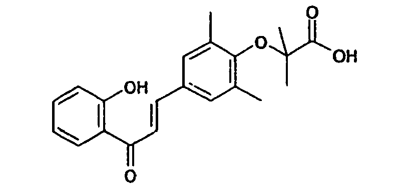

Ce composé est synthétisé à partir de 2'-hydroxyacétophénone et de 3,5-diméthyl-4-éthyloxycarbonyldiméthylméthyloxybenzaldéhyde (matière première 6) selon la méthode générale 2 précédemment décrite.
Purification par chromatographie sur gel de silice (élution : cyclohexane-acétate d'éthyle : 9-1) puis par HPLC préparative (phase inverse RP18, licrospher 12µm, élution : eau - méthanol - acide trifluoroacétique : 22-78-0.1).
RMN 1H DMSO δ ppm : 1.57 (s, 6H), 2.31 (s, 6H), 6.96(t, J= 8,17Hz, 1H), 7.04(d, J= 8,72 Hz, 1H), 7.35(s, 2H), 7.49(t, J= 8,2 Hz, 1H), 7.58(d, J= 15,8Hz, 1H), 7.84(d, J= 15,8Hz, 1 H), 7.94(d, J= 8,7Hz, 1 H), 12.87(s, 1H)
SM(ES-MS) : 353.1 (M-1)

### Composé 12 : 1-[2-hydroxy-4-carboxydiméthylméthyloxyphényl] -3-[4-méthylthiophényl]prop-2-èn-1-one :

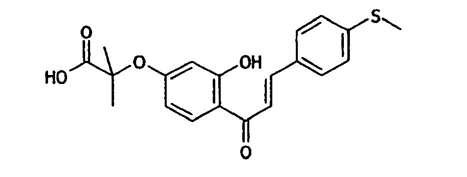

Ce composé est synthétisé à partir de 2'-hydroxy-4'-éthyloxycarbonyldiméthylméthyloxyacétophénone (matière première 1) et de 4-méthylthiobenzaldéhyde selon la méthode générale 2 précédemment décrite. Purification par chromatographie sur gel de silice (élution : cyclohexane-acétate d'éthyle : 9-1) puis HPLC préparative (phase inverse RP18, licrospher 12µm, élution : eau - méthanol - acide trifluoroacétique : 22-78-0.3).
RM N 1H DMSO δ ppm : 1.60 (s, 6H), 2.54(s, 3H), 6.25(d, 1H), 6.43(dd, J= 2.47 Hz, 1H), 7.33(d, J= 8.56 Hz, 2H), 7.8(d, 15.5Hz, 1H), 7.86(d, J = 8.56Hz, 2H ), 7.98(d, J = 15.5 Hz, 1 H), 8.29(d, J = 9.1 Hz, 1H), 13.34(s, 1 H)
SM(ES-MS) : 373.1 (M-1)

### Composé 15 : 1-[4-chlorophényl]-3-[3,5-diméthyl-4-isopropyloxycarbonyldiméthylméthyloxyphényl]prop-2-èn-1-one :

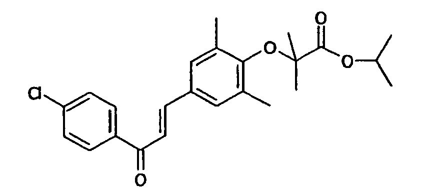

Ce composé est synthétisé à partir de 1-[4-chlorophényl]-3-[3,5-diméthyl-4-hydroxyphényl]prop-2-èn-1-one (composé intermédiaire 1) et de bromoisobutyrate d'isopropyle selon la méthode générale 4 précédemment décrite.
Purification par chromatographie sur gel de silice (élution : cyclohexane-acétate d'éthyle : 9-1)
RMN 1H DMSO δ ppm : 1.25(d, J = 6.06Hz, 6H), 1.39(s, 6H), 5.00(sept, J = 6.06Hz, 1H), 7.57(s, 2H), 7.62(d, J = 8.40Hz, 2H), 7.64(d, J = 15.8Hz, 1H), 7.81(d, J = 15.8, 1H), 8.16(d, J = 8.40Hz, 2H).
SM(Maldi-Tof) : 415.1(M+1)

### Composé 16 : 1-[4-chlorophényl]-3-[3,5-diméthyl-4-tertiobutyloxycarbonyldiméthylméthyloxyahényl]prop-2-èn-1-one :

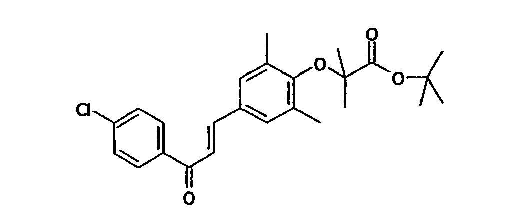

Ce composé est synthétisé à partir de 1-[4-chlorophényl]-3-[3,5-diméthyl-4-hydroxyphényl]prop-2-èn-1-one (composé intermédiaire 1) et de bromoisobutyrate de tertiobutyle selon la méthode générale 4 précédemment décrite.
Purification par chromatographie sur gel de silice (élution : cyclohexane-acétate d'éthyle : 9-1).

### Composé 17 : 1-[4-chlorophényl] -3-[3,5-diméthyl-4-carboxydiméthylméthyloxyphényl]prop-2-èn-1-one :

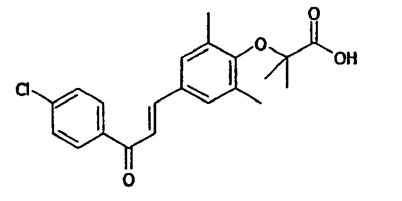

Ce composé est synthétisé à partir de 1-[4-chlorophényl]-3-[3,5-diméthyl-4-tertiobutyloxycarbonyldiméthylméthyloxyphényl]prop-2-èn-1-one (composé 16) selon la méthode générale 5 précédemment décrite.
Purification par chromatographie sur gel de silice (élution : dichlorométhane-méthanol : 98-2)
RMN 1H DMSO δ ppm : 1.39(s, 6H), 2.22(s, 6H), 7.58(s, 2H), 7.67-7.62(m, 3H), 7.82(d, J = 15,5 Hz, 1 H), 8.17(d, 1H), 12.96(s, 1 H)
SM(Maldi-Tof) : 373.3(M+1)

### Composé 19 : 1-[2-hydroxyphényl]-3-[4-carboxydiméthylméthyl thiophényl]prop-2-èn-1-one

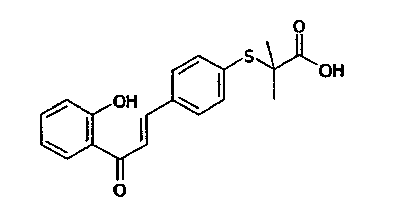

Ce composé est synthétisé à partir de 2'-hydroxyacétophénone et éthyloxycarbonyldiméthylméthylthiobenzaldéhyde (matière première 8) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.44(s, 6H), 6.99-7.05(m, 1 H), 7.52(d, J = 8.1 Hz, 2H), 7.58(m, 1H), 7.83(d, J = 15.5Hz, 1H), 7.92(d, J = 8.1 Hz, 1H), 8.09(d, J = 15.5Hz, 1 H), 8.26(dd, J = 1.62, J = 8.6 Hz, 1 H), 12.47(s, 1 H), 12.78(s, 1 H) SM(Maldi-Tof): 242.9 (M+1)

### Composé 20 : 1-[4-chloro-2-hydroxvphényl] -3-[4-carboxydiméthylméthylthiophényl]prop-2-èn-1-one

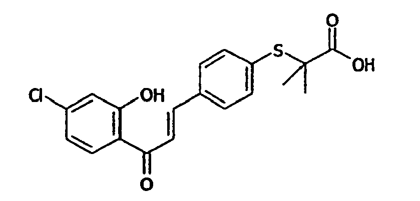

Ce composé est synthétisé à partir de 4'-chloro-2'-hydroxyacétophénone (matière première 3) et de 4-éthyloxycarbonyldiméthylméthylthiobenzaldéhyde (matière première 8) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.43(s, 6H), 7.05(dd, J = 1,7Hz, J = 8,46Hz, 1H), 7.11 (d, J = 2,25Hz, 1H), 7.51(d, J = 7,92Hz, 2H), 7.82(d, J = 15,8 Hz, 1H), 7.89(d, J = 7,9Hz , 2H), 8.05(d, J = 15,2Hz, 1H), 8.23(d, J = 8,46 Hz, 1H), 12.57(s, 1H), 12.78(s, 1H).
SM(Maldi-Tof) : 377.0(M-1)

### Composé 21 : 1-[4-carboxydiméthylméthyloxyphényl] -3-[3,5-diméthyl4-hydroxyphényl]prop-2-èn-1-one

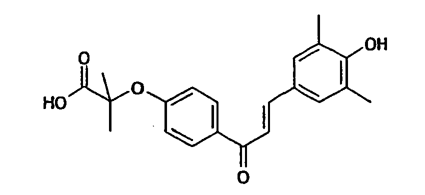

Ce composé est synthétisé à partir de 4-éthyloxycarbonyldiméthylméthyloxy acétophénone (matière première 9) et de 3,5-diméthyl-4-hydroxybenzaldéhyde selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.60(s, 6H), 2.21 (s, 6H), 6.91 (d, J = 9.09Hz, 2H), 7.48(s, 2H), 7.57(d, J = 15.12Hz, 1H), 7.70(d, J = 15.63Hz, 1H), 8.09(d, J = 9,06Hz, 2H), 8.9(s, 1H), 13.29(s, 1H)
SM(Maldi-Tof) : 355.2 (M+1)

### Composé 22 : 1-[4-méthylthiophényl] -3-[4-carboxydiméthylméthyloxyphényl]prop-2-èn-1-one

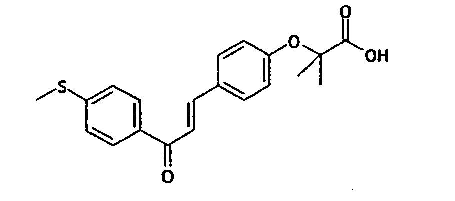

Ce composé est synthétisé à partir de 4'-méthylthioacétophénone (matière première 12) et de 4-éthyloxycarbonyldiméthylméthyloxybenzaldéhyde (matière première 9) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.57(s, 6H), 2.57(s, 3H), 6.86(d, J = 8,94Hz, 2H), 7.41 (d, J = 8,40Hz, 2H), 7.69(d, J = 15,2Hz, 1H), 7.84-7.78(m, 3H), 8.09(d, J = 8,4Hz, 2H), 13.21(s, 1H)
SM(Maldi-Tof) : 357.2 (M-1)

### Composé 24: 1-[4-carboxydiméthylméthylthiophényl] -3-[4-méthylthiophényl]prop-2-èn-1-one :

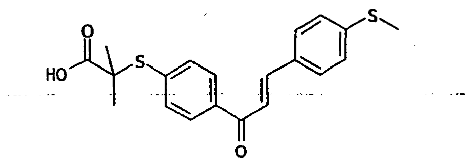

Ce composé est synthétisé à partir de 4-éthyloxycarbonyl diméthylméthylthiocétophénone (matière première 12) et de 4-méthylthiobenzaldéhyde selon la méthode générale 3 précédemment décrite. Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RM N 1H DMSO δ ppm : 1,46 (s, 6H), 2.54 (s, 3H), 7.33 (d, J = 8,61 Hz, 2H), 7.59 (d, J = 8.10Hz, 2H), 7.73 (d, J = 15.66Hz, 1 H), 7.85 (d, J = 8.10Hz, 2H), 7.92 (d, J = 15.66Hz, 1H), 8.13 (d , 8.10Hz, 2H), 12.85 (s, 1H)
SM(Maldi-Tof) : 373.1 (M+1)

### Composé 25 : 1-[2-hydroxy-4-bromophényl] -3-[3,5-diméthyl-4-carboxydiméthylméthyloxyphényl]prop-2-èn-1-one

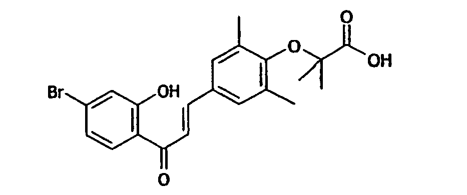

Ce composé est synthétisé à partir de 4'-bromo-2'-hydroxyacétophénone (matière première 11) et 3,5-diméthyl-4-éthyloxycarbonyldiméthyloxybenzaldéhyde (matière première 6) selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthane) - acide trifluoroacétique : 22-78-0.1).
RMN 1H DMSO δ ppm : 1.39(s, 6H), 2.22(s, 6H), 7.20(dd, J = 2.16, J = 8.55Hz, 1H), 7.25(d, J = 1.59Hz, 1H), 7.60(s, 2H), 7.73(d, J = 15.51Hz, 1H), 7.86(d, J = 15,51 Hz, 1H), 8.16(d, J = 8,58Hz, 1 H), 12.70(s, 1 H), 13.30(s, 1 H)
SM(ES-MS) : 432.9 (M-1)

### Composé 26 : 1-[4-carboxycriméthylméthyloxyphényl] -3-[4-méthylthiophényl]prop-2-èn-1-one :

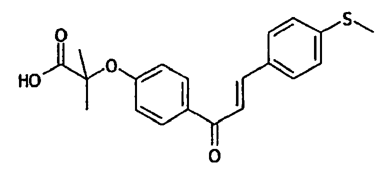

Ce composé est synthétisé à partir de 4'-éthyloxycarbonyldiméthylméthyloxyacétophénone (matière première 9) et de 4-méthylthiobenzaldéhyde selon la méthode générale 2 précédemment décrite.
Purification par chromatographie sur gel de silice (élution : dichlorométhane-méthanol : 95-5) puis par HPLC préparative (phase inverse RP18, licrospher 12µm, élution : eau - méthanol - acide trifluoroacétique : 22-78-0.1).
RMN 1H DMSO δ ppm : 1.60(s, 6H), 2.53(s, 3H), 6.93(d, J = 9.00Hz, 2H), 7.32(d, J = 8.49Hz, 2H), 7.68(d, 15.51 Hz, 1H), 7.82(d, J = 8.52Hz, 2H), 7.89(d, J = 15.51 Hz, 1 H), 8.13(d, 9.00Hz, 2H), 13.30(s, 1 H)
SM(Maldi-Tof) : 355.0(M+1)

### Composé 27: 1-[4-méthylthiophényl]-3-[3,5-diméthyl-4-tertiobutyloxycarbonyldiméthylméthyloxyphényl]prop-2-èn-1-one :

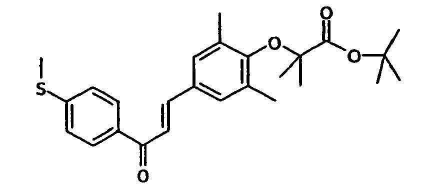

Ce composé est synthétisé à partir de 1-[4-méthylthiophényl]-3-[3,5-diméthyl-4-hydroxyphényl]prop-2-èn-1-one (composé intermédiaire 2) et de bromoisobutyrate de tertiobutyle selon la méthode générale 4 précédemment décrite.
Purification par chromatographie sur gel de silice (élution : cyclohexane-acétate d'éthyle : 8-2).

### Composé 28 : 1-[4-méthylthiophényl]-3-[3,5-diméthyl-4-isopropyloxycarbonyldiméthylméthyloxyphényl]prop-2-èn-1-one :

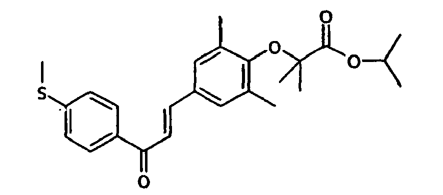

Ce composé est synthétisé à partir de 1-[4-méthylthiophényl]-3-[3,5-diméthyl-4-hydroxyphényl]prop-2-èn-1-one (composé intermédiaire 2) et de bromoisobutyrate d'isopropyle selon la méthode générale 4 précédemment décrite.
Purification par chromatographie sur gel de silice (élution : cyclohexane-acétate d'éthyle : 9-1).
RMN 1H DMSO δ ppm : 1.25(d, J = 6.18Hz, 6H), 1.39(s, 6H), 2.18(s, 6H), 2.57(s, 3H), 4.99(sept, J = 6.18Hz, 1H), 7.40(d, J = 8.28Hz, 2H), 7.58(s, 2H), 7.62(d, J = 15.5Hz, 1H), 7.82(d, J = 15.5Hz, 1H); 8.10(d, J = 8.28Hz, 2H), 12.97(s, 1H)
SM(Maldi-Tof) : 427.1 (M+1)

### Composé 29 : 1-[4-méthylthiophényl] -3-[3,5-diméthyl-4-carboxydiméthylméthyloxyphényl]prop-2-èn-1-one:

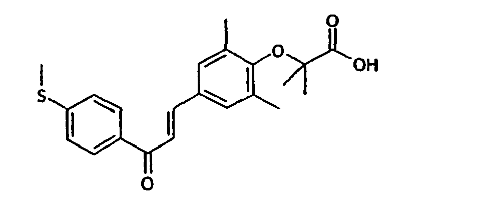

Ce composé est synthétisé à partir de 1-[4-méthylthiophényl]-3-[3,5-diméthyl-4-tertiobutyloxycarbonyldiméthylméthyloxyphényl]prop-2-èn-1-one (composé 28) selon la méthode générale 5 précédemment décrite.
Purification par chromatographie sur gel de silice (élution : dichlorométhane -méthanol : 98-2).
RMN 1H DMSO δ ppm : 1.39(s, 6H), 2.22(s, 6H), 2.57(s, 3H), 7.40(d, J = 8.55Hz, 2H), 7.57(s, 2H), 7.62(d, J = 15.5Hz, 1H), 7.83(d, J = 15.5Hz, 1H), 8.10(d, J = 8.55Hz, 2H), 12.97(s, 1H)
SM(ES-MS) : 383.3(M-1)

### Composé 30 : 1-[2-méthoxyphényl]-3-[3,5-diméthyl-4-tertiobutyloxycarbonyldiméthylméthyloxyphényl]prop-2-èn-1-one :

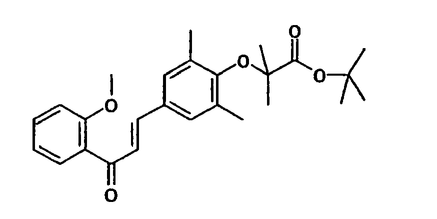

Ce composé est synthétisé à partir de 1-[2-méthoxyphényl]-3-[3,5-diméthy 1-4-hydroxyphényl]prop-2-èn-1-one (composé intermédiaire 3) et de bromoisobutyrate de tertiobutyle selon la méthode générale 4 précédemment décrite.
Purification par chromatographie sur gel de silice (élution : cyclohexane-acétate d'éthyle : 9-1).

### Composé 31 : 1-[2-méthoxyphényl] -3-[3,5-diméthyl-4-carboxydiméthylméthyloxyphényl]prop-2-èn-1-one :

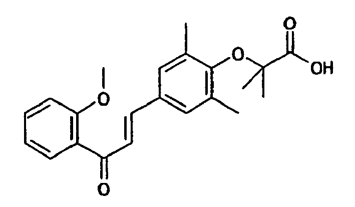

Ce composé est synthétisé à partir de 1-[2-méthoxyphényl]-3-[3,5-diméthyl-4-tertiobutyloxycarbonytdiméthylméthyloxyphénylJprop-2-èn-1-one (composé 30) selon la méthode générale 5 précédemment décrite.
Purification par chromatographie sur gel de silice (élution : dichlorométhane-méthanol : 98-2).
RMN 1H DMSO δ ppm : 1.38(s, 6H), 2.19(s, 6H), 3.93(s, 3H), 7.05(m, 1 H), 7.20(d, J = 8.31 Hz, 1H), 7.25(d, J = 15.5Hz, 1H), 7.37(d, J = 15.5Hz, 1H), 7.39(s, 2H), 7.46(d, J = 7.2Hz, 1 H), 7.53(m, 1 H), 12.93(s, 1 H)
SM(ES-MS) : 367.1(M-1)

### Composé 32 : 1-[4-hexyloxyphényl] -3-[3,5-diméthyl-4-tertiobutyloxycarbonyldiméthylméthyloxyphényl]prop-2-èn-1-one :

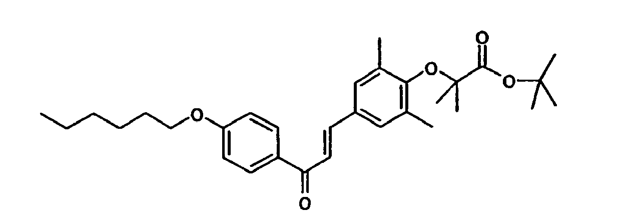

Ce composé est synthétisé à partir de 1-[4-hexyloxyphényl]-3-[3,5-diméthyl-4-hydroxyphényl]prop-2-èn-1-one (composé intermédiaire 4) de bromoisobutyrate de tertiobutyle selon la méthode générale 4 précédemment décrite.
Purification par chromatographie sur gel de silice (élution : cyclohexane-acétate d'éthyle : 95-5)

### Composé 33: 1-[4-hexyloxyphényl] -3-[3,5-diméthyl-4-carboxydiméthylméthyloxyphényl]prop-2-èn-1-one :

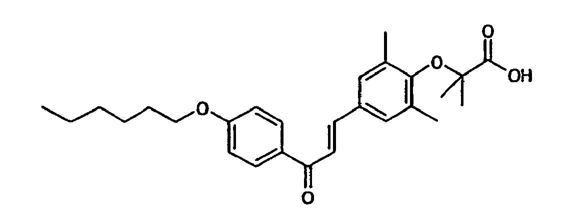

Ce composé est synthétisé à partir de 1-[4-héxyloxyphényl]-3-[3,5-diméthyl-4-tertiobutyloxycarbonyldiméthylméthyloxyphényl]prop-2-èn-1-one (composé 32) selon la méthode générale 5 précédemment décrite.
Purification par recristallisation dans le méthanol.
RM N 1H DMSO δ ppm : 0.88(t, J = 6.33Hz, 3H), 1.30(m, 4H), 1.39(s, 6H), 1.44(m, 2H), 1.73(m, 2H), 2.22(s, 6H), 4.06(t, J = 6.30Hz, 2H), 7.06(d, J = 8.61Hz, 2H), 7.56(s, 2H), 7.58(d, J = 15.5Hz, 1H), 7.82(d, J = 15.5Hz, 1H), 8.13(d, J = 6.61 Hz, 2H)
SM(ES-MS) : 437.2(M-1)

### Composé 34: 2-(3,5-diméthyl-4-tertiobutyloxycarbonyldiméthylméthyloxyphényl)-7-chloro-4H-1-benzopyran-4-one :

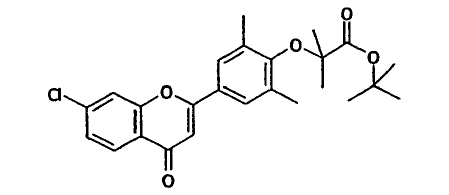

Ce composé est synthétisé à partir de 2-(3,5-diméthyl-4-hydroxyphényl)-7-chloro-4H-1-benzopyran-4-one (composé intermédiaire 6) et de bromoisobutyrate de tertiobutyle selon la méthode générale 4 précédemment décrite.
Purification par précipitation dans le mélange de solvants
dichlorométhane/heptane.

### Composé 35 : 2-(3,5-diméthyl-4-carboxydiméthylméthyloxyphényl)-7-chloro-4H-1-benzopyran-4-one

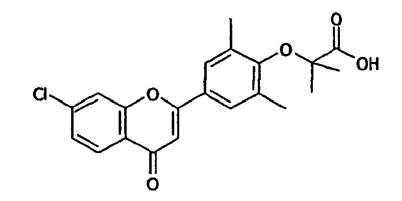

Ce composé est synthétisé à partir de 2-(3,5-diméthyl-4-tertiobutyloxycarbonyldiméthylméthyloxyphényl)-7-chloro-4H-1-benzopyran-4-one (composé 34) selon la méthode générale 5 précédemment décrite.
Purification par HPLC préparative (phase inverse RP18, licrospher 12µm, élution : eau - méthanol - acide trifluoroacétique : 22-78-0.1).
RMN 1H DMSO δ ppm : 1.24(s, 6H), 2.28(s, 6H), 7.02(s, 1H), 7.56(dd, J = 8.71 Hz, J = 1.75Hz, 1H), 7.85(s, 2H), 8.03(d, J = 1.75Hz, 1H), 8.06(d, J = 8.71 Hz, 1H)
SM(Maldi-Tof): 387.1(M+1)

### Composé 36: 1-[2-méthyloxy-4-chlorophényl] -3-[3,5-diméthyl-4-tertiobutyloxycarbonyldiméthylméthyloxyphényl]prop-2-èn-1-one :

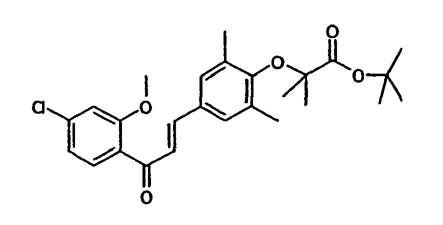

Ce composé est synthétisé à partir de 1-[2-méthyloxy-4-chlorophényl]-3-[3,5-diméthyl-4-hydroxydiméthylméthyloxyphényl]prop-2-èn-1-one (composé intermédiaire 7) et de bromoisobutyrate de tertiobutyle selon la méthode générale 4 précédemment décrite.
Purification par chromatographie sur gel de silice (élution : cyclohexane-acétate d'éthyle : 9-1)

### Composé 37 : 1-[2-méthyloxy-4-chlorophényl] -3-[3,5-diméthyl-4-carboxydiméthylméthyloxyphényl]prop-2-èn-1-one :

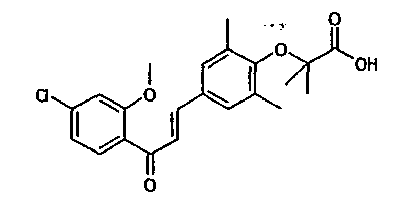

Ce composé est synthétisé à partir de 1-[2-méthoxy-4-chlorophényl]-3-[3,5-diméthyl-4-tertiobutyloxycarbonyldiméthylméthyloxyphényl]prop-2-èn-1-one (composé 36) selon la méthode générale 5 précédemment décrite.
Purification par chromatographie sur gel de silice (élution : dichlorométhane/méthanol : 98-2)
RMN 1H DMSO δ ppm : 1.38(s, 6H), 2.19(s, 6H), 3.89(s, 3H), 7.12(dd, J = 7.98, J = 1.71 Hz, 1H), 7.23(d, J = 15.56Hz, 1 H), 7.29(s, J = 1.71 Hz, 1 H), 7.38(d, J = 15.7 Hz, 1 H), 7.41 (s, 2H), 7.48(d, J = 7.98Hz, 1H)
SM(ES-SM) : 401.2(M-1)

### Composé 38 : 1-[4-heptylphényl] -3-[3,5-diméthyl-4-tertiobutyloxycarbonyldiméthylméthyloxyphényl]prop-2-èn-1-one :

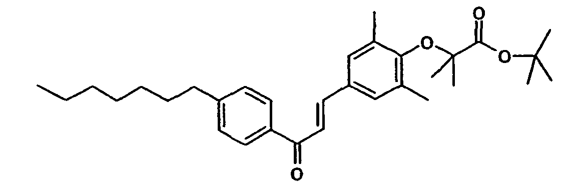

Ce composé est synthétisé à partir de 1-[4-heptylphényl]-3-[3,5-diméthyl-4-hydroxyphényl]prop-2-èn-1-one (composé intermédiaire 9) et de bromoisobutyrate de tertiobutyle selon la méthode générale 4 précédemment décrite.
Purification par chromatographie sur gel de silice (élution : cyclohexane-acétate d'éthyle : 9-1)

### Composé 39 : 1-[4-heptylphényl] -3-(3,5-diméthyl-4-carboxydiméthylméthyloxychényl]prop-2-èn-1-one:

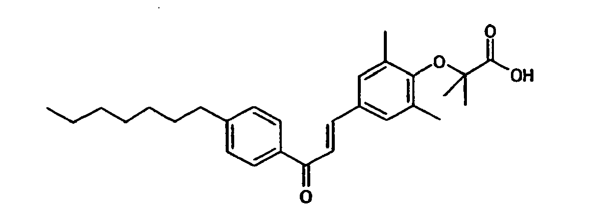

Ce composé est synthétisé à partir de 1-[4-heptylphényl]-3-[3,5-diméthyl-4-tertiobutyloxycarbonyldiméthylméthyloxyphényl]prop-2-èn-1-one (composé 38) et de bromoisobutyrate de tertiobutyle selon la méthode générale 4 précédemment décrite.
Purification par chromatographie sur gel de silice (élution : dichlorométhane/méthanol : 98-2)
RMN 1H DMSO δ ppm : 0.85(m, 3H), 1.30-1.24(m, 8H), 1.39(s, 6H), 1.60(m, 2H), 2.22(s, 6H), 2.67(t, 2H, J = 7.4Hz), 7.37(d, J = 8.04 Hz, 2H), 7.57(s, 2H), 7.62(d, J = 15.66 Hz, 1H), 7.82(d, J = 15.69 Hz, 1H), 8.07(d, J = 8.07 Hz, 2H)
SM(ES-MS) : 435.3(M-1)

### Composé 40 : 1-[4-bromophényl]-3-(3,5-diméthyl-4-tertiobutyloxycarbonyldiméthvlméthyloxyphényllprop-2-èn-1-one :

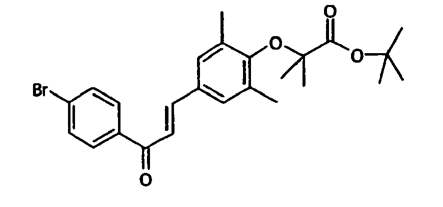

Ce composé est synthétisé à partir de 1-[4-bromophényl]-3-[3,5-diméthyl-4-hydroxyphényl]prop-2-èn-1-one (composé intermédiaire 8) et de bromoisobutyrate de tertiobutyle selon la méthode générale 4 précédemment décrite.
Purification par chromatographie sur gel de silice (élution : cyclohexane-acétate d'éthyle : 9-1)

### Composé 41 : 1-[4-bromophényl]-3-[3,5-diméthyl-4-carboxy diméthylméthyloxyphényllprop-2-èn-1-one :

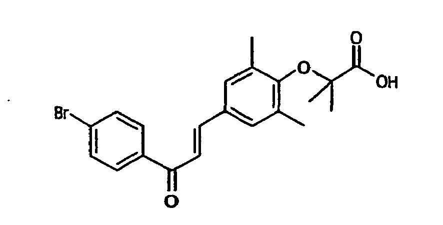

Ce composé est synthétisé à partir de 1-[4-bromophényl]-3-[3,5-diméthyl-4-tertiobutyloxycarbonyldiméthylméthyloxyphényl]prop-2-èn-1-one (composé 40) selon la méthode générale 5 précédemment décrite.
Purification par chromatographie sur gel de silice (élution : dichlorométhane-méthanol : 98-2)
RMN 1H DMSO δ ppm : 1.39 (s, 6H), 2.22 (s, 6H), 7.58 (s, 2H), 7.65 (d, J = 15.39Hz, 1H), 7.84-7.77 (m, 3H), 8.09 (d, J = 8.19Hz, 1H), 13.01 (s, 1H)
SM(ES-MS) : 417.2(M-1)

### EXEMPLE 2 : Evaluation des propriétés antioxydantes des composés selon l'invention

### 1. Protection de l'oxydation des LDL par le cuivre :

Les composés selon l'invention, testés sont les composés dont la préparation est décrite dans les exemples décrits ci-dessus.

L'oxydation des LDL est une modification importante et joue un rôle prépondérant dans la mise en place et le développement de l'athérosclérose (Jurgens, Hoff et al. 1987). Le protocole suivant permet la mise en évidence des propriétés antioxydantes des composés. Sauf mention différente, les réactifs proviennent de chez Sigma (St Quentin, France).

Les LDL sont préparés suivant la méthode décrite par Lebeau et al. (Lebeau, Furman et al. 2000).

Les solutions de composés à tester sont préparées à 10⁻² M dans un tampon bicarbonate (pH = 9) et diluées dans du PBS pour avoir des concentrations finales allant de 0,1 à 100 µM pour une concentration totale d'éthanol de 1% (v/v).

Avant l'oxydation, l'EDTA est retiré de la préparation de LDL par dialyse. L'oxydation a ensuite lieu à 30°C en ajoutant 100 µl d'une solution à 16,6 µM de CuSO₄ à 160 µL de LDL (125 µg de protéines/ml) et 20 µl d'une solution du composé à tester. La formation de diènes, l'espèce à observer, se mesure par densité optique à 234 nm dans les échantillons traités avec les composés mais avec ou sans cuivre. La mesure de la densité optique à 234 nm est réalisée toutes les 10 minutes pendant 8 heures à l'aide d'un spectrophotomètre thermostaté (tecan Ultra 380). Les analyses sont réalisées en triplicata. Nous considérons que les composés ont une activité antioxydante lorsqu'ils induisent un retardement de la lag phase, diminuent la vitesse d'oxydation et la quantité de diènes formés par rapport à l'échantillon témoin. Les inventeurs mettent en évidence que les composés selon l'invention présentent au moins une des propriétés antioxydantes citées ci dessus ceci indiquant que les composés selon l'invention possèdent un caractère antioxydant intrinsèque.

Des exemples de résultats sont donnés sur les figures 1-1, 1-2, 1-3, 1-4, 1-5, 1-6, 1-7, 1-8, 1-9, 1-10, 1-11, 1-12, 1-13 et 1-14 où les propriétés antioxydantes des composés 2, 5, 6, 7, 8, 17, 19, 21, 22, 25, 29, 31, 33, 35, 37, 38 et 41 selon l'invention sont illustrées.

### 2. Evaluation de la protection conférée par les composés selon l'invention vis-à-vis de la peroxydation lipidique :

Les composés selon l'invention testés sont les composés dont la préparation est décrite dans les exemples décrits ci-dessus.

La mesure de l'oxydation des LDL est réalisée par la méthode des TBARS.

Selon le même principe que celui décrit précédemment, les LDL sont oxydés avec du CuSO₄ et la peroxydation lipidique est déterminée de la manière suivante :

Les TBARS sont mesurés à l'aide d'une méthode spectrophotométrique, l'hydroperoxydation lipidique est mesurée en utilisant l'oxydation péroxyde-lipide dépendante de l'iodide en iodine. Les résultats sont exprimés en nmol de malondialdehyde (MDA) ou en nmol d'hydroperoxyde/mg de protéines.

Les résultats obtenus précédemment, en mesurant l'inhibition de la formation de diènes conjugués, sont confirmés par les expériences de mesure de peroxydation lipidique des LDL. Les composés selon l'invention protègent également de manière efficace les LDL contre la peroxydation lipidique induite par le cuivre (agent oxydant).

### Exemple 3: mesure des propriétés antioxydantes des composés selon l'invention sur des culture de cellules

### Protocole de culture :

Les lignées cellulaires utilisées pour ce type d'expériences sont de type neuronales, neuroblastomes (humains) et cellules PC12 (rat). Les cellules PC12 ont été préparées à partir d'un pheochromocytome de rat et sont caractérisées par Greene et Tischler (Greene and Tischler, 1976). Ces cellules sont couramment utilisées pour des études de différenciation neuronale, transduction du signal et mort neuronale. Les cellules PC12 sont cultivées comme précédemment décrit (Farinelli, Park et al. 1996), dans du milieu complet RPMI (Invitrogen) complémenté avec 10% de sérum de cheval et 5% de sérum de veau foetal.

Des cultures (primaires) de cellules endothéliales et muscles lisses sont également utilisées. Les cellules sont commandées chez Promocell (Promocell GmBH, Heidelberg) et sont cultivées selon les indications du fournisseur.

Les cellules sont traitées avec différentes doses de composés de 5 à 300 µM pendant 24 heures. les cellules sont alors récupérées et l'augmentation de l'expression des gènes cibles est évaluée par PCR quantitative.

### Mesure des ARMm :

Les ARNm sont extraits des cellules en culture traitées ou non avec les composés selon l'invention. L'extraction est réalisée à l'aide des réactifs du kit Absolutely RNA RT-PCR miniprep Kit (Stratagene, France) selon les indications du fournisseur. Les ARNm sont ensuite dosés par spectrométrie et quantifiés par RT-PCR quantitative à l'aide du kit Light Cycler Fast start DNA Master Sybr Green I kit (Roche) sur un appareil Light Cycler System (Roche, France). Des paires d'amorces spécifiques des gènes de la Super Oxyde Dismutase (SOD), de la Catalase et de la Glutathion Peroxydase (GPx), enzymes anti-oxydantes, sont utilisées comme sondes. Des paires d'amorces spécifiques des gènes β-actine et cyclophiline sont utilisées comme sondes témoin.

L'augmentation de l'expression des ARNm, mesurée par RT-PCR quantitative, des gènes des enzymes antioxydantes est mise en évidence dans les différents types cellulaires utilisés, lorsque les cellules sont traitées avec les composés selon l'invention.

### Contrôle du stress Oxydatif :

### Mesure des espèces oxydantes dans les cellules en culture :

Les propriétés antioxydantes des composés sont également évaluées à l'aide d'un indicateur fluorescent dont l'oxydation est suivie par l'apparition d'un signal fluorescent. La diminution d'intensité du signal fluorescent émis est mesurée dans les cellules traitées avec les composés de la manière suivante : les cellules PC12 cultivées comme précédemment décrit (plaques noire 96 puits fonds transparents, Falcon) sont incubées avec des doses croissantes de H₂O₂ (0,25 mM - 1 mM) dans du milieu sans sérum pendant 2 et 24 heures. Après l'incubation le milieu est enlevé et les cellules sont incubées avec une solution de dichlorodihydrofluorescéine diacetate (DCFDA, Molecular Probes, Eugene, USA) 10 µM dans du PBS pendant 30 min à 37°C et dans une atmosphère contenant 5% de CO₂. Les cellules sont ensuite rincées avec du PBS. La détection de la fluorescence émise par l'indicateur de l'oxydation est mesurée à l'aide d'un fluorimètre (Tecan Ultra 384) à une longueur d'onde d'excitation de 495 nm et une longueur d'onde d'émission de 535 nm. Les résultats sont exprimés en pourcentage de protection par rapport au témoin oxydé.

L'intensité de fluorescence est plus faible dans les cellules incubées avec les composés selon l'invention que dans les cellules non traitées. Ces résultats indiquent que les composés selon l'invention favorisent l'inhibition de la production d'espèces oxydantes dans des cellules soumises à un stress oxydatif. Les propriétés antioxydantes décrites précédemment sont également efficaces pour induire une protection antiradicalaire dans des cellules en culture.

### Mesure de la peroxydation lipidique :

L'effet protecteur des composés sur la péroxydation lipidique sur des cultures de cellules (modèles cellulaires cités précédemment) est déterminé de la façon suivante : les différentes lignées cellulaires ainsi que les cellules en culture primaire sont traitées comme précédemment, le surnageant des cellules est récupéré après le traitement et les cellules sont lysées et récupérées pour la détermination de la concentration protéique. La détection de la peroxydation lipidique est déterminée de la manière suivante :

La peroxydation lipidique est mesurée à l'aide d'acide thiobarbiturique (TBA) qui réagit avec la lipoperoxydation des aldéhydes tel que le malondialdéhyde (MDA). Après les traitements, le surnageant des cellules est collecté (900 µl) et 90 µl d'hydroxytoluène butylé y sont ajoutés (Morliere, Moysan et al. 1991). Un ml d'une solution de TBA à 0,375% dans 0,25M HCL contenant 15% d'acide trichloroacétique est également ajouté aux milieux réactionnels. Le mélange est chauffé à 80°C pendant 15 min, refroidit sur glace et la phase organique est extraite avec du butanol. L'analyse de la phase organique se fait par spectrofluorométrie (λexc=515 nm et λem=550 nm) à l'aide du spectrofluorimètre Shimazu 1501 (Shimadzu Corporation, Kyoto, Japon). Les TBARS sont exprimés en équivalents MDA en utilisant un standard le tetra-ethoxypropane. Les résultats sont normalisés par rapport au contenu en protéines.

La diminution de la peroxydation lipidique observée dans les cellules traitées avec les composés selon l'invention confirme les résultats obtenus précédemment.

Les composés selon l'invention présentent avantageusement des propriétés antioxydantes intrinsèques qui permettent de ralentir et/ou d'inhiber les effets d'un stress oxydatif. Les inventeurs montrent également que les composés selon l'invention sont capables d'induirent l'expression des gènes d'enzymes antioxydants. Ces caractéristiques particulières des composés selon l'invention permettent aux cellules de lutter plus efficacement contre le stress oxydatif et donc d'être protégées vis à vis des dommages induits par les radicaux libres.

### Exemple 4: Evaluation de l'activation des PPARs in vitro par les composés selon l'invention

Les composés selon l'invention, possédant une fonction acide carboxylique, testés sont les composés dont la préparation est décrite dans les exemples décrits ci-dessus.

Les récepteurs nucléaires membres de la sous-famille des PPARs qui sont activés par deux classes majeures de composés pharmaceutiques, les fibrates et les glitazones, abondamment utilisées en clinique humaine pour le traitement des dislipidémies et du diabète, jouent un rôle important dans l'homéostasie lipidique et glucidique. Les données expérimentales suivantes montrent que les composés selon l'invention activent PPARα et PPARγ *in vitro.*

L'activation des PPARs est évaluée *in vitro* dans des lignées de type fibroblastique RK13 par la mesure de l'activité transcriptionnelle de chimères constituées du domaine de liaison à l'ADN du facteur de transcription Gal4 de levure et du domaine de liaison du ligand des différents PPARs. Ces derniers résultats sont ensuite confirmés dans des lignées cellulaires selon les protocoles suivants :

L'exemple est donné pour les cellules RK13.

### a. Protocoles de culture

Les cellules RK13 proviennent de I' ECACC (Porton Down, UK) et sont cultivées dans du milieu DMEM supplémenté de 10% vol/vol sérum de veau foetal, 100 U/ml pénicilline (Gibco, Paisley, UK) et 2 mM L-Glutamine (Gibco, Paisley, UK). Le milieu de culture est changé tous les deux jours. Les cellules sont conservées à 37°C dans une atmosphère humide contenant 5% de CO₂ et 95% d'air.

### b. Description des plasmides utilisés en transfection

Les plasmides pG5TkpGL3, pRL-CMV, pGal4-hPPARa, pGal4-hPPARy et pGal4-φ ont été décrits par Raspe, Madsen et al. (1999). Les constructions pGal4-mPPARα et pGal4-hPPARγ ont été obtenues par clonage dans le vecteur pGal4-φ de fragments d'ADN amplifiés par PCR correspondants aux domaines DEF des récepteurs nucléaires PPARα et PPARγ humains.

### c. Transfection

Les cellules RK13 sont ensemencées dans des boîtes de culture de 24 puits à raison de 5x10⁴ cellules/puit et sont transfectées pendant 2 heures avec le plasmide rapporteur pG5TkpGL3 (50 ng/puit), les vecteurs d'expression pGal4-φ, pGal4-mPPARα, pGal4-hPPARα, pGal4-hPPARγ (100 ng/puit) et le vecteur de contrôle de l'efficacité de transfection pRL-CMV (1 ng/puit) suivant le protocole décrit précédemment (Raspe, Madsen et al. 1999) et incubées pendant 36 heures avec les composés testés. A l'issue de l'expérience, les cellules sont lysées (Gibco, Paisley, UK) et les activités luciférase sont déterminées à l'aide du kit de dosage Dual-Luciferase^{™} Reporter Assay System (Promega, Madison, WI, USA) selon la notice du fournisseur comme décrit précédemment. Le contenu en protéines des extraits cellulaires est ensuite évalué à l'aide du kit de dosage Bio-Rad Protein Assay (Bio-Rad, München, Allemagne) selon la notice du fournisseur.

Les inventeurs mettent en évidence une augmentation de l'activité luciférase dans les cellules traitées avec les composés selon l'invention et transfectées avec le plasmide pGal4-hPPARα. Cette induction de l'activité luciférase indique que les composés selon l'invention, sont des activateurs de PPARα.

Un exemple de résultats est donné sur les figures 2-1, 2-2, 2-3, 2-4, 2-5, 2-6 où les propriétés activatrices PPARα des composés 7, 8, 11, 12, 17, 19, 20, 21, 22, 24, 25, 26, 29, 31, 33, 37, 38, 41 selon l'invention sont illustrées.

Les inventeurs mettent en évidence une augmentation de l'activité luciférase dans les cellules traitées avec les composés selon l'invention et transfectées avec le plasmide pGal4-hPPARγ. Cette induction de l'activité luciférase indique que les composés selon l'invention, sont des activateurs de PPARγ.

Des exemples de résultats sont représentés par la figure 2-7 où les propriétés activatrices PPAR γ des composés 17, 33 et 29 sont illustrées.

### Exemple 5: évaluation des propriétés anti-inflammatoires des composés selon l'invention

La réponse inflammatoire apparaît dans de nombreux désordres neurologiques, comme les scléroses multiples, la maladie d'Alzheimer et de Parkinson, les ischémie cérébrales et les accidents traumatiques du cerveau, de plus l'inflammation est l'un des facteurs importants de la neurodégénérescence. Lors d'accidents cérébraux, une des premières réactions des cellules de la glie est de libérer des cytokines et des radicaux libres. La conséquence de cette libération de cytokines et de radicaux libres est une réponse inflammatoire au niveau cérébral qui peut mener à la mort des neurones (Rothwell 1997).

Les lignées cellulaires et les cellules primaires sont cultivées comme décrit précédemment.

Le LPS, endotoxine bactérienne *(Escherichia coli* 0111 :B4) (Sigma, France) est reconstitué dans de l'eau distillée et conservé à 4°C. les cellules sont traitées avec une concentration de LPS de 1 µg/ml pendant 24 heures. Pour éviter toutes interférences avec d'autres facteurs le milieu de culture des cellules est totalement changé.

Le TNF-α est un facteur important de la réponse inflammatoire à un stress (oxydant par exemple). Pour évaluer la sécrétion de TNF-α en réponse à une stimulation par des doses croissantes de LPS, le milieu de culture des cellules stimulées est prélevé et la quantité de TNF-α est évaluée avec un kit ELISA-TNF-α (Immunotech, France). Les échantillons sont dilués 50 fois afin d'être en adéquation avec la gamme étalon (Chang, Hudson et al. 2000).

La propriété anti-inflammatoire des composés est caractérisée de la manière suivante : le milieu de culture des cellules est totalement changé et les cellules sont incubées avec les composés à tester pendant 2 heures. Après cette incubation, du LPS est rajouté au milieu de culture à une concentration finale de 1 µg/ml. Après 24 heures d'incubation, le surnageant de cellules est récupéré et stocké à -80°C lorsqu'il n'est pas traité directement. Les cellules sont lysées et la quantité de protéines est quantifiée, à l'aide du kit de dosage Bio-Rad Protein Assay (Bio-Rad, München, Allemagne) selon la notice du fournisseur.

La mesure de la diminution de sécrétion de TNF-α favorisée par le traitement avec les composés testés est exprimée en pg/ml/µg de protéine et rapporté en pourcentage par rapport au témoin. Ces résultas montrent que les composés selon l'invention possèdent des propriétés anti-inflammatoires.

### Exemple 6: Evaluation des effets neuro-protecteurs des composés selon l'invention dans un modèle d'ischémie-reperfusion cérébral

### Modèle Prophylactique :

### 1/ Traitements des animaux

### 1.1 Animaux et administration des composés

Des souris C57 black/6 (sauvages) ont été utilisés pour cette expérience.

Les animaux sont maintenus sous un cycle lumière/obscurité de 12 h à une température de 20 +/- 3°C. Les animaux ont un accès libre à l'eau et à la nourriture. La prisé de nourriture et la prise de poids sont enregistrées.

Les animaux sont traités par gavage avec les composés selon l'invention (200 mg/kg/jour) ou le véhicule (carboxycellulose 0,5% (CMC)), pendant 14 jours avant l'induction de l'ischémie de l'artère moyenne cérébrale.

### 1.2 Induction d'une ischémie-reperfusion par occlusion intraluminale de l'artère moyenne cérébrale :

Les animaux ont été anesthésiés à l'aide d'une injection intra-péritonéale de 300 mg/kg d'hydrate de chloral. Une sonde rectale est mise en place et la température du corps est maintenue à 37 +/- 0,5°C. La pression artérielle est mesurée au cours de toute l'expérience.

Sous un microscope chirurgical, la carotide droite est mise à jour à l'aide d'une incision cervicale médiale. L'artère ptérygopalatine a été ligaturée à son origine et une artèriotomie est réalisée dans l'artère carotide externe afin d'y glisser un mono-filament de nylon. Ce filament est alors doucement avancé dans l'artère carotide commune puis dans l'artère carotide interne afin d'obturer l'origine de l'artère cérébrale moyenne. Après 1 heure, le filament est retiré pour permettre la reperfusion.

### 2/ Mesure du volume de l'infarctus cérébral:

24 heures après la reperfusion, les animaux préalablement traités ou non traités avec les composés sont tués par une overdose de pentobarbital.

Les cerveaux sont rapidement congelés et sectionnés. Les sections sont colorées au violet Cresyl. Les zones non colorées des sections cérébrales ont été considérées comme lésées par l'infarctus. Les aires ont été mesurées et le volume de l'infarctus et des deux hémisphères ont été calculés par la formule suivante (Volume de l'infarctus corrigé = Volume de l'infarctus - (volume de l'hémisphère droit - volume de l'hémisphère gauche)) pour compenser l'oedème cérébral.

L'analyse des coupes de cerveaux d'animaux traités révèle une nette diminution du volume de l'infarctus par rapport aux animaux non traités. Lorsque les composés selon l'invention sont administrés aux animaux avant l'ischémie (effet prophylactique), ils sont capables d'induire une neuroprotection.

Un exemple de résultats est donné sur les figures 3-1 où les propriétés neuroprotectrices prophylactiques des composés 15 et 42 selon l'invention sont illustrées.

### 3/ mesure de l'activité des enzymes anti-oxydantes :

Les cerveaux des souris sont congelés, écrasés et réduits en poudres puis re-suspendus dans une solution saline. Les différentes activités enzymatiques sont ensuite mesurées comme décrits par les auteurs suivants : superoxide dismutase (Flohe and Otting 1984); glutathion peroxidase (Paglia and Valentine 1967) ; glutathion reductase (Spooner, Delides et al. 1981); glutathion-S-transferase (Habig and Jakoby 1981); catalase (Aebi 1984).

Les différentes activités enzymatiques mentionnées ci-dessus sont augmentées dans les préparations de cerveaux des animaux traités avec les composés selon l'invention.

### Modèle curatif ou traitement de la phase aiguë

### 1/ Induction d'une ischémie-reperfusion par occlusion intraluminale de l'artère moyenne cérébrale.

Des animaux tels que décrits précédemment sont utilisés pour cette expérience. Les animaux sont anesthésiés à l'aide d'une injection intra-péritonéale de 300 mg/kg d'hydrate de chloral. Une sonde rectale est mise en place et la température du corps est maintenue à 37 +/- 0,5°C. La pression artérielle est mesurée au cours de toute l'expérience.

Sous un microscope chirurgical, la carotide droite est mise à jour à l'aide d'une incision cervicale médiale. L'artère ptérygopalatine a été ligaturée à son origine et une artèriotomie est réalisée dans l'artère carotide externe afin d'y glisser un mono-filament de nylon. Ce filament est ensuite doucement avancé dans l'artère carotide commune puis dans l'artère carotide interne afin d'obturer l'origine de l'artère cérébrale moyenne. Après 1 heure, le filament est retiré pour permettre la reperfusion.

### 2/ traitement des animaux :

Les animaux ayant subi une ischémie-reperfusion préalable sont traités par les composés selon l'invention par voie orale ou systémique une ou plusieurs fois après la reperfusion.

### 3/ Mesure du volume de l'infarctus cérébral :

72 heures après la reperfusion, les animaux préalablement traités ou non traités avec les composés sont tués par une overdose de pentobarbital.

Les cerveaux sont rapidement congelés et sectionnés. Les sections sont colorées au violet Cresyl. Les zones non colorées des sections cérébrales ont été considérées comme lésées par l'infarctus. Les aires ont été mesurées et le volume de l'infarctus et des deux hémisphères ont été calculés par la formule suivante (Volume de l'infarctus corrigé = Volume de l'infarctus - (volume de l'hémisphère droit - volume de l'hémisphère gauche)) pour compenser l'oedème cérébral.

Dans les cas d'un traitement curatif (traitement de la phase aiguë), les animaux traités avec les composés selon l'invention ont des dommages au niveau cérébral réduit par rapport aux animaux non traités. En effet le volume de l'infarctus est diminué lorsque les composés selon l'invention sont administrés une ou plusieurs fois après l'ischémie-reperfusion.

Un exemple de résultats est donné sur les figures 3-2 où les propriétés neuroprotectrices en phase aiguë des composés 15 et 42 selon l'invention sont illustrées.

L'utilisation des composés selon l'invention dans différents modèles expérimentaux montre que ces nouveaux composés possèdent une activité antioxydante intrinsèque, capable de retarder et de réduire les effets d'un stress oxydatif, de plus ils induisent également l'expression des gènes des enzymes antioxydantes ce qui associé à leur caractère antioxydant permet de renforcer les protections anti-radicalaires de cellules en culture. Par ailleurs les composés selon l'invention possèdent également un pouvoir anti-inflammatoire et la propriété d'activer le récepteur nucléaire PPARα.

Enfin, l'utilisation des composés selon l'invention, possédant une fonction ester ou une fonction acide carboxylique, dans un modèle d'ischémie reperfusion chez l'animal montre l'effet bénéfique sur la neuroprotection aussi bien avec un traitement préventif que curatif.

### BIBLIOGRAPHIE

Adams, H. P., Jr. (2002). "Emergent use of anticoagulation for treatment of patients with ischemic stroke." Stroke 33(3): 856-61.
Aebi, H. (1984). "Catalase in vitro." Methods Enzymol 105: 121-6.
Bordet, R., D. Deplanque, et al. (2000). "Increase in endogenous brain superoxide dismutase as a potential mechanism of lipopolysaccharide-induced brain ischemic tolerance." J Cereb Blood Flow Metab 20(8): 1190-6.
Chabrier, P. E., M. Auguet, et al. (1999). "BN 80933, a dual inhibitor of neuronal nitric oxide synthase and lipid peroxidation: a promising neuroprotective strategy." Proc Natl Acad Sci U S A 96(19): 10824-9.
Chang, R. C., P. Hudson, et al. (2000). "Influence of neurons on lipopolysaccharide-stimulated production of nitric oxide and tumor necrosis factoralpha by cultured glia." Brain Res 853(2): 236-44.
Clark, R. B. (2002). "The role of PPARs in inflammation and immunity." J Leukoc Biol 71(3): 388-400.
Dirnagl, U., C. ladecola, et al. (1999). "Pathobiology of ischaemic stroke: an integrated view." Trends Neurosci 22(9): 391-7.
Ellis, C. N., J. Varani, et al. (2000). "Troglitazone improves psoriasis and normalizes models of proliferative skin disease: ligands for peroxisome proliferator-activated receptor- gamma inhibit keratinocyte proliferation." Arch Dermatol 136(5): 609-16.
Farinelli, S. E., D. S. Park, et al. (1996). "Nitric oxide delays the death of trophic factor-deprived PC12 cells and sympathetic neurons by a cGMP-mediated mechanism." J Neurosci 16(7): 2325-34.
Flohe, L. and F. Otting (1984). "Superoxide dismutase assays." Methods Enzymol 105: 93-104.
Fruchart, J. C., B. Staels, et al. (2001). "PPARS, metabolic disease and atherosclerosis." Pharmacol Res 44(5): 345-52.
Gervois, P., N. Vu-Dac, et al. (2001). "Negative regulation of human fibrinogen gene expression by peroxisome proliferator-activated receptor alpha agonists via inhibition of CCAAT box/enhancer-binding protein beta." J Biol Chem 276(36): 33471-7.
Gilgun-Sherki, Y., E. Melamed, et al. (2001). "Oxidative stress induced-neurodegenerative diseases: the need for antioxidants that penetrate the blood brain barrier." Neuropharmacology 40(8): 959-75.
Gorelick, P. B. (2002). "Stroke prévention therapy beyond antithrombotics: unifying mechanisms in ischemic stroke pathogenesis and implications for therapy: an invited review." Stroke 33(3): 862-75.
Greene, L. A. and A. S. Tischler (1976). "Establishment of a noradrenergic clonal line of rat adrenal pheochromocytoma cells which respond to nerve growth factor." Proc Natl Acad Sci U S A 73(7): 2424-8.
Habig, W. H. and W. B. Jakoby (1981). "Assays for differentiation of glutathione S-transferases." Methods Enzymol 77: 398-405.
Jurgens, G., H. F. Hoff, et al. (1987). "Modification of human serum low density lipoprotein by oxidation- characterization and pathophysiological implications." Chem Phys Lipids 45(2-4): 315-36.
Kainu, T., A. C. Wikstrom, et al. (1994). "Localization of the peroxisome proliferator-activated receptor in the brain." Neuroreport 5(18): 2481-5.
Komuves, L. G., K. Hanley, et al. (2000). "Stimulation of PPARalpha promotes epidermal keratinocyte differentiation in vivo." J Invest Dermatol 115(3): 353-60.
Lebeau, J., C. Furman, et al. (2000). "Antioxidant properties of di-tert-butylhydroxylated flavonoids." Free Radic Biol Med 29(9): 900-12.
Lutsep, H. L. and W. M. Clark (2001). "Current status of neuroprotective agents in the treatment of acute ischemic stroke." Curr Neurol Neurosci Rep 1(1): 13-8.
Mates, J. M., C. Perez-Gomez, et al. (1999). "Antioxidant enzymes and human diseases." Clin Biochem 32(8): 595-603.
Morliere, P., A. Moysan, et al. (1991). "UVA-induced lipid peroxidation in cultured human fibroblasts." Biochim Biophys Acta 1084(3): 261-8.
Nandagopal, K., T. M. Dawson, et al. (2001). "Critical role for nitric oxide signaling in cardiac and neuronal ischemic preconditioning and tolerance." J Pharmacol Exp Ther 297(2): 474-8.
Paglia, D. E. and W. N. Valentine (1967). "Studies on the quantitative and qualitative characterization of erythrocyte glutathione peroxidase." J Lab Clin Med 70(1): 158-69.
Raspe, E., L. Madsen, et al. (1999). "Modulation of rat liver apolipoprotein gene expression and serum lipid levels by tetradecylthioacetic acid (TTA) via PPARalpha activation." J Lipid Res 40(11): 2099-110.
Rothwell, N. J. (1997). "Cytokines and acute neurodegeneration." Mol Psychiatry 2(2): 120-1.
Smith, K. J., E. Dipreta, et al. (2001). "Peroxisomes in dermatology. Part II." J Cutan Med Surg 5(4):315-22.
Spooner, R. J., A. Delides, et al. (1981). "Heat stability and kinetic properties of human serum glutathione reductase activity in various disease states." Biochem Med 26(2): 239-48.

## Revendications

1. Dérivés de 1,3-diphénylprop-2-èn-1-one substitués, **caractérisés en ce qu'**ils présentent la formule (I) suivante : 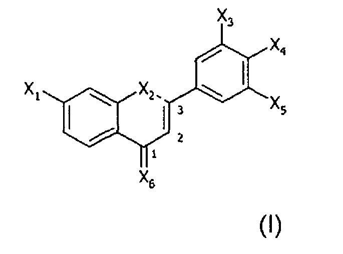 dans laquelle :
X1 représente un halogène ou un groupement -R1 ou un groupement répondant à la formule suivante : -G1-R1,
X2 représente un atome d'hydrogène ou un groupement thionitroso ou un groupement hydroxy ou un groupement alkylcarbonyloxy ou un groupement alkyloxy non substitué ou un groupement thiol ou un groupement alkylthio ou un groupement alkylcarbonylthio, X2 peut également représenter un atome d'oxygène ou de soufre lié au carbone 3 de la chaîne propène, pour former un dérivé de type 2-phényl-4H-1-benzopyran-4-one ou de type 2-phényl-4H-1-benzothiopyran-4-one,
X3 représente un groupement -R3 ou un groupement répondant à la formule suivante : -G3-R3,
X4 représente un halogène ou un groupement thionitroso ou un groupement -R4 ou un groupement répondant à la formule suivante : -G4-R4,
X5 représente un groupement -R5 ou un groupement répondant à la formule suivante : -G5-R5,
X6 est un atome d'oxygène,
R1, R3, R4, R5, identiques ou différents, représentent un atome d'hydrogène ou un groupement alkyle substitué ou non par un groupement faisant partie du groupe 1 ou du groupe 2 définis ci-dessous,
G1, G3, G4, G5, identiques ou différents, représentent un atome d'oxygène ou de soufre,
avec au moins un des groupements X1, X3, X4 ou X5 répondant à la formule -G-R, dans laquelle G est un atome de soufre, et
avec au moins un des groupements R1, R3, R4 ou R5 présent sous la forme d'un radical alkyle portant au moins un substituant du groupe 1 ou 2, ledit radical alkyle étant lié directement au cycle ou étant associé à un groupement G selon la formule -GR,
les substituants du groupe 1 sont choisis parmi les groupements carboxy de formule : -COOR₆ et les groupements carbamoyle de formule : -CONR₆R₇,
les substituants du groupe 2 sont choisis parmi l'acide sulfonique (-SO₃H) et les groupements sulfonamide de formule : -SO₂NR₆R₇,
avec R₆ et R₇, identiques ou différents, représentant un atome d'hydrogène ou un radical alkyle éventuellement substitué par au moins un groupe de type 1 ou 2,
leurs isomères optiques et géométriques, leurs racémates, leurs tautomères, leurs sels, leurs hydrates et leurs mélanges,
à l'exclusion des composés de formule (I) dans laquelle:
X₂ représente un atome d'hydrogène et X₁ représente -G1R1 où G1 représente un atome d'oxygène et R1 représente CH2COOH.

2. Dérivés de 1,3-diphénylprop-2-èn-1-one substitués, **caractérisés en ce qu'**ils présentent la formule (I) suivante : 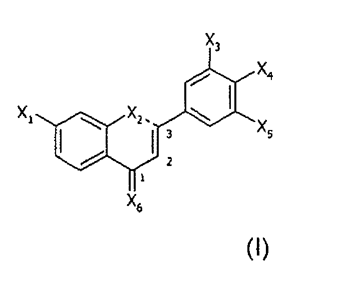 dans laquelle:
X1 représente un halogène ou un groupement -R1 ou un groupement répondant à la formule suivante : -G1-R1,
X2 représente un atome d'hydrogène ou un groupement thionitroso ou un groupement hydroxy ou un groupement alkylcarbonyloxy ou un groupement alkyloxy non substitué ou un groupement thiol ou un groupement alkylthio ou un groupement alkylcarbonylthio, X2 peut également représenter un atome de soufre lié au carbone 3 de la chaîne propène, pour former un dérivé de type 2-phényl-4H-1-benzothiopyran-4-one.
X3 représente un groupement -R3 ou un groupement répondant à la formule suivante : -G3-R3,
X4 représente un halogène ou un groupement thionitroso ou un groupement -R4 ou un groupement répondant à la formule suivante : -G4-R4,
X5 représente un groupement -R5 ou un groupement répondant à la formule suivante : -G5-R5,
X6 est un atome d'oxygène,
R1, R3, R4, R5, identiques ou différents, représentent un atome d'hydrogène ou un groupement alkyle substitué ou non par un groupement faisant partie du groupe 1 ou du groupe 2 définis ci-dessous,
G1, G3, G4, G5, identiques ou différents, représentent un atome d'oxygène ou de soufre,
avec au moins un des groupements X1, X3, X4 ou X5 répondant à la formule -G-R,
avec aucun des groupements X3, X4 ou X5 ne représentant un atome d'hydrogène, et
avec au moins un des groupements R1, R3, R4 ou R5 présent sous la forme d'un radical alkyle portant au moins un substituant du groupe 1 ou 2, ledit radical alkyle étant lié directement au cycle ou étant associé à un groupement G selon la formule -GR,
les substituants du groupe 1 sont choisis parmi les groupements carboxy de formule : -COOR₆ et les groupements carbamoyle de formule : -CONR₆R₇,
les substituants du groupe 2 sont choisis parmi l'acide sulfonique (SO₃H) et les groupements sulfonamide de formule : -SO₂NR₆R₇
avec R₆ et R₇, identiques ou différents, représentant un atome d'hydrogène ou un radical alkyle éventuellement substitué par au moins un groupe de type 1 ou 2,
leurs isomères optiques et géométriques, leurs racémates, leurs tautomères, leurs sels, leurs hydrates et leurs mélanges,
à l'exclusion des composés de formule (I) dans laquelle :
- X₂ représente un atome d'hydrogène et X₁ représente -G1R1 où G1 représente un atome d'oxygène et R1 représente CH2COOH,
- X₂ représente un atome d'hydrogène, X₆ représente un atome d'oxygène, X₁ représente -G1R1 où G1 représente un atome d'oxygène et R1 représente CH2COOC2H5 ou CH2CONHCH(CH3)2, et X₃, X₄, et X₅ représentent OCH3.

3. Dérivés selon la revendication 1 ou 2, **caractérisés en ce qu'**ils peuvent correspondre à la conformation cis, trans ou leur mélange.

4. Dérivés selon la revendication 1, **caractérisés en ce qu'**aucun des groupements X3, X4 et X5 ne représente un atome d'hydrogène.

5. Dérivés selon la revendication 1, **caractérisés en ce qu'**un ou deux des groupements X3, X4 et X5 représente un atome d'hydrogène.

6. Dérivés selon l'une des revendications précédentes, **caractérisés en ce que** les deux groupements G1 et G4 représentent un atome de soufre.

7. Dérivés selon l'une des revendications précédentes, **caractérisés en ce que** X2 est un atome d'hydrogène, un groupement thionitroso ou un groupement hydroxy ou un groupement alkyloxy ou un groupement thiol ou un groupement alkylthio.

8. Dérivés selon l'une des revendications précédentes, **caractérisés en ce que** X4 représente un groupement thionitroso ou un groupement -R4 ou un groupement de formule -G4R4 et X2 est un groupement thionitroso ou un groupement hydroxy, alkyloxy, thiol ou alkylthio, G4 et R4 étant tels que définis dans la revendication 1 ou 2.

9. Dérivés selon l'une des revendications précédentes, **caractérisés en ce que** X1 représente un groupement -R1 ou un groupement répondant à la formule -G1-R1, R1 étant un groupement alkyle substitué par un groupement faisant partie du groupe 1, G1 et le substituant du groupe 1 étant tels que définis à la revendication 1 ou 2.

10. Dérivés selon l'une des revendications précédentes, **caractérisés en ce que** X1 est un groupement -G1-R1, G1 et R1 étant tels que définis à la revendication 1 ou 2.

11. Dérivés selon l'une des revendications précédentes, **caractérisés en ce que** X1 est un groupement -G1-R1 dans lequel G1 est un atome d'oxygène, R1 étant tel que défini à la revendication 1 ou 2.

12. Dérivés selon l'une des revendications précédentes, **caractérisés en ce que** X1 représente un groupement -R1 ou un groupement répondant à la formule -G1-R1, R1 étant un groupement alkyle substitué par un groupement faisant partie du groupe 2, G1 et le substituant du groupe 2 étant tels que définis à la revendication 1 ou 2.

13. Dérivés selon l'une des revendications précédentes, **caractérisés en ce que** X3 représente un groupement -R3 ou un groupement répondant à la formule -G3-R3, R3 étant un groupement alkyle substitué par un groupement faisant partie du groupe 1, G3 et le substituant du groupe 1 étant tels que définis à la revendication 1 ou 2.

14. Dérivés selon l'une des revendications précédentes, **caractérisés en ce que** X3 représente un groupement -R3 ou un groupement répondant à la formule -G3-R3, R3 étant un groupement alkyle substitué par un groupement faisant partie du groupe 2, G3 et le substituant du groupe 2 étant tels que définis à la revendication 1 ou 2.

15. Dérivés selon l'une des revendications précédentes, **caractérisés en ce que** X4 représente un groupement -R4 ou un groupement répondant à la formule -G4-R4, R4 étant un groupement alkyle substitué par un groupement faisant partie du groupe 1, G4 et le substituant du groupe 1 étant tels que définis à la revendication 1 ou 2.

16. Dérivés selon l'une des revendications précédentes, **caractérisés en ce que** X4 est un groupement -G4-R4, G4 et R4 étant tels que définis à la revendication 1 ou 2.

17. Dérivés selon l'une des revendications précédentes, **caractérisés en ce que** X4 est un groupement -G4-R4 dans lequel G4 est un atome d'oxygène, R4 étant tel que défini à la revendication 1 ou 2.

18. Dérivés selon l'une des revendications précédentes, **caractérisés en ce que** X4 est un groupement -G4-R4 dans lequel G4 est un atome d'oxygène, et X3 ou X5 représente respectivement R3 ou G3R3, d'une part, et R5 ou G5R5, d'autre part, R3 ou R5 étant des groupements alkyle portant un substituant du groupe 1, R4, G3, G5 et le substituant du groupe 1 étant tels que définis à la revendication 1 ou 2.

19. Dérivés selon l'une des revendications précédentes, **caractérisés en ce que** X4 représente un groupement -R4 ou un groupement répondant à la formule -G4-R4, R4 étant un groupement alkyle substitué par un groupement faisant partie du groupe 2, G4 et le substituant du groupe 2 étant tels que définis à la revendication 1 ou 2.

20. Dérivés selon l'une des revendications précédentes, **caractérisés en ce que** X1 représente un halogène.

21. Dérivés selon l'une des revendications précédentes, **caractérisés en ce que** X1, X3, X4 ou X5 représente OC(CH3)2COOR6, R6 étant tel que défini à la revendication 1 ou 2.

22. Dérivés selon l'une des revendications précédentes, **caractérisés en ce que** X1, X3, X4 ou X5 représente SC(CH3)2COOR6, R6 étant tel que défini à la revendication 1 ou 2.

23. Dérivé selon l'une quelconque des revendications précédentes, **caractérisé en ce qu'**il est choisi parmi :
1-[2-hydroxy-4-carboxydiméthylméthyloxyphényl]-3-[3,5-ditertbutyl-4-hydroxyphényl]prop-2-èn-1-one,
1-[2-hydroxy-4-éthoxycarbonyldiméthylméthyloxyphényl]-3-[3,5-ditertbutyl-4-hydroxyphényl]prop-2-èn-1-one,
1-[2-hydroxy-4-isopropyloxycarbonyldiméthylméthyloxyphényl]-3-[3,5-di*tert*butyl-4-hydroxyphényl]prop-2-èn-1-one,
1-[2-hydroxyphényl]-3-[3-carboxydiméthylméthyloxy-4-hydroxy-5-*tert*butylphényl]prop-2-èn-1-one,
1-[2-hyd roxyphényl]-3-[3-isopropyloxycarbonyldiméthylméthyloxy-4-hyd roxy-5-*tert*butylphényl]prop-2-èn-1-one,
1-[2-hydroxy-4-chlorophényl]-3-[3-carboxydiméthylméthyloxy-4-hydroxy-5-*tert*butylphényl]prop-2-èn-1-one,
1-[2-hydroxy-4-chlorophényl]-3-[3-*is*opropyloxycarbonyldiméthylméthyloxy-4-hydroxy-5-*tert*butylphényl]prop-2-èn-1-one,
1-[2-hydroxyphényl]-3-[3-carboxydiméthylméthyl-4-hydroxy-5-*tert*butylphényl]prop-2-èn-1-one,
1-[2-hydroxyphényl]-3-[3-*iso*propyloxycarbonyldiméthylméthyl-4-hydroxy-5-*tert*butylphényl]prop-2-èn-1-one,
1-[2-hydroxy-4-chlorophényl]-3-[3-carboxydiméthylméthyl-4-hydroxy-5-*tert*butylphényl]prop-2-èn-1-one,
1-[2-hydroxy-4-chlorophényl]-3-[3-isopropyloxycarbonyldiméthylméthyl-4-hydroxy-5-*tert*butylphényl]prop-2-èn-1-one,
1-[2-hydroxy-4-chlorophényl]-3-[3,5-diméthoxy-4-carboxydiméthylméthyloxyphényl]prop-2-èn-1-one,
1-[2-hydroxy-4-chlorophényl]-3-[3,5-diméthoxy-4-isopropyloxycarbonyldiméthylméthyloxyphényl]prop-2-èn-1-one,
1-[2-hydroxyphényl]-3-[3,5-diméthoxy-4-carboxydiméthylméthyloxyphényl]prop-2-èn-1-one,
1-[2-hydroxyphényl]-3-[3,5-diméthoxy-4-*iso*propyloxycarbonyl diméthylméthyloxyphényl]prop-2-èn-1-one,
1-[2-hydroxy-4-carboxydiméthylméthyloxyphényl]-3-[3,5-diméthoxy-4-hydroxyphényl]prop-2-èn-1-one,
1-[2-hydroxy-4-isopropyloxycarbonyldiméthylméthyloxyphényl]-3-[3,5-diméthoxy-4-hydroxyphényl]prop-2-èn-1-one,
1-[2-hydroxy-4-chlorophényl]-3-[3 ,4-dihyd roxy-5-carboxydiméthyiméthyloxyphényl]prop-2-èn-1-one,
1-[2-hydroxy-4-chlorophényl]-3-[3,4-dihydroxy-5-isopropyloxycarbonyldiméthylméthyloxyphényi]-prop-2-èn-1-one,
1-[2-hydroxy-4-carboxydiméthylméthyloxyphényl]-3-[3,5-diméthy(-4-hydroxyphényl]prop-2-èn-1-one,
1-[2-hydroxy-4-isopropyloxycarbonyldiméthylméthyloxyphényl]-3-[3,5-diméthyl-4-hydroxyphényl]prop-2-èn-1-one,
1-[2-hydroxy-4-chlorophényl]-3-[3,5-diméthyl-4-carboxydiméthylméthyloxyphényl]prop-2-èn-1-one,
1-[2-hydroxy-4-chlorophényl]-3-[3,5-diméthyl-4-*iso*propyloxycarbonyldiméthylméthyloxyphényl]prop-2-èn-1-one,
1-[2-hydroxyphényl]-3-[3,5-diméthyl-4-carboxydiméthylméthyloxyphényl]prop-2-èn-1-one,
1-[2-hydroxyphényl]-3-[3,5-diméthyl-4-isopropyloxycarbonyl diméthylméthyloxyphényl]prop-2-èn-1-one,
1-[2-hydroxyphényl]-3-[4-carboxydiméthylméthylthiophényl]prop-2-èn-1-one,
1-[2-hydroxyphényl],3-[4-isopropyloxycarbonyldiméthylméthylthiophényl]prop-2-èn-1-one,
1-[2-hydroxy-4-carboxydiméthylméthyloxyphényl]-3-[4-méthylthiophényl]prop-2-èn-1-one,
1-[4-chlorophényl]-3-[3,5-diméthyl-4-tertiobutyloxycarbonyldiméthylméthyloxyphényl]prop-2-èn-1-one,
1-[4-chlorophényl]-3-[3,5-diméthyl-4-isopropyloxycarbonyldiméthylméthyloxyphènyl]prop-2-èn-1-one,
1-[4-chlorophényl]-3-[3,5-diméthyl-4-carboxydiméthylméthyloxyphényl]prop-2-èn-1-one,
1-[4-chloro-2-hydroxyphényl]-3-[4-carboxydiméthylméthylthiophényl]prop-2-èn-1-one,
1-[4-carboxydiméthylméthyloxyphényl]-3-[3,5-diméthyl-4-hydroxyphényl]prop-2-èn-1-one,
1-[4-méthylthiophényl]-3-[4-carboxydiméthylméthyloxyphényl]prop-2-èn-1-one,
1-[4-carboxydiméthylméthylthiophényl]-3-[4-méthylthiophényl]prop-2-èn-1-one,
1-[2-hydroxy-4-bromophényl]-3-[3,5-diméthyl-4-carboxydiméthylméthyloxyphényl]prop-2-èn-1-one,
1-[4-carboxydiméthylméthyloxyphényl]-3-[4-méthylthiophényl]prop-2-èn-1-one,
1-[4-méthylthiophényl]-3-[3,5-diméthyl-4-tertiobutyloxycarbonyldiméthylméthyloxyphényl]prop-2-èn-1-one,
1-[4-méthylthiophényl]-3-[3,5-diméthyl-4-isopropyloxycarbonyldiméthylméthyloxyphényl]prop-2-èn-1-one,
1-[4-méthylthiophényl]-3-[3,5-diméthyl-4-carboxydiméthylméthyloxyphényl]prop-2-èn-1-one,
1-[2-méthoxyphényl]-3-[3,5-diméthyl-4-tertiobutyloxycarbonyldiméthylméthyloxyphényl]prop-2-èn-1-one,
1-[2-méthoxyphényl]-3-[3,5-diméthyl-4-carboxydiméthylméthyloxyphényl]prop-2-èn-1-one,
1-[4-hexyloxyphényl]-3-[3,5-diméthyl-4-tertiobutyloxycarbonyldiméthylméthyloxyphényl]prop-2-èn-1-one,
1-[4-hexyloxyphényl]-3-[3,5-diméthyl-4-carboxydiméthylméthyloxyphényl]prop-2-èn-1-one,
1-[2-méthyloxy-4-chlorophényl]-3-[3,5-diméthyl-4-tertiobutyloxycarbonyldiméthylméthyloxyphényl]prop-2-én-1-one,
1-[2-méthyloxy-4-chlorophényl]-3-[3,5-diméthyl-4-carboxydiméthylméthyloxyphényl]prop-2-èn-1-one,
1-[4-heptyfphényl]-3-[3,5-diméthyl-4-tertiobutyloxycarbonyldiméthylméthyloxyphényl]prop-2-èn-1-one,
1-[4-heptylphényl]-3-[3,5-diméthyl-4-carboxydiméthylméthyloxyphényl]prop-2-èn-1-one,
1-[4-bromophényl]-3-[3,5-diméthyl-4-tertiobutyloxycarbonyldiméthylméthyloxyphényl]prop-2-èn-1-one,
1-[4-bromophényl]-3-[3,5-diméthyl-4-carboxydiméthylméthyloxyphényl]prop-2-èn-1-one.

24. Dérivé selon l'une quelconque des revendications précédentes, **caractérisé en ce que** le dérivé est choisi parmi :
1-[4-méthylthiophényl]-3-[3,5-diméthyl-4-carboxydiméthylméthyloxyphényl]prop-2-èn-1-one,
1-[4-hexyloxyphényl]-3-[3,5-diméthyl-4-carboxydiméthylméthyloxyphényl]prop-2-èn-1-one, et
1-[4-bromophényl]-3-[3,5-diméthyl-4-carboxydiméthylméthyloxyphényl]prop-2-èn-1-one.

25. Dérivé selon l'une quelconque des revendications précédentes, **caractérisé en ce que** le dérivé est la 1-[4-méthylthiophényl]-3-[3,5-diméthyl-4-carboxydiméthylméthyloxyphényl]prop-2-èn-1-one.

26. Procédé de préparation de composés de formule (I) tels que définis à l'une des revendications précédentes, **caractérisé en ce qu'**il comprend une mise en contact en milieu basique ou en milieu acide d'au moins un composé de formule (A) avec au moins un composé de formule (B), les formules (A) et (B) étant : 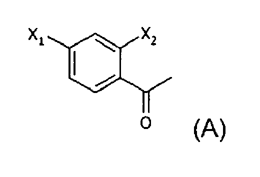 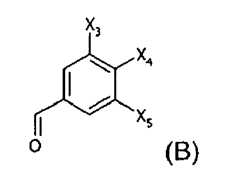 formules dans lesquelles X1, X2, X3, X4 et X5 ont les définitions données à la revendication 1 ou 2.

27. Composition pharmaceutique comprenant, dans un support acceptable sur le plan pharmaceutique, au moins un composé de formule (I) tel que défini dans l'une des revendications 1 à 25, y compris les composés de formule (I) où X₂ représente un atome d'hydrogène, X₆ représente un atome d'oxygène, X₁ représente -G1R1 où G1 représente un atome d'oxygène et R1 représente CH2COOC2H5 ou CH2CONHCH(CH3)2, et X₃, X₄, et X₅ représentent OCH3.

28. Composition pharmaceutique selon la revendication précédente, pour le traitement ou la prophylaxie d'une pathologie vasculaire cérébrale.

29. Composition pharmaceutique selon la revendication 28, **caractérisée en ce que** la pathologie vasculaire cérébrale est l'ischémie cérébrale.

30. Composition pharmaceutique selon la revendication 28, **caractérisée en ce que** la pathologie vasculaire cérébrale est un accident hémorragique cérébral.

31. Utilisation d'au moins un dérivé de 1,3-diphénylprop-2-èn-1-one substitué pour la préparation d'une composition pharmaceutique destinée à traiter de manière préventive ou de manière curative une pathologie vasculaire cérébrale, plus particulièrement l'ischémie cérébrale, **caractérisé en ce que** le dérivé de 1,3-diphénylprop-2-èn-1-one substitué présente une formule générale (I) telle que définie dans l'une des revendications 1 à 25, y compris les composés de formule générale (I) dans laquelle :
- X₂ représente un atome d'hydrogène et X₁ représente -G1R1 où G1 représente un atome d'oxygène et R1 représente CH2COOH, et
- X₂ représente un atome d'hydrogène, X₆ représente un atome d'oxygène, X₁ représente -G1R1 où G1 représente un atome d'oxygène et R1 représente CH2COOC2H5 ou CH2CONHCH(CH3)2, et X₃, X₄, et X₅ représentent OCH3.

## Claims

1. Substituted 1,3-diphenylprop-2-en-1-one derivatives, **characterized in that** they are represented by formula (I) below : 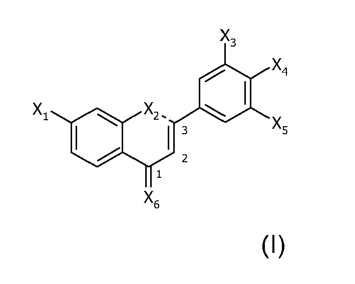 in which :
X1 represents a halogen or a -R1 group or a group corresponding to the following formula : -G1-R1,
X2 represents a hydrogen atom or a thionitroso group or a hydroxy group or an alkylcarbonyloxy or an unsubstituted alkyloxy group or a thiol group or an alkylthio group or an alkylcarbonylthio group, X2 can also represent an oxygen or sulfur atom bound to carbon 3 of the propene chain, so as to form a derivative of the type 2-phenyl-4H-1-benzopyran-4-one or of the type 2-phenyl-4H-1-benzothiopyran-4-one,
X3 represents a -R3 group or a group corresponding to the following formula: -G3-R3,
X4 represents a halogen or a thionitroso group or a -R4 group or a group corresponding to the following formula: -G4-R4,
X5 represents a -R5 group or a group corresponding to the following formula: -G5-R5,
X6 is an oxygen atom,
R1, R3, R4, R5, which are the same or different, represent a hydrogen atom or an alkyl group substituted or not by a substituent which is part of group 1 or group 2 defined hereinbelow,
G1, G3, G4, G5, which are the same or different, represent an oxygen or sulfur atom,
with at least one of the groups X1, X3, X4 or X5 corresponding to the formula -G-R in which G is a sulfur atom, and
with at least one of the groups R1, R3, R4 or R5 present in the form of an alkyl group containing at least one substituent from group 1 or 2, said alkyl group being bound directly to the ring or being associated with a group G according to the formula -GR,
substituents from group 1 are selected in the group consisting of carboxy groups corresponding to the formula: -COOR₆ and carbamoyl groups corresponding to the formula : -CONR₆R₇,
substituents from group 2 are selected in the group consisting of sulfonic acid (-SO₃H) and sulfonamide groups corresponding to the formula: -SO₂NR₆R₇,
with R₆ and R₇, which are the same or different, representing a hydrogen atom or an alkyl group possibly substituted by at least one group of the type 1 or 2,
the optical and geometric isomers, racemates, tautomers, salts, hydrates and mixtures thereof,
with the exception of compounds represented by formula (I) in which:
X₂ represents a hydrogen atom and X₁ represents -G1R1 where G1 represents an oxygen atom and R1 represents CH2COOH.

2. Substituted 1,3-diphenylprop-2-en-1-one derivatives, **characterized in that** they are represented by formula (I) below: 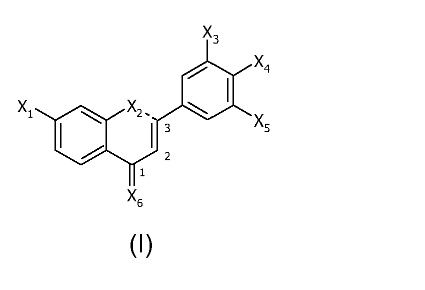 in which:
X1 represents a halogen or a -R1 group or a group corresponding to the following formula: -G1-R1,
X2 represents a hydrogen atom or a thionitroso group or a hydroxy group or an alkylcarbonyloxy or an unsubstituted alkyloxy group or a thiol group or an alkylthio group or an alkylcarbonylthio group, X2 can also represent a sulfur atom bound to carbon 3 of the propene chain, so as to form a derivative of the type 2-phenyl-4H-1-benzothiopyran-4-one,
X3 represents a -R3 group or a group corresponding to the following formula: -G3-R3,
X4 represents a halogen or a thionitroso group or a -R4 group or a group corresponding to the following formula: -G4-R4,
X5 represents a -R5 group or a group corresponding to the following formula: -G5-R5,
X6 is an oxygen atom,
R1, R3, R4, R5, which are the same or different, represent a hydrogen atom or an alkyl group substituted or not by a substituent which is part of group 1 or group 2 defined hereinbelow,
G1, G3, G4, G5, which are the same or different, represent an oxygen or sulfur atom, with at least one of the groups X1, X3, X4 or X5 corresponding to the formula -G-R, and
with none of the groups X3, X4 and X5 representing a hydrogen atom, and
with at least one of the groups R1, R3, R4 or R5 present in the form of an alkyl group containing at least one substituent from group 1 or 2, said alkyl group being bound directly to the ring or being associated with a group G according to the formula -GR,
substituents from group 1 are selected in the group consisting of carboxy groups corresponding to the formula : -COOR₆ and carbamoyl groups corresponding to the formula : -CONR₆R₇,
substituents from group 2 are selected in the group consisting of sulfonic acid (-SO₃H) and sulfonamide groups corresponding to the formula : -SO₂NR₆R₇,
with R₆ and R₇, which are the same or different, representing a hydrogen atom or an alkyl group possibly substituted by at least one group of the type 1 or 2,
the optical and geometric isomers, racemates, tautomers, salts, hydrates and mixtures thereof.
with the exception of compounds represented by formula (I) in which:
- X₂ represents a hydrogen atom and X₁ represents -G1R1 where G1 represents an oxygen atom and R1 represents CH2COOH,
- X₂ represents a hydrogen atom, X₆ represents an oxygen atom, X₁ represents - G1 R1 where G1 represents an oxygen atom and R1 represents CH2COOC2H5 or CH2CONHCH(CH3)2, and X₃, X₄, and X₅ represent OCH3.

3. Derivatives according to claim 1 or 2, **characterized in that** they can correspond to the cis or trans conformation or a mixture thereof.

4. Derivatives according to claim 1, **characterized in that** none of the groups X3, X4 and X5 represents a hydrogen atom.

5. Derivatives according to claim 1, **characterized in that** one or two of the groups X3, X4 and X5 represents a hydrogen atom.

6. Derivatives according to anyone of the preceding claims, **characterized in that** both G1 and G4 represent a sulfur atom.

7. Derivatives according to anyone of the preceding claims, **characterized in that** X2 is a hydrogen atom, a thionitroso group or a hydroxy group or an alkyloxy group or a thiol group or an alkylthio group.

8. Derivatives according to anyone of the preceding claims, **characterized in that** X4 is a thionitroso group or a -R4 group or a group corresponding to the formula -G4-R4 and X2 is a thionitroso group or a hydroxy group or an alkyloxy group or a thiol group or an alkylthio group, G4 and R4 being such as defined in claim 1 or 2.

9. Derivatives according to anyone of the preceding claims, **characterized in that** X1 represents a -R1 group or a group corresponding to the formula -G1-R1, with R1 being an alkyl group substituted by a substituent which is part of group 1 and G1 and the substituent from group 1 being such as defined in claim 1 or 2.

10. Derivatives according to anyone of the preceding claims, **characterized in that** X1 is a -G1-R1 group, G1 and R1 being such as defined in claim 1 or 2.

11. Derivatives according to anyone of the preceding claims, **characterized in that** X1 is a -G1-R1 group in which G1 is an oxygen atom, R1 being such as defined in claim 1 or 2.

12. Derivatives according to anyone of the preceding claims, **characterized in that** X1 represents a -R1 group or a group corresponding to the formula -G1-R1, with R1 being an alkyl group substituted by a substituent which is part of group 2 and G1 and the substituent from group 2 being such as defined in claim 1 or 2.

13. Derivatives according to anyone of the preceding claims, **characterized in that** X3 represents a -R3 group or a group corresponding to the formula -G3-R3, with R3 being an alkyl group substituted by a substituent which is part of group 1 and G3 and the substituent from group 1 being such as defined in claim 1 or 2.

14. Derivatives according to anyone of the preceding claims, **characterized in that** X3 represents a -R3 group or a group corresponding to the formula -G3-R3, with R3 being an alkyl group substituted by a substituent which is part of group 2 and G3 and the substituent from group 2 being such as defined in claim 1 or 2.

15. Derivatives according to anyone of the preceding claims, **characterized in that** X4 represents a -R4 group or a group corresponding to the formula -G4-R4, with R4 being an alkyl group substituted by a substituent which is part of group 1 and G4 and the substituent from group 1 being such as defined in claim 1 or 2.

16. Derivatives according to anyone of the preceding claims, **characterized in that** X4 is a -G4-R4 group, G4 and R4 being such as defined in 1 or 2.

17. Derivatives according to anyone of the preceding claims, **characterized in that** X4 is a -G4-R4 group in which G4 is an oxygen atom, R4 being such as defined in 1 or 2.

18. Derivatives according to anyone of the preceding claims, **characterized in that** X4 is a -G4-R4 group in which G4 is an oxygen atom, and X3 or X5 respectively represent R3 or G3R3, on the one hand, and R5 or G5R5, on the other hand, with R3 and R5 being alkyl groups containing a substituent from group 1, R4, G3, G5, and the substituent from group 1 being such as defined in claim 1 or 2.

19. Derivatives according to anyone of the preceding claims, **characterized in that** X4 represents a -R4 group or a group corresponding to the formula -G4-R4 with R4 being an alkyl group substituted by a substituent which is part of group 2 and G4 and the substituent from group 2 being such as defined in claim 1 or 2.

20. Derivatives according to anyone of the preceding claims, **characterized in that** X1 represents a halogen.

21. Derivatives according to anyone of the preceding claims, **characterized in that** X1, X3, X4 or X5 represents OC(CH3)2COOR6, R6 being such as defined in claim 1 or 2.

22. Derivatives according to anyone of the preceding claims, **characterized in that** X1, X3, X4 or X5 represents SC(CH3)2COOR6, R6 being such as defined in claim 1 or 2.

23. Derivative according to anyone of the preceding claims, **characterized in that** it is selected in the group consisting of:
1-[2-hydroxy-4-carboxydimethylmethyloxyphenyl]-3-[3,5-di*tert*butyl-4-hydroxyphenyl]prop-2-en-1-one,
1-[2-hydroxy-4-ethoxycarbonyldimethylmethyloxyphenyl]-3-[3,5-ditertbutyl-4-hydroxyphenyl]prop-2-en-1-one,
1-[2-hydroxy-4-isopropyloxycarbonyldimethylmethyloxyphenyl]-3-[3,5-ditertbutyl-4-hydroxyphenyl]prop-2-en-1-one,
1-[2-hydroxyphenyl]-3-[3-carboxydimethylmethyloxy-4-hydroxy-5-*tert*butyl phenyl]prop-2-en-1-one,
1-[2-hydroxyphenyl]-3-[3-*iso*propyloxycarbonyldimethylmethyloxy-4-hydroxy-5-*tert*butylphenyl]prop-2-en-1-one,
1-[2-hydroxy-4-chlorophenyl]-3-[3-carboxydimethylmethyloxy-4-hydroxy-5-*tert*butylphenyl]prop-2-en-1-one,
1-[2-hydroxy-4-chlorophenyl]-3-[3-*iso*propyloxycarbonyldimethylmethyloxy-4-hydroxy-5-*tert*butylphenyl]prop-2-en-1-one,
1-[2-hydroxyphenyl]-3-[3-carboxydimethylmethyl-4-hydroxy-5-tertbutylphenyl]prop-2-en-1-one,
1-[2-hydroxyphenyl]-3-[3-*iso*propyloxycarbonyldimethylmethyl-4-hydroxy-5-*tert*butylphenyl]prop-2-en-1-one,
1-[2-hydroxy-4-chlorophenyl]-3-[3-carboxydimethylmethyl-4-hydroxy-5-tertbutylphenyl]prop-2-en-1-one,
1-[2-hydroxy-4-chlorophenyl]-3-[3-*iso*propyloxycarbonyldimethylmethyl-4-hydroxy-5-tertbutylphenyl]prop-2-en-1-one,
1-[2-hydroxy-4-chlorophenyl]-3-[3,5-dimethoxy-4-carboxydimethylmethyloxyphenyl]prop-2-en-1-one,
1-[2-hydroxy-4-chlorophenyl]-3-[3,5-dimethoxy-4-*iso*propyloxycarbonyldimethylmethyloxyphenyl]prop-2-en-1-one,
1-[2-hydroxyphenyl]-3-[3,5-dimethoxy-4-carboxydimethylmethyloxyphenyl]prop-2-en-1-one,
1-[2-hydroxyphenyl]-3-[3,5-dimethoxy-4-isopropyloxycarbonyl dimethylmethyloxyphenyl]prop-2-en-1-one,
1-[2-hydroxy-4-carboxydimethylmethyloxyphenyl]-3-[3,5-di-methoxy-4-hydroxyphenyl]prop-2-en-1-one,
1-[2-hydroxy-4-*iso*propyloxycarbonyldimethylmethyloxyphenyl]-3-[3,5-dimethoxy-4-hydroxyphenyl]prop-2-en-1-one,
1-[2-hydroxy-4-chlorophenyl]-3-[3,4-dihydroxy-5-carboxydimethylmethyloxyphenyl] prop-2-en-1-one,
1-[2-hydroxy-4-chlorophenyl]-3-[3,4-dihydroxy-5-*iso*propyloxycarbonyldimethylmethyloxyphenyl]prop-2-en-1-one,
1-[2-hydroxy-4-carboxydimethylmethyloxyphenyl]-3-[3,5-dimethyl-4-hydroxyphenyl]prop-2-en-1-one,
1-[2-hydroxy-4-isopropyloxycarbonyldimethylmethyloxyphenyl]-3-[3,5-dimethyl-4-hydroxyphenyl]prop-2-en-1-one,
1-[2-hydroxy-4-chlorophenyl]-3-[3,5-dimethyl-4-carboxydimethylmethyloxyphenyl]prop-2-en-1-one,
1-[2-hydroxy-4-chlorophenyl]-3-[3,5-dimethyl-4-*iso*propyloxycarbonyldimethylmethyloxyphenyl]prop-2-en-1-one,
1-[2-hydroxyphenyl]-3-[3,5-dimethyl-4-carboxydimethylmethyloxyphenyl]prop-2-en-1-one,
1-[2-hydroxyphenyl]-3-[3,5-dimethyl-4-isopropyloxycarbonyl dimethylmethyloxyphenyl]prop-2-en-1-one,
1-[2-hydroxyphenyl]-3-[4-carboxydimethylmethylthiophenyl]prop-2-en-1-one,
1-[2-hydroxyphenyl]-3-[4-isopropyloxycarbonyldimethylmethylthiophenyl]prop-2-en-1-one,
1-[2-hydroxy-4-carboxydimethylmethyloxyphenyl]-3-[4-methylthiophenyl]prop-2-en-1-one,
1-[4-chlorophenyl]-3-[3,5-dimethyl-4-tertbutyloxycarbonyldimethylmethyloxyphenyl]prop-2-en-1-one,
1-[4-chlorophenyl]-3-[3,5-dimethyl-4-isopropyloxycarbonyldimethylmethyloxyphenyl]prop-2-en-1-one,
1-[4-chlorophenyl]-3-[3,5-dimethyl-4-carboxydimethylmethyloxyphenyl]prop-2-en-1-one,
1-[4-chloro-2-hydroxyphenyl]-3-[4-carboxydimethylmethylthiophenyl]prop-2-en-1-one,
1-[4-carboxydimethylmethyloxyphenyl]-3-[3,5-dimethyl-4-hydroxyphenyl]prop-2-en-1-one,
1-[4-methylthiophenyl]-3-[4-carboxydimethylmethyloxyphenyl]prop-2-en-1-one,
1-[4-carboxydimethylmethylthiophenyl]-3-[4-methylthiophenyl]prop-2-en-1-one,
1-[2-hydroxy-4-bromophenyl]-3-[3,5-dimethyl-4-carboxydimethylmethyloxyphenyl]prop-2-en-1-one,
1-[4-carboxydimethylmethyloxyphenyl]-3-[4-methylthiophenyl]prop-2-en-1-one,
1-[4-methylthiophenyl]-3-[3,5-dimethyl-4-tertbutyloxycarbonyldimethylmethyloxyphenyl]prop-2-en-1-one,
1-[4-methylthiophenyl]-3-[3,5-dimethyl-4-isopropyloxycarbonyldimethylmethyloxyphenyl]prop-2-en-1-one,
1-[4-methylthiophenyl]-3-[3,5-dimethyl-4-carboxydimethylmethyloxyphenyl]prop-2-en-1-one,
1-[2-methoxyphenyl]-3-[3,5-dimethyl-4-tertbutyloxycarbonyldimethylmethyloxyphenyl]prop-2-en-1-one,
1-[2-methoxyphenyl]-3-[3,5-dimethyl-4-carboxydimethylmethyloxyphenyl]prop-2-en-1-one,
1-[4-hexyloxyphenyl]-3-[3,5-dimethyl-4-tertbutyloxycarbonyldimethylmethyloxyphenyl]prop-2-en-1-one,
1-[4-hexyloxyphenyl]-3-[3,5-dimethyl-4-carboxydimethylmethyloxyphenyl]prop-2-en-1-one,
1-[2-methyloxy-4-chlorophenyl]-3-[3,5-dimethyl-4-tertbutyloxycarbonyldimethylmethyloxyphenyl]prop-2-en-1-one,
1-[2-methyloxy-4-chlorophenyl]-3-[3,5-dimethyl-4-carboxydimethylmethyloxyphenyl]prop-2-en-1-one,
1-[4-heptylphenyl]-3-[3,5-dimethyl-4-tertbutyloxycarbonyldimethylmethyloxyphenyl]prop-2-en-1-one,
1-[4-heptylphenyl]-3-[3,5-dimethyl-4-carboxydimethylmethyloxyphenyl]prop-2-en-1-one,
1-[4-bromophenyl]-3-[3,5-dimethyl-4-tertbutyloxycarbonyldimethylmethyloxyphenyl]prop-2-en-1-one,
1-[4-bromophenyl]-3-[3,5-dimethyl-4-carboxydimethylmethyloxyphenyl]prop-2-en-1-one.

24. Derivative according to anyone of the preceding claims, **characterized in that** it is selected in the group consisting of:
1-[4-methylthiophenyl]-3-[3,5-dimethyl-4-carboxydimethylmethyloxyphenyl]prop-2-en-1-one,
1-[4-hexyloxyphenyl]-3-[3,5-dimethyl-4-carboxydimethylmethyloxyphenyl]prop-2-en-1-one, and
1-[4-bromophenyl]-3-[3,5-dimethyl-4-carboxydimethylmethyloxyphenyl]prop-2-en-1-one.

25. Derivative according to anyone of the preceding claims, **characterized in that** the derivative is 1-[4-methylthiophenyl]-3-[3,5-dimethyl-4-carboxydimethylmethyloxyphenyl]prop-2-en-1-one.

26. A method for preparing compounds represented by formula (I) as defined in one of the preceding claims, **characterized in that** it comprises contacting in basic or acidic medium at least one compound corresponding to formula (A) with at least one compound corresponding to formula (B), formulas (A) and (B) being: 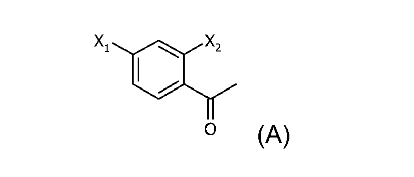 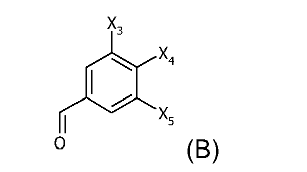 formulas in which X1, X2, X3, X4 and X5 are defined as in claim 1 or 2.

27. Pharmaceutical composition comprising, in a pharmaceutically acceptable support, at least one compound represented by formula (I) such as defined in one of claims 1 to 25, including compounds represented by general formula (I) in which X2 represents a hydrogen atom, X6 represents an oxygen atom, X1 represents -G1R1 where G1 represents an oxygen atom and R1 represents CH2COOC2H5 or CH2CONHCH(CH3)2, and X3, X4, and X5 represent OCH3.

28. Pharmaceutical composition according to the preceding claim, for the treatment or prophylaxis of a cerebrovascular pathology.

29. Pharmaceutical composition according to claim 28, **characterized in that** cerebrovascular pathology is cerebral ischemia.

30. Pharmaceutical composition according to claim 28, **characterized in that** cerebrovascular pathology is hemorrhagic stroke.

31. Use of at least one substituted 1,3-diphenylprop-2-en-1-one derivative for preparing a pharmaceutical composition for treating in a preventive or preferably curative manner a cerebrovascular pathology and more particularly cerebral ischemia, **characterized in that** the substituted 1,3-diphenylprop-2-en-1-one derivative is represented by general formula (I) such as defined in any one of claims 1 to 25, including compounds represented by general formula (I) in which :
- X₂ represents a hydrogen atom and X₁ represents -G1R1 where G1 represents an oxygen atom and R1 represents CH2COOH, and
- X2 represents a hydrogen atom, X6 represents an oxygen atom, X1 represents G1R1 where G1 represents an oxygen atom and R1 represents CH2COOC2H5 or CH2CONHCH(CH3)2, and X3, X4, and X5 represent OCH3.

## Patentansprüche

1. Substituierte 1,3-Diphenylprop-2-en-1-on-Derivate, **dadurch gekennzeichnet dass** sie die folgende Formel (I) aufweisen: 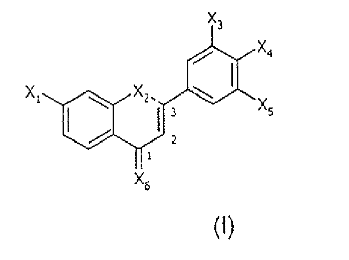 wobei:
X1 ein Halogenatom oder einen Rest -R1 oder einen Rest der folgenden Formel: -G1-R1 bedeutet;
X2 ein Wasserstoffatom oder einen Thionitrosorest oder einen Hydroxyrest oder einen Alkylcarbonyloxyrest oder einen unsubstituierten Alkyloxyrest oder einen Thiolrest oder einen Alkylthiorest oder einen Alkylcarbonylthioiest bedeutet, X2 auch ein Sauerstoffatom oder Schwefelatom bedeuten kann, das an das dritte Kohlenstoffatom, der Propenkette gebunden ist, um ein Derivat des Typs 2-Phenyl-4H-1-benzopyran-4-on oder des Typs 2-Phenyl-4H-1-benzothiopyran-4-on zu bilden;
X3 einen Rest -R3 oder einen Rest der folgenden Formel: -G3-R3 bedeutet;
X4 ein Halogenatom oder einen Thionitrosorest oder einen Rest -R4 oder einen Rest der folgenden Formel: -G4-R4 bedeutet;
X5 einen Rest -R5 oder einen Rest der folgenden Formel: -G5-R5 bedeutet;
X6 ein Sauerstoffatom ist;
R1, R3, R4, R5, gleich oder voneinander verschieden, ein Wasserstoffatom oder einen Alkylrest bedeuten, der mit einem Rest aus der nachstehend definierten Gruppe 1 oder Gruppe 2 substituiert ist oder nicht;
G1, G3, G4, G5, gleich oder voneinander verschieden, ein Sauerstoffatom oder Schwefelatom bedeuten;
wobei mindestens einer der Reste X1, X3, X4 oder X5 die Formel -G-R aufweist, wobei G ein Schwefelatom ist, und
wobei mindestens einer der Reste R1, R3, R4 oder R5 in Form eines Alkylrests vorliegt, der mindestens einen Substituenten aus der Gruppe 1 oder 2 trägt, wobei der Alkylrest direkt an den Ring gebunden ist oder mit einem Rest G gemäß der Formel-GR assoziiert ist;
wobei die Substituenten der Gruppe 1 ausgewählt sind aus Carboxyresten der Formel: -COOR₆ und Carbamoylresten der Formel: -CONR₆R₇;
wobei die Substituenten der Gruppe 2 ausgewählt sind aus Sulfonsäure (-SO₃H) und Sulfonamidresten der Formel: -SO₂NR₆R₇;
wobei R₆ und R₇, gleich oder voneinander verschieden, ein Wasserstoffatom oder einen Alkylrest bedeuten, der gegebenenfalls mit mindestens einem Rest vom Typ 1 oder 2 substituiert ist;
deren optische und geometrische Isomere, deren Racemate, deren Tautomere, deren Salze, deren Hydrate und deren Gemische;
ausgenommen Verbindungen der Formel (I), in denen:
X₂ ein Wasserstoffatom bedeutet und X₁ -GIRI bedeutet, wobei G1 ein Sauerstoffatom bedeutet und R1 CH2COOH bedeutet.

2. Substituierte 1,3-Diphenylprop-2-en-1-on-Derivate, **dadurch gekennzeichnet, dass** sie die folgende Formel (I) auf weisen: 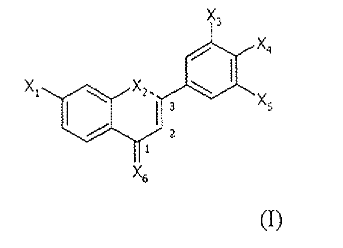 wobei:
X1 ein Halogenatom oder einen Rest -R1 oder einen Rest der folgenden Formel: -G1-R1 bedeutet;
X2 ein Wasserstoffatom oder einen Thionitrosorest oder einen Hydroxyrest oder einen Alkylcarbonyloxyrest oder einen unsubstituierten Alkyloxyrest oder einen Thiolrest oder einen Alkylthiorest oder einen Alkylcarbonylthiorest bedeutet, X2 auch ein Schwefelatom bedeuten kann, das an das Kohlenstoffatom 3 der Propenkette gebunden ist, um ein Derivat des Typs 2-Phenyl-4H-1-benzothiopyran-4-on zu bilden;
X3 einen Rest -R3 oder einen Rest der folgenden Formel: -G3-R3 bedeutet;
X4 ein Halogenatom oder einen Thionitrosorest oder einen Rest -R4 oder einen Rest der folgenden Formel: -G4-R4 bedeutet;
X5 einen Rest -R5 oder einen Rest der folgenden Formel: -G5-R5 bedeutet;
X6 ein Sauerstoffatom ist;
R1, R3, R4, R5, gleich oder voneinander verschieden, ein Wasserstoffatom oder einen Alkylrest bedeuten, der mit einem Rest aus der nachstehend definierten Gruppe 1 oder Gruppe 2 substituiert ist oder nicht;
G1, G3, G4, G5, gleich oder voneinander verschieden, ein Sauerstoffatom oder Schwefelatom bedeuten;
wobei mindestens einer der Reste X1, X3, X4 oder X5 die Formel -G-R aufweist;
wobei keiner der Reste X3, X4 oder X5 ein Wasserstoffatom bedeutet; und
wobei mindestens einer der Reste R1, R3, R4 oder R5 in Form eines Alkylrests vorliegt, der mindestens einen Substituenten aus der Gruppe 1 oder 2 trägt, wobei der Alkylrest direkt an den Ring gebunden ist oder mit einem Rest G gemäß der Formel -GR assoziiert ist;
wobei die Substituenten der Gruppe 1 ausgewählt sind aus Carboxyresten der Formel: -COOR₆ und Carbamoylresten der Formel: -CONR₆R₇;
wobei die Substituenten der Gruppe 2 ausgewählt sind aus Sulfonsäure (SO₃H) und Sulfonamidresten der Formel: -SO₂NR₆R₇;
wobei R₆ und R₇, gleich oder voneinander verschieden, ein Wasserstoffatom oder einen Alkylrest bedeuten, der gegebenenfalls mit mindestens einem Rest vom Typ 1 oder 2 substituiert ist;
deren optische und geometrische Isomere, deren Racemate, deren Tautomere, deren Salze, deren Hydrate und deren Gemische;
ausgenommen Verbindungen der Formel (I), in denen:
X₂ ein Wasserstoffatom bedeutet und X₁ -G1R1 bedeutet, wobei G1 ein Sauerstoffatom bedeutet und R1 CH2COOH bedeutet;
X₂ ein Wasserstoffatom bedeutet, X₆ ein Sauerstoffatom bedeutet, X₁ -G1R1 bedeutet, wobei G 1 ein Sauerstoffatom bedeutet und R1 CH2COOC2H5 oder CH2CONHCH(CH3)2 bedeutet und X₃, X₄ und X₅ OCH3 bedeuten.

3. Derivate nach Anspruch 1 oder 2, **dadurch gekennzeichnet, dass** sie in cis-Konformntion, trans-Konformation oder einer Mischung daraus vorliegen können.

4. Derivate nach Anspruch 1, **dadurch gekennzeichnet, dass** keiner der Reste X3, X4 und X5 ein Wasserstoffatom bedeutet.

5. Derivate nach Anspruch 1, **dadurch gekennzeichnet, dass** einer oder zwei der Reste X3, X4 und X5 ein Wasserstoffatom bedeuten.

6. Derivate nach einem der vorhergehenden Ansprüche, **dadurch gekennzeichnet, dass** die beiden Reste G1 und G4 ein Schwefelatom bedeuten.

7. Derivate nach einem der vorhergehenden Ansprüche, **dadurch gekennzeichnet, dass** X2 ein Wasserstoffatom, ein Thionitrosorest oder ein Hydroxyrest oder ein Alkyloxyrest oder ein Thiolrest oder ein Alkylthiorest ist.

8. Derivate nach einem der vorhergehenden Ansprüche, **dadurch gekennzeichnet, dass** X4 einen Thionitrosorest oder einen Rest -R4 oder einen Rest der Formel -G4R4 bedeutet und X2 ein Thionitrosorest oder ein Hydroxy-, Alkyloxy-, Thiol- oder Alkylthiorest ist, wobei G4 und R4 wie in Anspruch 1 oder 2 definiert sind.

9. Derivate nach einem der vorhergehenden Ansprüche, **dadurch gekennzeichnet, dass** X1 einen Rest -R1 oder einen Rest der Formel -G1-R1 bedeutet, wobei R1 ein Alkylrest ist, der mit einem Rest aus der Gruppe 1 substituiert ist, wobei G1 und der Substituent aus der Gruppe 1 wie in Anspruch 1 oder 2 definiert sind.

10. Derivate nach einem der vorhergehenden Ansprüche, **dadurch gekennzeichnet, dass** X1 ein Rest -G1-R1 ist, wobei G1 und R1 wie in Anspruch 1 oder 2 definiert sind.

11. Derivate nach einem der vorhergehenden Ansprüche, **dadurch gekennzeichnet, dass** X1 ein Rest -G1-R1 ist, wobei G1 ein Sauerstoffatom ist, wobei R1 wie in Anspruch 1 oder 2 definiert ist.

12. Derivate nach einem der vorhergehenden Ansprüche, **dadurch gekennzeichnet, dass** X1 einen Rest -R1 oder einen Rest der Formel -G1-R1 bedeutet, wobei R1 ein Alkylrest ist, der mit einem Rest aus der Gruppe 2 substituiert ist, wobei G1 und der Substituent aus der Gruppe 2 wie in Anspruch 1 oder 2 definiert sind.

13. Derivate nach einem der vorhergehenden Ansprüche, **dadurch gekennzeichnet, dass** X3 einen Rest -R3 oder einen Rest der Formel -G3-R3 bedeutet, wobei R3 ein Alkylrest ist, der mit einem Rest aus der Gruppe 1 substituiert ist, wobei G3 und der Substituent aus der Gruppe 1 wie in Anspruch 1 oder 2 definiert sind.

14. Derivate nach einem der vorhergehenden Ansprüche, **dadurch gekennzeichnet, dass** X3 einen Rest -R3 oder einen Rest der Formel -G3-R3 bedeutet, wobei R3 ein Alkylrest ist, der mit einem Rest aus der Gruppe 2 substituiert ist, wobei G3 und der Substituent aus der Gruppe 2 wie in Anspruch 1, oder 2 definiert sind.

15. Derivate nach einem der vorhergehenden Ansprüche, **dadurch gekennzeichnet, dass** X4 einen Rest -R4 oder einen Rest der Formel -G4-R4 bedeutet, wobei R4 ein Alkylrest ist, der mit einem Rest aus der Gruppe 1 substituiert ist, wobei G4 und der Substituent aus der Gruppe 1 wie in Anspruch 1 oder 2 definiert sind.

16. Derivate nach einem der vorhergehenden Ansprüche, **dadurch gekennzeichnet, dass** X4 ein Rest -G4-R4 ist, wobei G4 und R4 wie in Anspruch 1 oder 2 definiert sind.

17. Derivate nach einem der vorhergehenden Ansprüche, **dadurch gekennzeichnet, dass** X4 ein Rest -G4-R4 ist, wobei G4 ein Sauerstoffatom ist, R4 ist wie in Anspruch 1 oder 2 definiert.

18. Derivate nach einem der vorhergehenden Ansprüche, **dadurch gekennzeichnet, dass** X4 ein Rest -G4-R4 ist, wobei G4 ein Sauerstoffatom ist, und X3 bzw. X5 einerseits R3 oder G3R3 und andererseits R5 oder G5R5 bedeuten, wobei R3 oder R5 Alkylreste sind, die einen Substituenten aus der Gruppe 1 tragen, wobei R4, G3, G5 und der Substituent aus der Gruppe 1 wie in Anspruch 1 oder 2 definiert sind.

19. Derivate nach einem der vorhergehenden Ansprüche, **dadurch gekennzeichnet, dass** X4 einen Rest -R4 oder einen Rest der Formel -G4-R4 bedeutet, wobei R4 ein Alkylrest ist, der mit einem Rest aus der Gruppe 2 substituiert ist, wobei G4 und der Substituent aus der Gruppe 2 wie in Anspruch 1 oder 2 definiert sind.

20. Derivate nach einem der vorhergehenden Ansprüche, **dadurch gekennzeichnet, dass** X1 ein Halogenatom bedeutet.

21. Derivate nach einem der vorhergehenden Ansprüche, **dadurch gekennzeichnet, dass** X1, X3, X4 oder X5 OC(CH3)2COOR6 bedeuten, wobei R6 wie in Anspruch 1 oder 2 definiert ist.

22. Derivate nach einem der vorhergehenden Ansprüche, **dadurch gekennzeichnet, dass** X1, X3, X4 oder X5 SC(CH3)2COOR6 bedeuten, wobei R6 wie in Anspruch 1 oder 2 definiert ist.

23. Derivat nach einem der vorhergehenden Ansprüche, **dadurch gekennzeichnet, dass** es ausgewählt ist aus:
1-[2-Hydroxy-4-carboxydimethylmethyloxyphenyl]-3-[3,5-di*tert*butyl-4-hydroxyphenyl]prop-2-en-1-on,
1-[2-Hydroxy-4-ethoxycarbonyldimethylmethyloxyphenyl]-3-[3,5-di*tert*butyl-4-hydroxyphenyl]prop-2-en-1-on,
1-[2-Hydroxy-4-isopropyloxycarbonyldimethylmethyloxyphenyl]-3-[3,5-di*tert*butyl-4-hydroxyphenyl]prop-2-en-1-on,
1-[2-Hydroxyphenyl]-3-[3-carboxydimethylmethyloxy-4-hydroxy-5-*tert*butylphenyl]-prop-2-en-1-on,
1-[2-Hydroxyphenyl]-3-[3-*iso*propyloxycarbonyldimethylmethyloxy-4-hydroxy-5-*tert*butylhhenyl]prop-2-en-1-on,
1-[2-Hydroxy-4-chlorphenyl]-3-[3-carboxydimethylmethyloxy-4-hydroxy-5-*tert*butylphenyl]prop-2-en-1-on,
1-[2-Hydroxy-4-chlorphenyl]-3-(3-*iso*propyloxycarbonyldimethylmethyloxy-4-hydroxy-5-*tert*butylphenyl]prop-2-en-1-on,
1-[2-Hydroxyphenyl]-3-[3-carboxydimethylmethyl-4-hydroxy-5-*tert*butylphenyl]prop-2-en-1-on,
1-[2-Hydroxyphenyl]-3-[3-*iso*propyloxycarbonyldimethylmethyl-4-hydroxy-5-*tert*butylphenyl]prop-2-en-1-on,
1-[2-hydroxy-4-chlorphenyl]-3-[3-carboxydimethylmethyl-4-hydroxy-5-*tert*butyl-phenyl]prop-2-en-1-on,
1-[2-Hydroxy-4-chlorphenyl]-3-[3-*iso*propyloxycarbonyldimethylmethyl]4-hydroxy-5-*tert*butylphenyl]prop-2-en-1-on,
1-[2-Hydroxy-4-chlorphenyl]-3-[3,5-dimethoxy-4-carboxydimethylmethyloxyphenyl]-prop-2-en-1-on,
1-[2-Hydroxy-4-chlorphenyl]-3-[3,5-dimethoxy-4-*iso*propyloxycarbonyldimethyl-methyloxyphenyl]prop-2-en-1-on,
1-[2-Hydroxyphenyl]-3-[3,5-dimethoxy-4-carboxydimethylmethyloxyphenyl]prop-2-en-1-on,
1-[2-Hydroxyphenyl]-3-[3,5-dimethoxy-4-*iso*propyloxycarbonyldimethylmethyloxyphenyl]prop-2-en-1-on,
1-[2-Hydroxy-4-carboxydimethylmethyloxyphenyl]-3-[3,5-dimethoxy-4-hydroxyphenyl]prop-2-en-1-on,
1-[2-Hydroxy-4-*iso*propyloxycarbonyldimethylmethyloxyphenyl]-3-[3,5-dimethoxy-4-hydl-oxyphenyl]prop-2-en-1-on,
1-[2-Hydroxy-4-chlorphenyl]-3-[3,4-dihydroxy-5-carboxydimethylmethyloxyphenyl]-prop-2-en-1-on,
1-[2-Hydroxy-4-chlorphenyl]-3-[3,4-dihydroxy-5-*iso*propyloxycarbonyldimethylmethyloxyphenyl]-prop-2-en-1-on,
1-[2-Hydroxy-4-carboxydimethylmethyloxyphenyl]-3-[3,5-dimethyl-4-hydroxyphenyl]-prop-2-en-1-on,
1-[2-Bydroxy-4-*iso*propyloxycarbonyldimethyl methyloxyphenyl]-3-[3,5-dimethyl-4-hydroxyphenyl]prop-2-en-1-on,
1-[2-Hydroxy-4-chlorphenyl]-3-[3,5-dimethyl-4-carboxyclimethylmethyloxyphenyl]-prop-2-en-1-on,
1-[2-Hydroxy-4-chlorphenyl]-3-[3,5-dimethyl-4-*is*opropyloxycarbonyldimethylmethyloxyphenyl]prop-2-en-1-on,
1-[2-Hydroxyphenyl]-3-[3,5-dimethyl-4-carboxydimethylmethyloxyphenyl]prop-2-en-1-on,
1-[2-Hydroxyphenyl]-3-[3,5-dimethyl-4-*iso*propyloxycarbonyldimethylmethyloxyphenyl]prop-2-en-1-on,
1-[2-Hydroxyphenyl]-3-[4-carboxydimethylmethylthiophenyl]prop-2-en-1-on,
1-[2-Hydroxyphenyl]-3-[4-*iso*propyloxycarbonyldimethylmethylthiophenyl]prop-2-en-1-on,
1-[2-Hydroxy-4-carboxydimethylmethyloxyphenyl]-3-[4-methylthiophenyl]prop-2-en-1-on,
1-[4-Chlorphenyl]-3-[3,5-dimethyl-4-tert-butyloxycarbonyldimethylmethyloxyphenyl]-prop-2-en-1-on,
1-[4-Chlorphenyl]-3-[3,5-dimethyl-4-isopropyloxycarbonyldimethylmethyloxyphenyl]-prop-2-en-1-on,
1-[4-Chlorphenyl]-3-[3,5-dimethyl-4-carboxydimethyloxyphenyl]prop-2-en-1-on,
1-[4-Chlor-2-hydroxyphenyl]-3-[4-carboxydimethylmethylthiophenyl]prop-2-en-1-on,
1-[4-Carboxydimethylmethyloxyphenyl]-3-[3,5-dimethyl-4-hydroxyphenyl]prop-2-en-1-on,
1-[4-Methylthiophenyl]-3-[4-carboxydimethylmethyloxyphenyl]prop-2-en-1-on,
1-[4-Carboxydimethylmethylthiophenyl]-3-[4-methylthiophenyl]prop-2-en-1-on,
1-[2-Hydroxy-4-bromphenyl]-3-[3,5-dimethyl-4-carboxydimethylmethyloxyphenyl]prop-2-en-1-on,
1-[4-Carboxydimethylmethyloxyphenyl]-3-[4-methylthiophenyl]prop-2-en-1-on,
1-[4-Methylthiophenyl]-3-[3,5-dimethyl-4-tert-butyloxycarbonyldimethylmethyloxyphenyl]prop-2-en-1-on,
1-[4-Methylthiophenyl]-3-[3,5-dimethyl-4-isopropyloxycarbonyldimethylmethyloxyphenyl]prop-2-en-1-on,
1-[4-Methylthiophenyl]-3-[3,5-dimethyl-4-carboxydimethylmethyloxyphenyl]prop-2-en-1-on,
1-[2-Methoxyphenyl]-3-[3,5-dimethyl-4-tert-butyloxycarbonyldimethylmethyloxyphenyl]prop-2-en-1-on,
1-[2-Methoxyphenyl]-3-[3,5-dimethyl-4-carboxydimethylmethyloxyphenyl]prop-2-en-1-on,
1-[4-Hexyloxyphenyl]-3-[3,5-dimethyl-4-tert-butyloxycarbonyldimethylmethyloxyphenyl]prop-2-en-1-on,
1-[4-Hexyloxyphenyl]-3-[3,5-dimethyl-4-carboxydimethylmethyloxyphenyl]prop-2-en-1-on,
1-[2-Methyloxy-4-chlorphenyl]-3-[3,5-dimethyl-4-tert-butyloxycarbonyldimethylmethyloxyphenyl]prop-2-en-1-on,
1-[2-Methyloxy-4-chlorphenyl]-3-[3,5-dimethyl-4-carboxydimethylmethybxyphenyl]-prop-2-en-1-on,
1-[4-Heptylphenyl]-3-[3,5-dimethyl-4-tert-butyloxycarbonyldimethylmethyloxyphenyl]-prop-2-en-1-on,
1-[4-Heptylphenyl]-3-[3,5-dimethyl-4-carboxydimethylmethyloxyphenyl]prop-2-en-1-on,
1-[4-Bromphenyl]-3-[3,5-dimethyl-4-tert-butyloxycarbonyldimethylmethyloxyphenyl]-prop-2-en-1-on,
1-[4-Bromphenyl]-3-[3,5-dimethyl-4-carboxydimethyloxyphenyl]prop-2-en-1-on.

24. Derivat nach einem der vorhergehenden Ansprüche, **dadurch gekennzeichnet, dass** das Derivat ausgewählt ist aus:
1-[4-Methylthiophenyl]-3-[3,5-dimethyl-4-carboxydimethylmethyloxyphenyl]prop-2-en-1-on,
1-[4-Hexyloxyphenl]-3-[3,5-dimethyl-4-carboxydimethylmethyloxyphenyl]prop-2-en-1-on und
1-[4-Bromphenyl]-3-[3,5-dimethyl-4-carboxydimethylmethyloxyphenyl]prop-2-en-1-on.

25. Derivat nach einem der vorhergehenden Ansprüche, **dadurch gekennzeichnet, dass** das Derivat 1-[4-Methylthiophenyl]-3-[3,5-dimethyl-4-carboxydimethylmethyloxyphenyl]-prop-2-en-1-on ist.

26. Verfahren zur Herstellung von Verbindungen der Formel (1) wie in einem der vorhergehenden Ansprüche definiert, **dadurch gekennzeichnet, dass** es ein Kontaktieren mindestens einer Verbindung der Formel (A) mit mindestens einer Verbindung der Formel (B) in basischem oder saurem Milieu umfasst, wobei die Formeln (A) und (B) die folgenden Formeln sind: 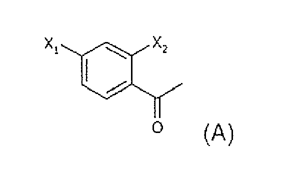 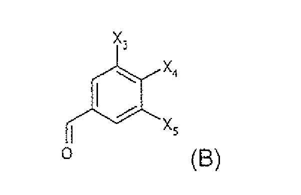 wobei X1, X2, X3, X4 und X5 in den Formeln die in Anspruch 1 oder 2 gegebenen Definitionen haben.

27. Arzneimittel, umfassend in einem pharmazeutisch verträglichen Träger mindestens eine Verbindung der Formel (I) wie in einem der Ansprüche 1 bis 25 definiert, einschließlich Verbindungen der Formel (I), in denen X₂ ein Wasserstoffatom bedeutet, X₆ ein Sauerstoffatom bedeutet, X₁ -G1R1 bedeutet, wobei G1 ein Sauerstoffatom bedeutet und R1 CH2COOC2H5 oder CH2CONHCH(CH3)2 bedeutet und X₃, X₄ und X₅ OCH3 bedeuten.

28. Arzneimittel nach dem vorhergehenden Anspruch zur Behandlung oder Prophylaxe einer zerebralen Gefäßerkrankung.

29. Arzneimittel nach Anspruch 28, **dadurch gekennzeichnet, dass** die zerebrale Gefäßerkrankung zerebrale Ischämie ist.

30. Arzneimittel nach Anspruch 28, **dadurch gekennzeichnet, dass** die zerebrale Gefäßerkrankung eine zerebrale Blutung ist.

31. Verwendung mindestens eines substituierten 1,3-Diphenylprop-2-en-1-on-Derivats zur Herstellung eines Arzneimittels, das zur präventiven oder kurativen Behandlung einer zerebralen Gefäßerkrankung, insbesondere zerebraler Ischämie, bestimmt ist, **dadurch gekennzeichnet, dass** das substituierte 1,3-Diphenylprop-2-en-1-on-Derivat eine allgemeine Formel (I), wie in einem der Ansprüche 1 bis 25 definiert, aufweist, einschließlich Verbindungen der allgemeinen Formel (I), in der:
X₂ ein Wasserstoffatom bedeutet und X₁ -G1R1 bedeutet, wobei G1 ein Sauerstoffatom bedeutet und R1 CH2COOH bedeutet; und
X₂ ein Wasserstoffatom bedeutet, X₆ ein Sauerstoffatom bedeutet, X₁ -G1R1 bedeutet, wobei G1 ein Sauerstoffatom bedeutet und R1 CH2COOC2H5 oder CH2CONHCH(CH3)2 bedeutet und X₃, X₄ und X₅ OCH₃ bedeuten.
